(19) Europäisches Patentamt / European Patent Office / Office européen des brevets

(11) EP 2 770 983 B1

(12) **EUROPEAN PATENT SPECIFICATION**

(45) Date of publication and mention of the grant of the patent:
**19.04.2017 Bulletin 2017/16**

(21) Application number: **12780396.3**

(22) Date of filing: **24.10.2012**

(51) Int Cl.:
*A61K 9/20* (2006.01) *A61K 9/00* (2006.01)

(86) International application number:
**PCT/US2012/061701**

(87) International publication number:
**WO 2013/063125 (02.05.2013 Gazette 2013/18)**

(54) **IMPLANTABLE DRUG DELIVERY COMPOSITIONS AND METHODS OF TREATMENT THEREOF**

IMPLANTIERBARE ARZNEIMITTELABGABEZUSAMMENSETZUNGEN UND BEHANDLUNGSVERFAHREN DAMIT

COMPOSITIONS IMPLANTABLE POUR ADMINISTRATION DE MÉDICAMENTS ET MÉTHODES DE TRAITEMENT CORRESPONDANTES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.10.2011 US 201161550653 P**
**08.08.2012 US 201261680856 P**

(43) Date of publication of application:
**03.09.2014 Bulletin 2014/36**

(73) Proprietor: **Braeburn Pharmaceuticals, Inc.**
**Princeton, NJ 08542 (US)**

(72) Inventors:
- **SCHWARZ, Alexander**
  **Brookline, MA 02446 (US)**
- **BOSE, Sagarika**
  **Edison, NJ 08820 (US)**
- **QUANDT, Harry**
  **Bensalem, PA 19020 (US)**
- **KUZMA, Petr**
  **Princeton, NJ 08540 (US)**
- **CAIZZA, Richard**
  **Vernon, NJ 07462 (US)**
- **BAI, Stephen**
  **Newark, DE 19711 (US)**
- **KIRBY, Mark, Toddman**
  **Pottstown, PA 19465 (US)**
- **TZANIS, Evangelos, Loucas**
  **Ardmore, PA 19003 (US)**

(74) Representative: **RatnerPrestia**
**Altheimer Eck 2**
**80331 München (DE)**

(56) References cited:
**WO-A1-2010/039643** **WO-A1-2010/039717**
**WO-A1-2011/116132** **WO-A2-2009/060473**
**US-A- 5 035 891** **US-A1- 2009 208 540**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 770 983 B1

## Description

FIELD OF THE INVENTION

The invention relates to reservoir-based drug delivery compositions that are implantable into a subject in order to deliver therapeutically effective amounts of a drug, for example, at a pseudo-zero order rate, for extended periods of time (*e.g.*, at least one month, one year, etc.).

BACKGROUND OF THE INVENTION

Drug compositions come in many different forms and may be administered to a patient via several different routes, such as oral, parenteral, topical, intravenous, subcutaneous, intranasal, etc. Depending on the active and the treatment desired, different routes of administration may be preferable.

Some diseases and conditions may be long lasting, requiring treatment for many weeks, months, or even years. Typically, a patient taking a traditional oral dosage form (*e.g.*, tablets or capsules) may be required to take the oral dose at least once per day for the duration of the treatment. For example, a patient may need to take an oral dose twice a day for a year or longer. The problem with treatments that require continuous dosage over a long period of time is that often the patient may not be compliant in taking the medications. In other words, the patient may forget, believe the treatment is unnecessary, or grow tired of having to take many pills over an extremely long period of time. Accordingly, treatments are necessary which can alleviate these compliance issues, but still provide effective and efficient treatment to the patient.

As one example, compliance with breast cancer medications has been an issue. Breast cancer is the leading life-threatening cancer affecting women, and 200,000 new cases of breast cancer are diagnosed each year. There are 2.5 million women alive in the U.S. who have a history of breast cancer of which 75% are estrogen receptor(s) positive (ER+) and progesterone receptor(s) positive (PR+), and 88% are post-menopausal. Typical treatment is interventional (*e.g.*, chemotherapy/radiation/ surgery) followed by adjunctive therapy (*e.g.*, used with or after the primary treatment), where appropriate. After interventional therapy, almost all (*e.g.*, about 95%) hormone receptor positive, post-menopausal patients are prescribed an aromatase inhibitor to suppress estrogen and prevent recurrence. In recent years, aromatase inhibitors have replaced tamoxifen as the products of choice in post-menopausal women. Astra Zeneca's ARIMIDEX™ anastrozole, Novartis' FEMARA® letrozole, and Pfizer's AROMASIN® exemestane are the leading oral aromatase inhibitors on the market.

Studies have shown that there is significant treatment fatigue among patients who are supposed to follow the oral aromatase inhibitor treatment regime, however. Studies have documented low compliance, low adherence, and low persistence, for example, with patients prescribed a five-year aromatase inhibitor therapy. In one study, Adherence to Initial Adjuvant Anastrozole Therapy Among Women With Early-Stage Breast Cancer, Partridge et al., Journal of Clinical Oncology, Volume 26, No. 4, February 1, 2008, which is hereby incorporated by reference in its entirety for all purposes, the mean adherence decreased each year for women with 36 months of continuous eligibility from 78% to 86% adherence in the first year to 62% to 79% adherence by the third year. Adherence was defined as the proportion of eligible days during the 360 days after a patient's first anastrozole prescription for which the patient had filled prescriptions for anastrozole or alternative endocrine therapy. The study found that approximately one in four women with early-stage breast cancer may be suboptimally adherent to adjuvant anastrozole therapy during the first year and adherence was found to decline over time.

In another study, Early Discontinuation and Nonadherence to Adjuvant Hormonal Therapy in a Cohort of 8,769 Early-Stage Breast Cancer Patients, Hershman et al., Journal of Clinical Oncology, Volume 26, No. 27, September 20, 2010, which is hereby incorporated by reference in its entirety for all purposes, women younger than forty were found to have the highest risk of discontinuation. In particular, women at the extremes of the age range (i.e., those <40 years or >75 years) were particularly likely to be non-adherent. Barriers to adherence may include lack of communication between physician and patient, not appreciating the potential adverse effects, and cost, for example.

As a second example, compliance with schizophrenia medications also has been an issue. Schizophrenia is a complex mental disorder, which affects both men and women equally. It often begins in the teen years or young adulthood, but may also begin later in life. People with any type of schizophrenia may have difficulty keeping friends and working, and may also have problems with anxiety, depression, and suicidal thoughts or behaviors. Schizophrenia ranks among the top ten causes of disability in developed countries worldwide, and as many as 51 million people worldwide suffer from schizophrenia. Although there is no cure for schizophrenia, the treatment success rate with antipsychotic medications and psycho-social therapies can be high. Janssen Pharmaceuticals' RISPERDAL® risperidone is one of the oral antipsychotics on the market. However, an estimated 40% of all relapses suffered by schizophrenic patients are due to noncompliance in taking their prescribed medicine. Patient relapse from noncompliance may also result in the return of more severe and dangerous psychotic symptoms, and persistent noncompliance can worsen the

prognosis and make the patient less likely to respond to medication.

Accordingly, there has remained a need for effective dosage forms that provide therapeutically effective amounts of a drug over a long period of time, especially for drugs where non-compliance is a major issue.

SUMMARY OF THE INVENTION

Aspects of the present invention include reservoir-based drug delivery compositions, which may be implanted into a subject in order to deliver a therapeutically effective amount of a drug (such as aromatase inhibitors or risperidone) to the subject for long periods of time (*e.g.*, at least one month, at least three months, at least six months, at least one year, etc.). The therapeutically effective amount of the drug may be delivered at a pseudo-zero order rate. Accordingly, the present invention is directed to drug compositions, methods of treatment (*e.g.*, treating estrogen-related disorders, such as breast cancer, endometriosis, uterine fibroids, short stature in a child or adolescent, etc. or psychotic disorders, such as schizophrenia, bipolar disorder, autism, etc.), methods of delivering the drug, subcutaneous delivery systems, and kits regarding the same.

According to an embodiment of the present invention, a method of treating an estrogen-related disorder comprises implanting a reservoir-based drug delivery composition into a subject to systemically deliver a therapeutically effective amount of an aromatase inhibitor to the subject for a period of time of at least one month. The drug delivery composition may comprise at least one discrete solid dosage form comprising at least one aromatase inhibitor surrounded by an excipient comprising at least one polymer. The therapeutically effective amount of the at least one aromatase inhibitor may be delivered at a pseudo-zero order rate.

According to another embodiment of the present invention, a method of treating an estrogen-related disorder includes implanting a reservoir-based drug delivery composition into a subject to systemically deliver a therapeutically effective amount of anastrozole to the subject at a pseudo-zero order rate for a period of time of at least one month. The reservoir-based drug delivery composition comprises at least one discrete solid dosage form surrounded by an excipient comprising at least one polymer, and the at least one discrete solid dosage form comprises 75-97 wt% (*e.g.*, about 88 wt%) anastrozole or a pharmaceutically effective salt thereof based on the total weight of the at least one discrete solid dosage form and 1-25 wt% (*e.g.*, about 10 wt%) of at least one sorption enhancer based on the at least one discrete solid dosage form.

According to another embodiment of the present invention, a method of systemically delivering an aromatase inhibitor to a subject includes releasing a therapeutically effective amount of an aromatase inhibitor from a reservoir-based composition comprising a polymeric rate-controlling excipient defining a reservoir containing at least one discrete solid dosage form comprising at least one aromatase inhibitor to provide a pseudo-zero order elution rate to the subject for a period of time of at least one month.

According to another embodiment of the present invention, a drug delivery composition comprises a drug elution rate-controlling excipient comprising an elastomeric polymer defining a reservoir, and the reservoir contains at least one discrete solid dosage form comprising at least one aromatase inhibitor.

According to another embodiment of the present invention, a subcutaneous delivery system comprises an elastomeric reservoir implant comprising at least one discrete solid dosage form surrounded by a polymeric rate-controlling excipient. The at least one discrete solid dosage form comprises at least one aromatase inhibitor (*e.g.*, anastrozole). The subcutaneous delivery system provides for release of the aromatase inhibitor at an elution rate suitable to provide a therapeutically effective amount of the aromatase inhibitor to a subject at a pseudo-zero order rate for a period of time of at least one month.

According to another embodiment of the present invention, a kit for subcutaneously placing a drug delivery composition comprises a reservoir-based drug delivery composition comprising a polymeric rate-controlling excipient defining a reservoir containing at least one discrete solid dosage form comprising at least one aromatase inhibitor; and an implanter for inserting the reservoir-based drug delivery composition beneath the skin, and optionally instructions for performing the implantation and explantation of the drug delivery composition.

According to another embodiment of the present invention, a method of delivering a therapeutically effective amount of an active pharmaceutical ingredient from an implantable drug delivery composition comprises implanting a reservoir-based drug delivery composition into a subject to systemically deliver a therapeutically effective amount of an active pharmaceutical ingredient to the subject at a pseudo-zero order rate for a period of time of at least one month. The drug delivery composition comprises at least one discrete solid dosage form surrounded by an excipient comprising at least one polymer, and the at least one discrete solid dosage form comprises the active pharmaceutical ingredient or a pharmaceutically acceptable salt thereof. The polymer comprises a substantially non-porous, elastomeric polymer comprising soft and hard segments, and the relative content of the soft and hard segments provide an elution rate within a target range of average daily elution rate for the active pharmaceutical ingredient.

According to another embodiment of the present invention, a drug delivery composition includes a rate-controlling excipient defining a reservoir which contains at least one discrete solid dosage form comprising an active pharmaceutical ingredient or a pharmaceutically acceptable salt thereof. The rate-controlling excipient comprises a substantially non-porous, elastomeric polymer comprising soft and hard segments selected based on the relative content of soft and hard segments of the polymer to obtain an elution rate within a target range of average daily elution rate for the active pharmaceutical ingredient. The at least one discrete solid dosage form comprises at least one sorption enhancer in an amount effective to modulate the average daily elution rate of the active pharmaceutical ingredient to provide for release of the active pharmaceutical ingredient at pseudo-zero order within the target range at the therapeutically effective amount for a period of time of at least one month. The amount of sorption enhancer is directly proportional to the average daily elution rate.

According to another embodiment, a method of treating the symptoms of a psychotic disorder comprises implanting a reservoir-based drug delivery composition into a subject to systemically deliver a therapeutically effective amount of risperidone or a pharmaceutically acceptable salt thereof to the subject at a pseudo-zero order rate for a period of time of at least one month, wherein the drug delivery composition comprises at least one discrete solid dosage form comprising risperidone or a pharmaceutically acceptable salt thereof and at least one sorption enhancer surrounded by an excipient comprising an aliphatic polyether-based polyurethane or a polyether-amide. An average daily elution rate of the risperidone or a pharmaceutically acceptable salt thereof varies in direct proportion to the amount of sorption enhancer in the drug delivery composition.

According to another embodiment, a method of treating the symptoms of a psychotic disorder comprises implanting a reservoir-based drug delivery composition into a subject to systemically deliver a therapeutically effective amount of risperidone to the subject at a pseudo-zero order rate for a period of time of at least one month. The drug delivery composition comprises at least one discrete solid dosage form comprising risperidone or a pharmaceutically acceptable salt thereof surrounded by an aliphatic polyether-based polyurethane comprising poly(tetramethylene oxide) and polymerized 4,4'-diisocyanato dicyclohexylmethane (H12MDI) and 1,4-butanediol. Alternatively, the at least one discrete solid dosage form may comprise risperidone or a pharmaceutically acceptable salt thereof surrounded by a polyether-amide. The at least one discrete solid dosage form comprises 75-97 wt% risperidone or a pharmaceutically acceptable salt thereof based on the total weight of the at least one discrete solid dosage form and 1-25 wt% of at least one sorption enhancer based on the total weight of the at least one discrete solid dosage form. An average daily elution rate of the risperidone or a pharmaceutically acceptable salt thereof varies in direct proportion to the amount of sorption enhancer in the drug delivery composition.

According to another embodiment, a risperidone composition comprises a drug elution rate-controlling excipient consisting of an aliphatic polyether-based polyurethane comprising poly(tetramethylene oxide) and polymerized 4,4'-diisocyanato dicyclohexylmethane (H12MDI) and 1,4-butanediol defining a reservoir. The reservoir contains at least one discrete solid dosage form comprising 75-97 wt% risperidone or a pharmaceutically acceptable salt thereof based on the total weight of the at least one discrete solid dosage form; 1-25 wt% of at least one sorption enhancer based on the total weight of the at least one discrete solid dosage form; and 0-5 wt% lubricant based on the total weight of the at least one discrete solid dosage form. The risperidone composition delivers a therapeutically effective amount of risperidone to a subject at a target range of about 1000 micrograms/day to about 6000 micrograms/day.

According to another embodiment of the present invention, a subcutaneous delivery system for releasing an active pharmaceutical ingredient at a pseudo-zero order comprises an elastomeric reservoir implant comprising a rate-controlling excipient defining a reservoir. The rate-controlling excipient comprises a substantially non-porous elastomeric polymer having a relative content of hard segments and soft segments to provide an elution rate within a target range of average daily elution rate for the active pharmaceutical ingredient. The reservoir containing at least one discrete solid dosage form comprising the active pharmaceutical ingredient or a pharmaceutically acceptable salt thereof and an effective amount of at least one sorption enhancer to modulate the elution rate of the active pharmaceutical ingredient for release of a therapeutically effective amount of the active pharmaceutical ingredient within the target range at pseudo-zero order for a period of time of at least one month. The amount of sorption enhancer is directly proportional to the average daily elution rate.

According to another embodiment of the present invention, a kit for subcutaneously placing a drug delivery composition includes a reservoir-based drug delivery composition comprising a rate-controlling excipient defining a reservoir containing at least one discrete solid dosage form and an implanter for inserting the reservoir-based drug delivery composition beneath the skin.

According to another embodiment of the present invention, a method of choosing an implantable drug delivery composition comprises selecting a rate-controlling excipient comprising a substantially non-porous, elastomeric polymer comprising soft and hard segments for defining a reservoir based on the relative content of soft and hard segments of the polymer to adjust the elution rate within a target range of average daily elution rate for an active pharmaceutical ingredient; and selecting and formulating the active pharmaceutical ingredient or a pharmaceutically acceptable salt thereof and at least one sorption enhancer in order to modulate the elution rate at a therapeutically effective amount of the active pharmaceutical ingredient at pseudo-zero order for a period of time of at least one month, wherein the amount of sorption enhancer is directly proportional to the average daily elution rate.

According to another embodiment of the present invention, a method of making an implantable drug delivery composition includes: (a) selecting a substantially non-porous elastomeric polymer comprising soft and hard segments based on the relative content and molecular weights of the soft and hard segments of the polymer to provide an elution rate within a target range of average daily elution rate for an active pharmaceutical ingredient; (b) forming a hollow tube from the elastomeric polymer (see *e.g.*, Figure 2); (c) selecting and formulating the active pharmaceutical ingredient or a pharmaceutically acceptable salt thereof and at least one sorption enhancer in order to produce an elution rate at a therapeutically effective amount of the active pharmaceutical ingredient at pseudo-zero order for a period of time of at least one month, wherein the amount of sorption enhancer is directly proportional to the average daily elution rate; (d) loading at least one discrete solid dosage form comprising the active pharmaceutical ingredient and the at least one sorption enhancer into the tube; and (e) sealing both ends of the tube to form a sealed cylindrical reservoir-based drug delivery composition.

According to another embodiment of the present invention, an implantable reservoir-based drug delivery composition comprises a formulation comprising risperidone or a pharmaceutically acceptable salt thereof, wherein the composition yields (i.e., is effective to provide) a therapeutically effective systemic active moiety plasma concentration for at least one month (*e.g.*, at least one month, at least three months, at least six months, at least one year, etc.) when subcutaneously administered to a subject, wherein the trough active moiety plasma concentration is not less than about 50% of the peak active moiety plasma concentration over the at least one month (*e.g.*, at least one month, at least three months, at least six months, at least one year, etc.).

Another embodiment of the present invention provides a method of treating one or more symptoms of a psychotic disorder comprising implanting a reservoir-based drug delivery composition into a subject, wherein the composition comprises a formulation comprising risperidone or a pharmaceutically acceptable salt thereof, wherein the composition yields a therapeutically effective systemic active moiety plasma concentration for at least one month (*e.g.*, at least one month, at least three months, at least six months, at least one year, etc.), wherein the trough active moiety plasma concentration is not less than about 50% of the peak active moiety plasma concentration over the at least one month (*e.g.*, at least one month, at least three months, at least six months, at least one year, etc.).

According to another embodiment of the present invention, an implantable reservoir-based drug delivery composition comprises a formulation comprising risperidone or a pharmaceutically acceptable salt thereof, wherein the composition yields (i.e., is effective to provide) a therapeutically effective systemic active moiety plasma concentration for at least one month (*e.g.*, at least one month, at least three months, at least six months, at least one year, etc.) when subcutaneously administered to a subject, wherein the peak active moiety plasma concentration over the at least one month (*e.g.*, at least one month, at least three months, at least six months, at least one year, etc.) is not more than about 1.5 times the trough active moiety plasma concentration achieved by a once-daily oral dose (e.g., a 4 mg dose) of risperidone.

Another embodiment of the present invention provides a method of treating one or more symptoms of a psychotic disorder comprising implanting a reservoir-based drug delivery composition into a subject, wherein the composition comprises a formulation comprising risperidone or a pharmaceutically acceptable salt thereof, wherein the composition yields (i.e., is effective to provide) a therapeutically effective systemic active moiety plasma concentration for at least one month (*e.g.*, at least one month, at least three months, at least six months, at least one year, etc.) when subcutaneously administered to the subject, wherein the peak active moiety plasma concentration over the at least one month (*e.g.*, at least one month, at least three months, at least six months, at least one year, etc.) is not more than about 1.5 times the trough active moiety plasma concentration achieved by a once-daily oral dose (e.g., a 4 mg dose) of risperidone.

According to another embodiment of the present invention, an implantable reservoir-based drug delivery composition comprises a formulation comprising risperidone or a pharmaceutically acceptable salt thereof, wherein the composition yields (i.e., is effective to provide) a therapeutically effective systemic active moiety plasma concentration for at least one month (*e.g.*, at least one month, at least three months, at least six months, at least one year, etc.) when subcutaneously administered to a subject, wherein the peak active moiety plasma concentration over the at least one month (*e.g.*, at least one month, at least three months, at least six months, at least one year, etc.) is not more than about 50% of the peak active moiety plasma concentration achieved by a once-daily oral dose (e.g., a 4 mg dose) of risperidone. For example, the peak active moiety plasma concentration over the at least one month may be not more than about 40% of the peak active moiety plasma concentration achieved by the once-daily oral dose (e.g., a 4 mg dose) of risperidone.

Another embodiment of the present invention provides a method of treating one or more symptoms of a psychotic disorder comprising implanting a reservoir-based drug delivery composition into a subject, wherein the composition comprises a formulation comprising risperidone or a pharmaceutically acceptable salt thereof, wherein the composition yields (i.e., is effective to provide) a therapeutically effective systemic active moiety plasma concentration for at least one month (*e.g.*, at least one month, at least three months, at least six months, at least one year, etc.), wherein the peak active moiety plasma concentration over the at least one month (*e.g.*, at least one month, at least three months, at least six months, at least one year, etc.) is not more than about 50% of the peak active moiety plasma concentration achieved

by a once-daily oral dose (e.g., a 4 mg dose) of risperidone.

According to another embodiment of the present invention, an implantable reservoir-based drug delivery composition comprises a formulation comprising risperidone or a pharmaceutically acceptable salt thereof, wherein the composition yields (i.e., is effective to provide) a therapeutically effective systemic active moiety plasma concentration for at least one month (*e.g.*, at least one month, at least three months, at least six months, at least one year, etc.) when subcutaneously administered to a subject, wherein the difference between peak and trough active moiety plasma concentrations over the at least one month (e.g., over the at least one month, at least two months, or at least three months) is at least 2 times less than (i.e., is not more than about 50% of) the difference between peak and trough active moiety plasma concentrations achieved by a once-daily oral dose (e.g., a 4 mg dose) of risperidone.

Another embodiment of the present invention provides a method of treating one or more symptoms of a psychotic disorder comprising implanting a reservoir-based drug delivery composition into a subject, wherein the composition comprises a formulation comprising risperidone or a pharmaceutically acceptable salt thereof, wherein the composition yields (i.e., is effective to provide) a therapeutically effective systemic active moiety plasma concentration for at least one month (*e.g.*, at least one month, at least three months, at least six months, at least one year, etc.) when subcutaneously administered to a subject, wherein the difference between peak and trough active moiety plasma concentrations over the at least one month (*e.g.*, at least one month, at least three months, at least six months, at least one year, etc.) is at least 2 times less than (i.e., is not more than about 50% of) the difference between peak and trough active moiety plasma concentrations achieved by a once-daily oral dose (e.g., a 4 mg dose) of risperidone (in the same subject).

According to another embodiment of the present invention, a kit for subcutaneously placing a drug-eluting implant in a subject, includes at least one drug-eluting implant and at least one insertion instrument for subcutaneously placing the at least one drug-eluting implant in a subject. In one embodiment, the insertion instrument may include a cannula having a hub and a hollow shaft, and a stop rod extending through the hub and into the hollow shaft, with the cannula being slidably displaceable over the stop rod. The hub may include a handle portion offset to one side of the longitudinal axis of the hollow shaft. The hollow shaft may include an open distal end and an interior lumen adapted to receive and store the at least one drug-eluting implant. The distal end of the hollow shaft may include a beveled tip. The stop rod may include a handle portion and an abutment face disposed within the hollow shaft. The hollow shaft may be preloaded with the at least one drug-eluting implant. The abutment face of the stop rod may be positioned inside the hollow shaft to engage the at least one drug-eluting implant inside the hollow shaft and prevent movement of the at least one drug-eluting implant in a proximal direction relative to the stop rod when the cannula is slidably displaced over the stop rod in a proximal direction relative to the stop rod. The stop rod may be axially rotatable relative to the hollow shaft between an unlocked orientation, in which the cannula can be axially displaceable over the stop rod, and a locked orientation, in which the cannula is prevented from being axially displaced over the stop rod.

A first locking feature may be provided on the stop rod and a second locking feature may be provided on the cannula. The first and second locking features may be radially aligned with one another when the stop rod is rotated to the unlocked orientation, and the first and second locking features may be radially misaligned with one another when the stop rod is rotated to the locked orientation. The first locking feature may include a longitudinal groove extending along the stop rod, and the second locking feature may include a projection that extends radially inwardly toward the horizontal axis. The groove may be adapted to receive the projection in a slidable arrangement when the stop rod is rotated to the unlocked orientation. The at least one drug-eluting implant may be packaged separately the insertion instrument. The at least one drug-eluting implant may be in the form of a polymeric rate-controlling excipient defining a reservoir containing at least one discrete solid dosage form. The at least one drug-eluting implant may include a plurality of polymeric rate-controlling excipients, each polymeric rate-controlling excipient defining a reservoir containing at least one discrete solid dosage form.

The kit may further include at least one tunneling instrument for preparing a subcutaneous cavity in a subject. In one embodiment, the tunneling instrument may include a horizontal axis corresponding to an insertion direction, and a vertical axis that is normal to the insertion direction. The tunneling instrument may also include a blade having a proximal end and a distal end, the blade having a dimension with respect to the vertical axis that gradually increases from the distal end of the blade toward the proximal end of the blade. The tunneling instrument may further include a handle having a proximal end and a distal end, the distal end of the handle attached to the proximal end of the blade. The blade may include a superior surface and an inferior surface opposite the superior surface. The inferior surface of the blade may extend between the proximal and distal ends of the blade and feature a substantially flat portion that extends parallel to the horizontal axis. The superior surface of the blade may form an inclined surface extending at an acute angle with respect to the flat portion of the inferior surface of the blade. The handle of the tunneling instrument may include a base portion and an elongated gripping portion extending from the base portion. An inferior surface of the base portion may extend substantially coplanar with the flat portion of the inferior surface of the blade to form a substantially continuous surface between the blade and the base portion. The gripping portion may extend upwardly from the base portion with respect to the vertical axis of the tunneling instrument. The gripping portion may include an overmolded grip extending over the superior surface of the gripping portion.

According to another embodiment of the present invention, a method for subcutaneously placing an drug-eluting implant in a subject includes the step of making an incision in the subject. The method may also include the step of inserting a cannula into the incision, the cannula featuring a hub and a hollow shaft, a stop rod extending through the hub and into the hollow shaft, and at least one drug-eluting implant pre-loaded inside the hollow shaft. The method may further include the step of holding the stop rod in a fixed position with respect to the subject and withdrawing the cannula from the incision and over the stop rod to deposit the at least one drug-eluting implant inside the subject. The step of inserting a cannula into the incision may include the step of inserting the cannula into subcutaneous tissue. The step of withdrawing the cannula from the incision and over the stop rod to deposit the at least one drug-eluting implant inside the patient may include the step of partially removing the hollow shaft of the cannula from the incision. Alternatively, the step of withdrawing the cannula from the incision and over the stop rod to deposit the at least one drug-eluting implant inside the patient may include the step of completely removing the hollow shaft of the cannula from the incision. The step of withdrawing the cannula from the incision and over the stop rod to deposit the at least one drug-eluting implant inside the patient may further include the step of displacing the drug-eluting implant relative to the stop rod until the drug-eluting implant abuts a distal end of the stop rod.

The method may also include the step of preparing a subcutaneous cavity adjacent the incision prior to the step of inserting a cannula into the incision. The step of preparing a subcutaneous cavity adjacent the incision includes the step of inserting a blade into the incision and advancing the blade into subcutaneous tissue to create an elongated tunnel by blunt dissection of the tissue immediately beneath the cutis of the subject. The method may further include the step of moving the stop rod from a locked position to an unlocked position, prior to the step of withdrawing the cannula from the incision and over the stop rod to deposit the at least one drug-eluting implant inside the patient. The step of withdrawing the cannula from the incision and over the stop rod to deposit the at least one drug-eluting implant inside the patient may include the step of grasping a handle portion on the hub of the cannula and applying a force on the handle portion in a direction away from the incision. Moreover, the step of withdrawing the cannula from the incision and over the stop rod to deposit the at least one drug-eluting implant inside the subject may include the step of releasing only a single drug-eluting implant from the cannula and depositing the single drug-eluting implant into the subject. Alternatively, the step of withdrawing the cannula from the incision and over the stop rod to deposit the at least one drug-eluting implant inside the subject may include the step of releasing a plurality of drug-eluting implants from the cannula and depositing the plurality of drug-eluting implants into the subject.

## BRIEF DESCRIPTION OF THE DRAWINGS

The invention may be further understood by reference to the drawings in which:

Figure 1 depicts the role of the excipient in a reservoir-based drug delivery composition according to one aspect of the present invention;

Figure 2 depicts the cylindrical shape of a reservoir-based drug delivery composition according to one embodiment of the present invention;

Figure 3 depicts the difference between a drug reservoir and a matrix-based implant;

Figure 4 is a graph showing the elution rate (μg/day) of anastrozole from an aliphatic, polyether-based urethane implant over about 400 days, according to an embodiment of the present invention described in Example 2;

Figure 5 is a graph showing the elution rate (μg/day) of anastrozole from an aliphatic, polycarbonate-based urethane implant over about 100 days, according to an embodiment of the present invention described in Example 3;

Figure 6 is a graph showing the plasma concentration (ng/mL) of anastrozole as eluted from an aliphatic, polyether-based urethane in beagle dogs, according to an embodiment of the present invention described in Example 4;

Figure 7 is a graph of the *in vitro* elution of anastrozole from aliphatic, polycarbonate-based urethane implants containing 0 wt% and 10 wt% croscarmellose sodium, according to embodiments of the present invention described in Example 5;

Figure 8 is a graph showing the *in vitro* elution of anastrozole from an aliphatic, polyether-based urethane implant over about 400 days, according to an embodiment of the present invention;

Figure 9 is a graph comparing the plasma concentration (ng/mL) of anastrozole from an oral dosage form and an

implant according to an embodiment of the present invention;

Figure 10 is a graph showing the elution rate (μg/day) of anastrozole from implants according to embodiments of the invention having differing quantities of pellets within the reservoir;

Figure 11 is a graph showing the elution rate (μg/day) of anastrozole from a polyurethane-based implant according to an embodiment of the invention having a polycarbonate soft segment;

Figure 12 is a graph showing the elution rate (μg/day) of anastrozole from polyether-block polyamide polymers according to embodiments of the invention having varying Shore hardness values;

Figure 13 is a graph showing the elution rate (μg/day) of anastrozole from implants according to embodiments of the invention having varying amounts of a sorption enhancer;

Figure 14 is a graph showing the elution rate (μg/day) of anastrozole from implants according to embodiments of the invention containing different types of sorption enhancers;

Figure 15 is a graph showing the elution rate (μg/day) of letrozole from implants according to embodiments of the invention containing different grades of TECOFLEX® polyurethane for the excipient;

Figure 16 is a graph showing the elution rate (μg/day) of anastrozole from an implant according to an embodiment of the invention containing a specific CARBOTHANE® polyurethane for the excipient;

Figure 17 is a graph showing the elution rate (μg/day) of anastrozole from an implant according to an embodiment of the invention containing an ELAST-EON™ polyurethane for the excipient;

Figure 18 is a graph showing the elution rate (μg/day) of anastrozole from implants according to embodiments of the invention containing different numbers of pellets and corresponding different API loading;

Figure 19 is a graph showing the elution rate (μg/day) of anastrozole from implants according to embodiments of the invention containing different types of sorption enhancers;

Figure 20 is a graph showing the elution rate (μg/day) of anastrozole from implants according to embodiments of the invention containing CARBOTHANE® polyurethane as the excipient and varying the amount of sorption enhancer as set forth in Example 13;

Figure 21 is a graph showing the elution rate (μg/day) of anastrozole from implants according to embodiments of the invention containing varying wall thicknesses as set forth in Example 14;

Figure 22 is a graph showing the elution rate (μg/day) of anastrozole from implants according to embodiments of the invention containing varying amounts of sorption enhancer as set forth in Example 15; and

Figure 23 is a graph showing the elution rate (μg/day) of risperidone from an implant over about 40 days, according to an embodiment of the present invention described in Example 16.

Figure 24 is a graph showing the elution rate (μg/day) of anastrozole from implants according to embodiments of the invention containing varying amounts of croscarmellose sodium as set forth in Example 17;

Figure 25 is a graph showing the elution rate (μg/day) of anastrozole from implants according to embodiments of the invention containing varying amounts of sodium polyacrylate as set forth in Example 18;

Figure 26 is a graph showing the elution rate (μg/day) of risperidone from implants according to embodiments of the invention containing varying amounts of croscarmellose sodium as set forth in Example 19;

Figure 27 is a graph showing the elution rate (μg/day) of risperidone from implants according to embodiments of the invention containing polyethylene oxide, sodium polyacrylate, or chondroitin sulfate as sorption enhancers, as set forth in Example 20;

Figure 28 is a graph showing the elution rate (μg/day) of risperidone from an implant according to embodiments of the invention containing carboxymethyl cellulose, as set forth in Example 21;

Figure 29 is a graph showing the elution rate (μg/day) of guanfacine from implants according to embodiments of the invention containing PEBAX® 2533 or PEBAX® 3533 as the excipient, as set forth in Example 23;

Figure 30 is a graph showing the elution rate (μg/day) of paliperidone from implants according to embodiments of the invention containing PEBAX® 2533 or PEBAX® 3533 as the excipient, as set forth in Example 24;

Figure 31 is a graph showing the elution rate (μg/day) of letrozole from implants according to embodiments of the invention containing PEBAX® 2533 or PEBAX® 3533 as the excipient, as set forth in Example 25;

Figure 32 is a graph showing the elution rate (μg/day) of fingolimod free base and fingolimod HCl from implants according to embodiments of the invention as set forth in Example 27;

Figure 33 is a graph showing the elution rate (μg/day) of varenicline tartrate from implants according to embodiments of the invention as set forth in Example 28;

Figure 34 is a graph showing the elution rate (μg/day) of varenicline free base from an implant according to embodiments of the invention as set forth in Example 29;

Figure 35 is a graph showing the elution rate (μg/day) of oxybutynin HCl from implants according to embodiments of the invention as set forth in Example 30;

Figure 36 is a graph showing the elution rate (μg/day) of oxybutynin free base from implants according to embodiments of the invention as set forth in Example 31;

Figure 37 is a graph showing the elution rate (μg/day) of risperidone from an implant according to embodiments of the invention as set forth in Example 32;

Figure 38 is a graph showing the mean risperidone active moiety plasma concentration (ng/mL) for six subjects over 24 hours, following oral administration of 4 mg risperidone as set forth in Example 33;

Figure 39 is a graph showing the mean risperidone, 9-OH-risperidone, and total active moiety (i.e., risperidone + 9-OH-risperidone) plasma concentrations (ng/mL) for six subjects over 90 days, following subcutaneous delivery of an implant according to embodiments of the invention, as set forth in Example 33;

Figure 40 shows graphs of (i) the mean risperidone active moiety plasma concentration (ng/mL) for six subjects over 24 hours, following oral administration of 4 mg risperidone, and (ii) the mean risperidone active moiety (i.e., risperidone + 9-OH-risperidone) plasma concentrations (ng/mL) for six subjects over 90 days, following subcutaneous delivery of an implant according to embodiments of the invention, as set forth in Example 33;

Figure 41 is a perspective view of a kit for subcutaneously placing a drug-eluting implant in a subject according to embodiments of the invention;

Figure 42 is a perspective view of an insertion instrument used in the kit of Figure 41;

Figure 42A is a cross-sectional view about section line A-A in Figure 42;

Figure 43 is another perspective view of the insertion instrument of Figure 41;

Figure 44 is a distal end view of the insertion instrument of Figure 41;

Figure 45 is a proximal end view of the insertion instrument of Figure 41;

Figure 46 is a side elevation view of the insertion instrument of Figure 41;

Figure 47 is another side elevation view of the insertion instrument of Figure 41;

Figure 48 is a top plan view of the insertion instrument of Figure 41;

Figure 49 is a bottom plan view of the insertion instrument of Figure 41;

Figure 50 is a cross-sectional view about section line B-B in Figures 43 and 48 of the insertion instrument of Figure 41;

Figure 51 is a perspective view of another kit for subcutaneously placing a drug-eluting implant in a subject, according to another aspect of the invention;

Figure 52 is a side elevation view of a tunneling instrument used in the kit of Figure 51;

Figure 53 is another side elevation view of the tunneling instrument of Figure 51;

Figure 54 is a perspective view of the tunneling instrument of Figure 51;

Figure 55 is another perspective view of the tunneling instrument of Figure 51;

Figure 56 is a top plan view of the tunneling instrument of Figure 51;

Figure 57 is a bottom view of the tunneling instrument of Figure 51;

Figure 58 is a cross-sectional view about section line C-C in Figures 55 and 56 of the tunneling instrument of Figure 51;

Figure 59 is a distal end view of the tunneling instrument of Figure 51;

Figure 60 is a proximal end view of the tunneling instrument of Figure 51;

Figure 61 is a graph showing the mean anastrozole plasma concentrations (pg/mL) over time, following subcutaneous delivery of an implant according to embodiments of the invention, as set forth in Example 34; and

Figure 62 is a graph showing the mean estradiol plasma concentrations (pg/mL) over time, following subcutaneous delivery of an implant according to embodiments of the invention, as set forth in Example 34.

## DETAILED DESCRIPTION OF THE INVENTION

Aspects of the present invention include methods of treatment, such as methods of treating an estrogen-related disorder, such as breast cancer, methods of treating a psychotic disorder, such as schizophrenia; methods of delivering an active pharmaceutical agent from an implantable composition in a therapeutically effective amount, such as delivering an aromatase inhibitor or risperidone to a patient; reservoir-based drug delivery compositions; subcutaneous delivery systems; and kits for subcutaneous delivery.

As used herein, the term "therapeutically effective amount" refers to those amounts that, when administered to a particular subject in view of the nature and severity of that subject's disease or condition, will have a desired therapeutic effect, *e.g.,* an amount which will cure, prevent, inhibit, or at least partially arrest, delay the onset of or partially prevent a target disease or condition or one or more symptoms thereof.

The terms "active pharmaceutical ingredient," "API," "drug," or "active" may be used herein interchangeably to refer to the pharmaceutically active compound(s) in the drug delivery composition. This is in contrast to other ingredients in the drug delivery composition, such as excipients, which are substantially or completely pharmaceutically inert. A suitable API in accordance with the present invention is one where there is or likely may be patient compliance issues for treating a certain disease or condition.

The term "pharmaceutically acceptable," as used herein, means approved by a regulatory agency, *e.g.* of the U.S. Federal or a state government or listed in the U.S. Pharmacopeia or other generally recognized pharmacopeia for use in animals, and more particularly in humans.

The terms "subject" and "patient", are used interchangeably herein and refer to a mammalian individual, such as a human being.

Each compound used herein may be discussed interchangeably with respect to its chemical formula, chemical name, abbreviation, etc. For example, PTMO may be used interchangeably with poly(tetramethylene oxide). Additionally, each polymer described herein, unless designated otherwise, includes homopolymers, copolymers, terpolymers, and the like.

As used herein and in the claims, the terms "comprising" and "including" are inclusive or open-ended and do not exclude additional unrecited elements, compositional components, or method steps. Accordingly, the terms "comprising" and "including" encompass the more restrictive terms "consisting essentially of" and "consisting of." Unless specified otherwise, all values provided herein include up to and including the endpoints given, and the values of the constituents or components of the compositions are expressed in weight percent of each ingredient in the composition.

Treatment of an Estrogen-Related Disorder

Treatment of an estrogen-related disorder or estrogen-receptor disorder may include treatment of any estrogen-related or estrogen-receptor disorders, diseases, or conditions known to one of ordinary skill in the art, such as breast cancer, endometriosis, uterine fibroids (also called leiomyomas), short stature in children or adolescents and the like. Estrogen-related disorders may include high estrogen levels or normal estrogen levels that need to be reduced. Estrogen-receptor disorders may include estrogen receptors positive (ER+ ) and/or progesterone receptors positive (PR+) disorders.

In one embodiment of the present invention, the estrogen-related disorder is breast cancer. As previously discussed, the treatment of breast cancer can require long-lasting treatment, often on the order of many years, and compliance with breast cancer medications has been an ongoing issue. The treatment of breast cancer in accordance with the present invention is directed to adjunctive therapy (used with or after primary treatment, such as chemotherapy). The treatment is especially suitable for patients that are either or both hormone receptor positive (estrogen and/or progesterone receptors positive) and post-menopausal (the period of time after menopause).

By "treatment," it is intended that a pharmaceutically effective amount of an aromatase inhibitor would be administered via the drug delivery composition, which will inhibit, or at least partially arrest or partially prevent or suppress estrogen. For example, treatment may include treatment that can suppress or delay the recurrence of breast cancer. The treatment is particularly effective in that once the implant is administered to the patient, the patient will continue to receive a therapeutically effective dose for the intended duration of the implant (*e.g.*, one year). This is in contrast to the oral dose, which requires compliance by the patient and continued oral administration consistently over the same duration of time. The treatment is especially beneficial for younger (*e.g.*, <40 years) and older (*e.g.*, >75 years) women who are at a heightened risk for non-compliance and also for those who may be at a greater risk for recurrence of breast cancer.

According to one aspect of the present invention, a method of treating an estrogen-related disorder, such as breast cancer, for example, comprises implanting a reservoir-based drug delivery composition into a subject to systemically deliver a therapeutically effective amount of an aromatase inhibitor to the subject for a period of time of at least one month, etc. The drug delivery composition comprises at least one discrete solid dosage form comprising at least one aromatase inhibitor surrounded by an excipient comprising at least one polymer.

In another embodiment of the present invention, the estrogen-related disorder is short stature in children or adolescents. Estrogens have been found to be important for bone maturation, growth plate fusion, and cessation of longitudinal growth in children and adolescents. The rate of linear growth accelerates during puberty, followed by deceleration and cessation of growth. Clinical findings in patients with estrogen insensitivity or estrogen deficiency suggest that estrogen is essential for these changes in puberty. It is believed that prolonging the period of growth during puberty by diminishing the biological action of estrogen, and thereby delaying the senescence of the growth plate, can increase adult height. Thus, by blocking estrogen biosynthesis in males with the use of aromatase inhibitors, it is believed that bone maturation can be delayed in order to improve the subject’s final height. Short stature is a condition for which aromatase inhibitors (e.g., anastrozole or letrozole) has been beneficial in reducing clinical signs of estrogenization and/or estrogen-mediated skeletal maturation.

According to one aspect of the present invention, a method of treating short stature in a child or adolescent (e.g., a male child or adolescent) comprises implanting a reservoir-based drug delivery composition into a subject to systemically deliver a therapeutically effective amount of an aromatase inhibitor (e.g., anastrozole or letrozole) to the subject for a period of time of at least one month, at least 2 months, at least 3 months, etc. The drug delivery composition comprises at least one discrete solid dosage form comprising at least one aromatase inhibitor (e.g., anastrozole or letrozole) surrounded by an excipient comprising at least one polymer. According to preferred embodiments, treating short stature in a child or adolescent (e.g., a male child or adolescent) with an aromatase inhibitor is effective in increasing the predicted adult height of the subject. A subject’s predicted adult height can be determined by examining the subject’s bone age according to known methods.

According to another aspect of the present invention, a method of systemically delivering an aromatase inhibitor to a subject includes releasing a therapeutically effective amount of an aromatase inhibitor from a reservoir-based composition comprising a polymeric rate-controlling excipient defining a reservoir containing at least one discrete solid dosage form comprising at least one aromatase inhibitor to provide a pseudo-zero order elution rate to the subject for a period of time of at least one month.

Treatment of the Symptoms of a Psychotic Disorder

The treatment of the symptoms of a psychotic disorder (such as schizophrenia, bipolar disorder, or autism) can require long lasting treatment, often on the order of many years, even for the life of the patient. Compliance with antipsychotic medications has also been an ongoing issue. The treatment of a psychotic disorder in accordance with the present invention may be directed to men or women. By "treatment," it is intended that a pharmaceutically effective amount of risperidone would be administered via the drug delivery composition, which will cure, prevent, inhibit, or at least partially arrest or partially prevent or suppress the symptoms of the psychotic disorder. The treatment is particularly effective in that once the implant is administered to the patient, the patient will continue to receive a therapeutically effective dose for the intended duration of the implant (*e.g.*, one year). This is in contrast to the oral dosage form, which requires compliance by the patient and continued oral administration consistently over the same duration of time.

As used herein, the terms "psychotic disorder" or "psychosis" may be used interchangeably to refer to a disorder in which psychosis is a recognized symptom. The symptoms of psychosis may include, but are not limited to, hallucinations, delusions, paranoia, mania, depression, emotional changes, personality changes, behavioral changes, and lack of awareness of mental changes. The term "antipsychotic" refers to drugs used to treat psychosis. Common conditions for which antipsychotics are prescribed include schizophrenia, mania, and delusional disorders. Antipsychotics also act as mood stabilizers making them suitable for the treatment of bipolar disorder (even when no symptoms of psychosis are present).

The psychotic disorder may include, for example, schizophrenia, bipolar disorder, autism, or any variations thereof. Schizophrenia may include various types including paranoid subtype, disorganized subtype, catatonic subtype, undifferentiated subtype, or residual subtype. For example, the paranoid subtype (also known as paranoid schizophrenia) may include the presence of auditory hallucinations or prominent delusional thoughts about persecution or conspiracy. Bipolar disorder may include different versions including bipolar disorder I, bipolar disorder II, cyclothymic disorder, bipolar disorder not otherwise specified (NOS), or bipolar disorder with rapid cycling. For example, bipolar disorder I may be characterized by at least one manic episode or mixed episode (symptoms of both mania and depression occurring simultaneously), and one or more depressive episodes, that lasts for at least 7 days. Autism includes developmental disorders that appear in the first 3 years of life and affects the brain's normal development of social and communication skills.

In one embodiment of the present invention, a method of treating the symptoms of a psychotic disorder comprises implanting a reservoir-based drug delivery composition into a subject to systemically deliver a therapeutically effective amount of risperidone or a pharmaceutically acceptable salt thereof to the subject at a pseudo-zero order rate for a period of time of at least one month. The drug delivery composition comprises at least one discrete solid dosage form comprising risperidone or a pharmaceutically acceptable salt thereof and at least one sorption enhancer surrounded by an excipient comprising an aliphatic polyether-based polyurethane or a polyether-amide. An average daily elution rate of the risperidone or a pharmaceutically acceptable salt thereof varies in direct proportion to the amount of sorption enhancer in the drug delivery composition.

According to another embodiment, a risperidone composition comprises a drug elution rate-controlling excipient consisting of an aliphatic polyether-based polyurethane comprising poly(tetramethylene oxide) and polymerized 4,4'-diisocyanato dicyclohexylmethane (H12MDI) and 1,4-butanediol defining a reservoir. The reservoir contains at least one discrete solid dosage form comprising 75-97 wt% risperidone or a pharmaceutically acceptable salt thereof based on the total weight of the at least one discrete solid dosage form; 1-25 wt% of at least one sorption enhancer based on the total weight of the at least one discrete solid dosage form; and 0-5 wt% lubricant based on the total weight of the at least one discrete solid dosage form. The risperidone composition delivers a therapeutically effective amount of risperidone to a subject at a target range of about 1000 micrograms/day to about 6000 micrograms/day.

Reservoir-Based Drug Delivery Composition

The drug delivery composition is a reservoir-based drug delivery composition. As used herein, the "reservoir-based composition" is intended to encompass a composition having a substantially or completely closed, surrounded, or encased hollow space or reservoir, where the hollow space or reservoir is filled, at least partially, with at least one discrete solid dosage form.

In one embodiment of the present invention, a drug delivery composition comprises a drug elution rate-controlling excipient comprising an elastomeric polymer defining a reservoir, and the reservoir contains at least one discrete solid dosage form comprising at least one API.

A reservoir-based composition, as used herein, is in contradistinction to a matrix-based composition. As depicted in Figure 3, a drug reservoir includes a reservoir portion 120 and a rate controlling portion (excipient 110) whereas a matrix-based implant only consists of the matrix material 130 with the drug incorporated therein. In other words, in a reservoir system, the drug is contained within or is surrounded by some type of rate-controlling material (e.g., a wall,

membrane, or casing). In a matrix system, the drug is combined within some type of matrix, often polymeric, which often erodes or degrades in order to release the active to the subject.

Thus, there are some major distinctions between the two types of systems. The reservoir-based system allows for a much higher drug loading (*e.g.*, on the order of 98% maximum) whereas a matrix-based system contains a much smaller amount (*e.g.,* on the order of 25% maximum). Although a higher drug loading may be beneficial, it can also be dangerous because of the increased risk of drug overdose or dumping into the subject if the surrounding material were to break or rupture. Additionally, the reservoir-based composition of the present invention allows for a zero-order or pseudo-zero order rate of release of the active. A matrix-based system, on the contrary, provides for a first order rate of release. A first order rate may be characterized by a high initial rate of release that decays or diminishes quickly over time.

As used herein, the term "pseudo-zero order" or "pseudo-zero order rate" refers to a zero-order, near-zero order, substantially zero order, or controlled or sustained release of an API. A zero order release profile may be characterized by release of a constant amount of the API per unit time. A pseudo-zero order release profile may be characterized by approximating a zero-order release by release of a relatively constant amount of the API per unit time (*e.g.*, within 40%, 30%, 20%, or 10% of the average value). Under a pseudo-zero order rate, the composition may initially release an amount of the API that produces the desired therapeutic effect, and gradually and continually release other amounts of the API to maintain the level of therapeutic effect over an extended period of time (*e.g.*, at least one month, six months, or one year). In order to maintain a near-constant level of API in the body, the API may be released from the composition at a rate that will replace the amount of API being metabolized and/or excreted from the body. It will be appreciated by one of ordinary skill in the art that there may be some initial period of time before steady state is reached (*e.g.*, a ramp up before the target range is reached), which still complies with the definition of "pseudo-zero order."

Without wishing to be bound to a particular theory, it is believed that a concentration gradient occurs where the concentration of API within the reservoir is "infinite" (*e.g.*, the reservoir acts an infinite supply, but the concentration is practically limited by the amount of active for the given duration of release) and the concentration outside the drug delivery composition is zero (*e.g.*, the subject acts as an infinite sink where the active is constantly being taken away from the composition by the subject's body, such as circulatory, lymphatic systems, etc.). Additionally, the excipient 110 (*e.g.*, the wall through which the active passes) becomes fully saturated with the active ingredient at steady state. Accordingly, this gradient allows the "infinite" supply of API to be adsorbed into the excipient, dissolve in and diffuse through the polymer wall, and then be desorbed for release into the subject. The selection of the excipient 110 may help to provide the pseudo-zero order release of the drug. Without wishing to be limited, it is believed that the release of the drug is not dependent on the desorption from the excipient.

Dosage Form(s)

The drug delivery composition comprises at least one dosage form comprising at least one API. In one embodiment of the present invention, the drug delivery composition comprises at least one discrete solid dosage form comprising at least one API surrounded by an excipient comprising at least one polymer.

As used herein, the term "discrete solid dosage form" is intended to encompass any dosage form that is in the form of a solid. The solid dosage form may include any cohesive solid form (*e.g.*, compressed formulations, pellets, tablets, etc.) The solid dosage form may include a solid body or mass comprising the API, which may be prepared in any suitable manner known to one of ordinary skill in the art (*e.g.*, compressed, pelleted, extruded).

The solid dosage forms are "discrete" in that there are one or more dosage forms contained within the reservoir. In other words, the discrete solid dosage form includes one or more solid formulations which are separate and distinct from the polymeric rate-controlling excipient. In an exemplary embodiment, the discrete solid dosage form(s) do not fill the entire reservoir or cavity (*e.g.*, the solid dosage forms are substantially spherical and the reservoir is substantially cylindrical). For example, the solid dosage form need not be co-extruded with the surrounding excipient such that the solid dosage form fills the entire cavity.

The discrete solid dosage forms may be of any suitable shape and of any suitable quantity. In one embodiment of the present invention, the discrete solid dosage forms are substantially spherical in shape. The discrete solid dosage form(s) may be "substantially spherical" in that the solid dosage forms are spherical or nearly spherical in that the length of the longest radius is approximately equal to the shortest radius of the dosage form. For example, the shape of the dosage form may not deviate from a perfect sphere by more than about 10%. In another embodiment, the discrete solid dosage forms comprise more than one pellet (*e.g.*, 2-9 pellets). The number of discrete solid dosage forms may be proportional to the elution rate. In other words, a higher number of dosage forms may result in a higher average elution rate than a smaller number of dosage forms. Thus, it may be preferable to include more discrete solid dosage forms to give a higher elution rate (*e.g.*, 7-9 pellets).

The discrete solid dosage form may comprise one or more active pharmaceutical ingredients. A suitable API in accordance with the present invention is one where there is or likely may be compliance issues for treating a certain disease or condition.

In one embodiment, the discrete solid dosage form comprises at least one aromatase inhibitor. As used herein, "aromatase inhibitors" include substances that inhibit the enzyme aromatase (estrogen synthetase), which is responsible for converting androgens to estrogens. The aromatase inhibitors may have a steroidal or non-steroidal chemical structure. Any suitable aromatase inhibitor may be selected by one of ordinary skill in the art. Particularly suitable aromatase inhibitors may include, for example, anastrozole, letrozole, exemestane, pharmaceutically acceptable salts, and combinations thereof. In an exemplary embodiment, the aromatase inhibitor is anastrozole or a pharmaceutically acceptable salt thereof. The amount of aromatase inhibitor is not particularly limited, but may be preferably on the order of about 75-97 wt% of the solid dosage form or 85-95 wt% of the solid dosage form (*e.g.*, about 88 wt%).

In another embodiment, the discrete solid dosage form comprises risperidone, and optionally, other active pharmaceutical ingredient(s). Risperidone is an antipsychotic agent also known as 4-[2-[4-(6-fluorobenzo[d]isoxazol-3-yl)-1-piperidyl]ethyl]-3-methyl-2,6-diazabicyclo[4.4.0]deca-1,3-dien-5-one and may have the following general formula:

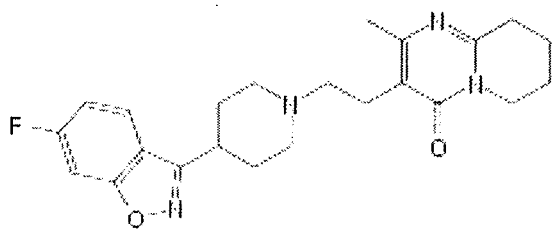

Reference to "risperidone" herein may include risperidone *per se,* active metabolites thereof (*e.g.*, 9-hydroxy-risperidone (paliperidone)), and/or pharmaceutically acceptable salts thereof. The amount of risperidone is not particularly limited, but may be preferably on the order of about 75-97 wt% of the solid dosage form or 85-95 wt% of the solid dosage form (*e.g.*, about 88 wt%).

The discrete solid dosage form may also be administered with at least one other active pharmaceutical ingredient(s). In the case of risperidone, for example, administration of other actives may improve or enhance the treatment of the psychotic disorder. In the case of treatment of bipolar disorder (such as bipolar disorder I), for instance, the at least one other active pharmaceutical ingredient may comprise lithium or valproate.

The discrete solid dosage form may also comprise a sorption enhancer. As used herein, the term "sorption enhancer" is intended to encompass compounds which improve release of the API from the drug delivery composition. Without wishing to be bound to a particular theory, the sorption enhancers may improve release of the API from the drug delivery composition by drawing water or other fluids into the reservoir from the subject, disintegrating or breaking apart the discrete solid dosage form(s), and/or allowing the API to come into contact or remain in contact the inner walls of the excipient. Such a mechanism may be depicted, for example, in Figure 1. Figure 1 represents the rate-controlling excipient 110. The API, located in the reservoir on the left side of the diagram, is sorbed 112 from the reservoir to the excipient. The API then crosses through the excipient 110. The API is then desorbed 114 from the excipient into the subject.

Any suitable sorption enhancer(s) may be selected by one of ordinary skill in the art. Particularly suitable sorption enhancer(s) may include, for example, negatively-charged polymers, such as croscarmellose sodium, sodium carboxymethyl starch, sodium starch glycolate, sodium acrylic acid derivatives, chondroitin sulfate, polyglutamic acid, polyaspartic acid, and combinations thereof. In an exemplary embodiment, the sorption enhancer is croscarmellose sodium. The amount of the sorption enhancer may be present on the order of about 1-25 wt% of the solid dosage form, about 2-20 wt% of the solid dosage form, about 2-12 wt% of the solid dosage form, about 5-10 wt% of the solid dosage form (*e.g.*, about 5 wt% or about 10 wt% of the solid dosage form).

The amount of sorption enhancer may be proportional to the elution rate. In other words, a higher weight percent of sorption enhancer in the drug composition may result in a higher average elution rate than a smaller weight percentage. Thus, it may be preferable to include a higher weight percent of sorption enhancer to give a higher elution rate (*e.g.*, 8-25 wt%).

The discrete solid dosage form may also comprise other ingredients as long as they do not adversely impact the elution rate. Other suitable ingredients may include, for example, lubricants, excipients, preservatives, etc. A lubricant may be used in the pelleting or tableting process to form the discrete solid dosage form(s), as would be well known by one of ordinary skill in the art. Suitable lubricants may include, but are not limited to, magnesium stearate, calcium stearate, zinc stearate, stearic acid, polyethylene glycol, and the like. The amount of any additional ingredients is not particularly limited, but is preferably on the order of less than about 5 wt% of the solid dosage form, and most preferably less than about 3 wt% of the solid dosage form, particularly preferably about 2% or less of the solid dosage form.

In one embodiment of the present invention, the at least one discrete solid dosage form comprises: 75-97 wt% aromatase inhibitor (*e.g.*, anastrozole) based on the total weight of the at least one discrete solid dosage form; 1-25 wt% of at least one sorption enhancer based on the total weight of the at least one discrete solid dosage form; and 0-5

wt% lubricant based on the total weight of the at least one discrete solid dosage form. For example, 85-95 wt% aromatase inhibitor based on the total weight of the at least one discrete solid dosage form; 5-20 wt% of croscarmellose sodium based on the total weight of the at least one discrete solid dosage form; and 0-5 wt% stearic acid based on the total weight of the at least one discrete solid dosage form. Preferably, each component of the drug delivery composition is provided in an amount effective for the treatment of an estrogen-related disorder, such as breast cancer.

In another embodiment of the present invention, the at least one discrete solid dosage form comprises: 75-97 wt% risperidone or a pharmaceutically acceptable salt thereof based on the total weight of the at least one discrete solid dosage form; 1-25 wt% of at least one sorption enhancer based on the total weight of the at least one discrete solid dosage form; and 0-5 wt% lubricant based on the total weight of the at least one discrete solid dosage form. For example, 85-95 wt% (*e.g.*, 88 wt%) risperidone based on the total weight of the at least one discrete solid dosage form; 5-20 wt% (*e.g.,* 10 wt%) of croscarmellose sodium based on the total weight of the at least one discrete solid dosage form; and 0-5 wt% (*e.g.,* 2 wt%) stearic acid based on the total weight of the at least one discrete solid dosage form. According to another embodiment, the at least one discrete solid dosage form comprises about 89.25% risperidone, about 10% croscarmellose sodium, and about 0.75% magnesium stearate. Preferably, each component of the drug delivery composition is provided in an amount effective for the treatment of a psychotic disorder.

<u>Excipient</u>

The discrete solid dosage form(s) is/are surrounded by an excipient. In other words, the discrete solid dosage form(s) is/are substantially or completely surrounded, encased, or enclosed by the excipient. In the present invention, there are no holes or pores in the excipient to allow egress of the API or ingress of bodily fluids, unlike an osmotic system, which requires a hole to allow release of the API. Moreover, there is no (or negligible) build up of pressure within a drug delivery composition in accordance with the present invention, unlike an osmotic system, which requires pressure to force the API out of the device.

In one embodiment of the present invention, the excipient is substantially or completely non-porous. "Substantially nonporous" may refer to a material which has a porosity or void percentage less than about 10%, about 5%, or about 1%, for example. In particular, the excipient is substantially non-porous in that there are no physical pores or macropores, which would allow for egress of the API from the drug delivery composition. In another embodiment, the excipient is practically insoluble in water. Solubility is the concentration of a solute when the solvent has dissolved all the solute that it can at a given temperature (*e.g.*, the concentration of solute in a saturated solution at equilibrium). As used herein, the term "practically insoluble in water" is consistent with the definition in The United States Pharmacopeia - National Formulary (USP-NF) definition, which provides for more than 10,000 parts solvent to one part solute (*e.g.*, one gram of the excipient in greater than 10,000 mL of water).

Without wishing to be bound to a particular theory, it is believed that a concentration gradient across the excipient (*e.g.*, wall, membrane, layer) allows for continuous release of the API. As depicted in Figure 1, sorption 112 of the API occurs from the reservoir onto the rate-controlling excipient 110. The API then dissolves into and fully saturates the excipient 110, diffuses through it, and the API is then desorbed 114 from the excipient into the subject. Accordingly, this gradient allows the "infinite" supply of API to be adsorbed onto the excipient, diffuse through it and desorbed into the subject, which, based on the excipient selected, may help to provide the pseudo-zero order release of the drug. Thus, the excipient may also be called a drug elution rate-controlling or rate-controlling excipient herein. The "rate-controlling excipient" is intended to encompass materials which control the elution rate of the API. In other words, a polymeric excipient, that when encasing the drug delivery composition, provides a different rate of release, namely, a controlled rate of release (*e.g.*, pseudo-zero order) as compared to the release of an API from an identical composition without a rate-controlling excipient.

The excipient defines the shape of the reservoir. The reservoir may be of any suitable size and shape. In an exemplary embodiment, the excipient is substantially cylindrically shaped. As used herein, the terms "cylindrical" or "cylindrically shaped" may be used interchangeably to mean at least substantially having the shape of a cylinder. As used herein, the term "cylinder" includes and refers to, but is not limited to: circular cylinders, having a circular cross-section; elliptical cylinders, having an elliptical cross-section; generalized cylinders, having any shape in cross-section; oblique cylinders, in which the end surfaces are not parallel to one another and/or are not normal to the axis of the cylinder; and conical and frusto-conical analogs thereof. In accordance with one aspect of the invention, a hollow tube may include a substantially consistent cross-sectional area and two substantially equally-sized circular ends. The cylindrical shape defines the shape of the excipient defining the reservoir (e.g., the outer portion of the drug delivery composition). An embodiment of the cylindrically shaped excipient is depicted, for example, in Figure 2. Preferably, the dimensions of the cylindrical hollow tube should be as precise as possible (*e.g.*, a consistent shape and dimension along the length of the tube, in particular, a consistent circular cross-section). The reservoir may be of any suitable size depending on the active and location of delivery. For example, the composition may range in size from about 2mm to about 4mm in diameter (*e.g.*, about 2.7 mm in diameter) and about 6mm to about 50mm in length, for example about

45 mm in length.

The excipient comprises at least one polymer. Any suitable polymer may be selected by one of ordinary skill in the art, as long as the polymer allows for delivery of a therapeutically effective amount of the API to the subject, for example, at a pseudo-zero order rate, for the intended period of time that the implant resides in a patient. In one embodiment, the polymer comprises a thermoplastic elastomer. As used herein, "thermoplastic," "thermoplastic elastomers (TPE)" or "thermoplastic rubbers" may be used to denote a class of copolymers or a physical mix of polymers (*e.g.*, a plastic and a rubber), which consist of materials with both thermoplastic and elastomeric properties. The crosslinking in thermoplastic elastomeric polymers may include a weaker dipole or hydrogen bond or the crosslinking occurs in one of the phases of the material. The class of copolymer may include, for example, styrenic block copolymers, polyolefin blends, elastomeric alloys, thermoplastic polyurethanes, thermoplastic copolyester, and thermoplastic polyamides.

As used herein, "elastomer" or "elastomeric polymer" is intended to encompass polymers (homopolymers, copolymers, terpolymers, oligomers, and mixtures thereof) having elastomeric properties (*e.g.*, the tendency to revert to its original shape after extension). In other words, the polymeric backbone may contain one or more elastomeric subunits (*e.g.*, an elastomeric soft segment or block). In one embodiment, the elastomeric polymer comprises polyurethane, polyether, polyamide, polycarbonate, polysilicone, or copolymers thereof. Thus, the elastomeric polymer may include polyurethane-based polymers, polyether-based polymers, polysilicone-based polymers, polycarbonate-based polymers, or combinations thereof.

The polymer may be formed by any suitable means or techniques known to one of ordinary skill in the art. For example, the polymer may be formed from monomers, polymer precursors, pre-polymers, polymers, etc. Polymer precursors may include monomeric as well as oligomeric substances capable of being reacted or cured to form polymers. The polymers may be synthesized using any suitable constituents.

In one embodiment of the present invention, the polymer comprises polyurethanes (*e.g.*, comprising a urethane linkage, -RNHCOOR'-). Polyurethanes may include polyether-based polyurethanes, polycarbonate-based polyurethanes, polyamide-based polyurethanes, polysilicone-based polyurethanes, or the like. Polyurethanes may be formed, for example, from polyols (*e.g.*, comprising two or more hydroxyl or alcohol functional groups, -OH), isocyanates (*e.g.*, comprising an isocyanate group, -N=C=O), and, optional chain extenders, catalysts, and other additives.

Suitable polyols may include, for example, polyether polyols, polycarbonate-based polyols, and the like, which may include diols, triols, etc. Polyether polyols may include, for example, polyalkylene glycols (*e.g.*, polyethylene glycols, polypropylene glycols, polybutylene glycols), poly(ethylene oxide) polyols (e.g., polyoxyethylene diols and triols), polyoxypropylene diols and triols, and the like. Alternative polyols may include, for example, 1,4-butanediol, 1,6-hexanediol, 1,12-dodecanediol, and the like.

For example, the polyol segment or segments may be represented by one or more of the following formulas:

$O\text{-}(CH_2\text{-}CH_2\text{-}CH_2\text{-}CH_2)_x\text{-}O\text{-}$ (Formula 1)

$\text{-}[O\text{-}(CH2)_n]_x\text{-}O\text{-}$ (Formula 2)

$O\text{-}[(CH_2)_6\text{-}CO_3]_n\text{-}(CH_2)\text{-}O\text{-}$ (Formula 3)

Formula (1) may depict a suitable polyether-based polyol, which may be representative of a polyol to produce TECOFLEX® polyurethanes. Formula (2) may depict a suitable polyether-based polyol, which may representative of a polyol to produce TECOPHILIC® polyurethanes. Formula (3) may depict a suitable polycarbonate-based polyol, which may be representative of a polyol to produce CARBOTHANE® polyurethanes (all of which are obtainable from the Lubrizol Corporation with offices in Wickliffe, Ohio). The polyols may also include mixtures of one or more types of polyol segments.

Suitable isocyanates may include, for example, aliphatic and cycloaliphatic isocyanates, such as 1,6-hexamethylene diisocyanate (HDI), 1-isocyanato-3-isocyanatomethyl-3,5,5-trimethyl-cyclohexane (isophorone diisocyanate, IPDI), and 4,4'-diisocyanato dicyclohexylmethane (H12MDI).

Suitable chain extenders may include, for example, ethylene glycol, 1,4-butanediol (1,4-BDO or BDO), 1,6-hexanediol, cyclohexane dimethanol, and hydroquinone bis(2-hydroxyethyl) ether (HQEE).

In one embodiment of the present invention, the polymer comprises a polyether-based polyurethane. For example, the polymer may be an aliphatic polyether-based polyurethane comprising poly(tetramethylene oxide) and polymerized 4,4'-diisocyanato dicyclohexylmethane (H12MDI) and 1,4-butanediol. An exemplary type of suitable polyether-based polyurethanes includes TECOFLEX® polymers available from the Lubrizol Corporation. For example, TECOFLEX® polymers include aliphatic block copolymer with a hard segment consisting of polymerized 4,4'-diisocyanato dicyclohexylmethane (H12MDI) and 1,4-butanediol, and a soft segment consisting of the macrodiol poly(tetramethylene oxide). In one embodiment, the TECOFLEX® polymer comprises TECOFLEX® EG-93A polyurethane. In another embodiment, the TECOFLEX® polymer comprises TECOFLEX® EG-80A polyurethane.

In another embodiment of the present invention, the polymer comprises polyether-amides (*e.g.,* thermoplastic poly(ether-block-amide)s, *e.g.,* PEBA, PEB, TPE-A, and commercially known as PEBAX® polyether-amides obtainable from Arkema Chemicals Inc., headquartered in Philadelphia, PA). Synthesis may be carried out, for example, in the molten state by polycondensation between polyether blocks (*e.g.*, a diol, such as polyoxyalkylene glycols) and polyamide blocks (*e.g.*, carboxylic acid terminated amide blocks, such as dicarboxylic blocks), which results in a thermoplastic copolymer. The long chain molecules may consist of numerous blocks where the polyamide provides rigidity and the polyether provides flexibility to the polymer. Thus, the polyether-amides may consist of linear chains of hard polyamide (PA) blocks covalently linked to soft polyether (PE) blocks via ester groups. The polyether-amides may also be synthesized via a catalyst (*e.g.*, metailic $Ti(OR)_4$), which facilitates the melt polycondensation of the polyether and polyamide blocks. The general structural formula of these block copolymers may be depicted as follows:

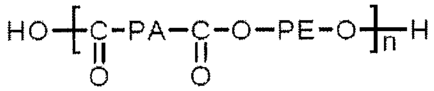

(Formula 4)

The polyamide block may include various amides including nylons (such as nylon 6, nylon 11, nylon 12, etc.). The polyether block may also include various polyethers, such as polytetramethylene oxide (PTMO), polypropylene oxide (PPO), polyethylene glycol (PEG), poly(hexamethylene oxide), polyethylene oxide (PEO), and the like. The ratio of polyether to polyamide blocks may vary from 80:20 to 20:80 (PE:PA). As the amount of polyether increases, a more flexible, softer material may result.

For example, the thermoplastic elastomer may be selected from the group consisting of TECOFLEX® polyurethanes, CARBOTHANE® polyurethanes, PEBAX® polyether-amides, and combinations thereof. For example, the elastomer may include TECOFLEX® EG-93A polyurethane, TECOFLEX® EG-80A polyurethane, TECOFLEX® EG-85A polyurethane, PEBAX® 2533 polyether-amide, PEBAX® 3533 polyether-amide, CARBOTHANE® PC-3585A polyurethane, and combinations thereof.

TECOFLEX® polyurethanes and CARBOTHANE® polyurethanes are described, for example, in Lubrizol's brochure for Engineered Polymers for Medical & Healthcare dated 2011, the disclosure of which is hereby incorporated by reference in its entirety, for all purposes. For example, TECOFLEX® aliphatic polyether polyurethanes may have the following characteristics:

**Table 1**

| Product | Hardness | Flex Modulus | Feature |
|---|---|---|---|
| EG80A | 72A | 1,000 | Clear |
| EG85A | 77A | 2,300 | Clear |
| EG93A | 87A | 3,200 | Clear |
| EG100A | 94A | 10,000 | Clear |
| EG60D | 51D | 13,000 | Clear |
| EG65D | 60D | 37,000 | Clear |
| EG68D | 63D | 46,000 | Clear |
| EG72D | 67D | 92,000 | Clear |
| EG80A B20/B40 | 73A/78A | 1,200/1,500 | Radiopaque |
| EG85A B20/B40 | 83A/86A | 2,700/3,700 | Radiopaque |
| EG93A B20/B40 | 90A/95A | 5,000/4,700 | Radiopaque |
| EG100A B20/B40 | 93A/98A | 17,000/14,000 | Radiopaque |
| EG60D B20/B40 | 55D/65D | 27,000/27,000 | Radiopaque |
| EG65D B20/B40 | 63D/78D | 82,000/97,000 | Radiopaque |
| EG68D B20 | 73D | 76,600 | Radiopaque |
| EG72D B20/B40 | 75D/82D | 125,000/179,000 | Radiopaque |

CARBOTHANE® aliphatic polycarbonate polyurethanes may have the following characteristics, for example:

**Table 2**

| Product | Hardness | Flex Modulus | Feature |
|---|---|---|---|
| PC-3575A | 71A | 620 | Clear |
| PC-3585A | 78A | 1,500 | Clear |
| PC-3595A | 91A | 4,500 | Clear |
| PC-3555D | 52D | 24,000 | Clear |
| PC-3572D | 71D | 92,000 | Clear |
| PC-3575A-B20 | 79A | 860 | Radiopaque |
| PC-3585A-B20 | 81A | 1,700 | Radiopaque |
| PC-3595A-B20 | 90A | 8,600 | Radiopaque |
| PC-3555D-B20 | 54D | 25,000 | Radiopaque |
| PC-3572D-B20 | TBD | 141,000 | Radiopaque |

The polymers may be processed using any suitable techniques, such as extrusion, injection molding, compression molding, spin-casting. For example, the polymer may be extruded or injection molded to produce hollow tubes having two open ends (see *e.g.*, Figure 2). The hollow tube can be loaded with the discrete solid dosage form(s). The open ends are sealed to form the reservoir-based drug delivery composition. A first open end may be sealed before filling the tube with the discrete solid dosage form(s), and the second open end may be sealed after the tube is filled with all of the discrete solid dosage form(s). The tube may be sealed using any suitable means or techniques known in the art. For example, the ends may be plugged, filled with additional polymers, heat sealed, or the like. The tubes should be permanently sealed such that the discrete solid dosage form(s) may not be removed. Also, the ends should be suitably sealed such that there are no holes or openings that would allow egress of the active once implanted.

The wall thickness of the excipient may be selected to provide for the desired elution rate. The wall thickness may be inversely proportional to elution rate. Thus, a larger wall thickness may result in a lower elution rate. The excipient may form a wall having an average thickness of about 0.05 to about 0.5 mm, or about 0.1 mm to about 0.3 mm (*e.g.*, about 0.1 mm, about 0.2 mm, or about 0.3 mm). Figure 21 shows an example of anastrozole elution rates from implants having varying wall thicknesses (i.e., 0.15 mm, 0.2 mm, and 0.25 mm, respectively).

In one embodiment of the present invention, the drug delivery composition does not require erosion or degradation of the excipient *in vivo* in order to release the API in a therapeutically effective amount. Alternatively, the excipient is not substantially erodible and/or not substantially degradable *in vivo* for the intended life of the implantable composition. As used herein, "erosion" or "erodible" are used interchangeably to mean capable of being degraded, disassembled, and/or digested, *e.g.,* by action of a biological environment. A compound that is "not substantially erodible" is not substantially degraded, disassembled, and/or digested over time (e.g., for the life of the implant). Alternatively, the material may be "not substantially erodible" or "does not require erosion" *in vivo* in order to provide for release of the API. In other words, the compound may erode over time, but the API is not substantially released due to erosion of the material. With respect to "degradation" or "degradable," these are intended to mean capable of partially or completely dissolving or decomposing, *e.g.*, in living tissue, such as human tissue. Degradable compounds can be degraded by any mechanism, such as hydrolysis, catalysis, and enzymatic action. Accordingly, a compound that is "not substantially degradable" does not substantially dissolve or decompose over time (*e.g.,* for the life of the implant) *in vivo.* Alternatively, the material may be "not substantially degradable" or "not requiring degradation" in order to provide for release of the API. In other words, the compound may degrade over time, but the API is not substantially released due to degradation of the material.

Implantation

The method of treating an estrogen-related disorder includes implanting a reservoir-based drug delivery composition into a subject. The terms "subject" and "patient", are used interchangeably herein and refer to a mammalian individual, such as a human being. Depending on the API at issue, the subject may vary. In the case of the treatment of an estrogen-related disorder, the subject would likely be a female human. In particular, the subject may be a postmenopausal female human. In the case of the treatment of a psychotic disorder, the subject may include a male or female human.

The drug delivery composition may be implanted into the subject in any suitable area of the subject using any suitable means and techniques known to one of ordinary skill in the art. For example, the composition may be implanted subcutaneously, *e.g.*, at the back of the upper arm or the upper back (*e.g.* in the scapular region). As used herein, the terms "subcutaneous" or "subcutaneously" or "subcutaneous delivery" means directly depositing in or underneath the skin, a subcutaneous fat layer, or intramuscularly. The drug delivery composition may be delivered subcutaneously using any suitable equipment or techniques. In one embodiment, the drug delivery composition is placed subcutaneously in the subject's arm. Alternative sites of subcutaneous administration may also be used as long as a pharmaceutically acceptable amount of the API would be released into the subject in accordance with the present invention. Preferably, the drug delivery composition should not migrate significantly from the site of implantation. Methods for implanting or otherwise positioning the compositions into the body are well known in the art. Removal and/or replacement may also be accomplished using suitable tools and methods known in the art.

Once implanted, the reservoir-based drug delivery composition may systemically deliver a therapeutically effective amount of the API to the subject at a pseudo-zero order rate for a long duration (*e.g.*, a period of time of at least one month). As used herein, the term "systemic" or "systemically" refers to the introduction of the API into the circulatory, vascular and/or lymphatic system (*e.g.*, the entire body). This is in contrast to a localized treatment where the treatment would only be provided to a specific, limited, localized area within the body. Thus, the API is systemically delivered to the subject by implanting the drug delivery composition subcutaneously into the subject.

A therapeutically effective amount of the API is delivered to the subject at a pseudo-zero order rate. Pseudo-zero order refers to a zero-order, near-zero order, substantially zero order, or controlled or sustained release of the API. A pseudo-zero order release profile may be characterized by approximating a zero-order release by release of a relatively constant amount of the API per unit time (*e.g.*, within about 30% of the average value). Thus, the composition may initially release an amount of the API that produces the desired therapeutic effect, and gradually and continually release other amounts of the API to maintain the level of therapeutic effect over the intended duration (*e.g.*, about one year). In order to maintain a near-constant level of API in the body, the API may be released from the composition at a rate that will replace the amount of API being metabolized and/or excreted from the body.

Without wishing to be bound to a particular theory, it is believed that the reservoir-based drug composition works by releasing the active (*e.g.*, aromatase inhibitor) through the excipient membrane or wall. In other words, the active diffuses across the excipient, *e.g.*, as depicted in Figure 1. Thus, sorption 112 of the active occurs from the reservoir onto the rate-controlling excipient 110. The active fully saturates the excipient 110 at steady state, and the active diffuses through the excipient and is then desorbed 114 from the excipient into the subject at a pseudo-zero order rate.

The therapeutically effective amount of the active may be delivered to the subject at a target range between a maximum value and a minimum value of average daily elution rate for the API. As used herein, the term "elution rate" refers to a rate of API delivery, which in one embodiment is based on the oral dose rate multiplied by the fractional oral bioavailability, which may be depicted as follows:

$$\text{Oral Dose X Fractional Oral Bioavailability \%} = \text{Target Elution Rate (mg/day)}$$

The elution rate may be an average rate, *e.g.*, based on the mean average for a given period of time, such as a day (i.e., average daily elution rate). Thus, a daily elution rate or average daily elution rate may be expressed as target daily oral dosage multiplied by oral bioavailability. For example, a desired daily dose of 1 mg/day for a drug that has 85% oral bioavailability would expect delivery of about 850 micrograms per day.

The maximum and minimum values refer to a maximum average daily elution rate and a minimum average daily elution rate, respectively. The minimum value required for a pharmaceutically effective dose may be correlated to or determined from a trough value for an oral dosage version of the API (*e.g.*, based on the blood/plasma concentrations for oral formulations). Similarly, maximum value may be correlated to or determined from the peak value for an oral dosage version of the API (*e.g.*, the maximum blood/plasma concentration when an oral dosage is first administered or a pharmaceutically toxic amount). In other words, the target range is a range between maximum and minimum average daily elution rates, respectively, which may be determined based on blood/plasma concentrations for equivalent oral dosage forms containing the same active. Table 3 provides two examples of aromatase inhibitors (anastrozole and letrozole) including the calculated target elution rates and maximum and minimum values for average daily elution rate.

**Table 3**

| API | Oral Dosage | Bioavailability | Target Elution Rate | Min Average Daily Elution Rate | Max Average Daily Elution Rate |
|---|---|---|---|---|---|
| Anastrozole | 1 mg/day | 80- 85% | 800 - 850 μg/day | 100 μg/day | 10,000 μg/day |
| Letrozole | 2.5 mg/day | 99% | 2,500 μg/day | 100 μg/day | 10,000 μg/day |

In one embodiment of the present invention, anastrozole is delivered to the subject at a target range of about 100 to about 10,000 micrograms/day (*e.g.*, about 300 to about 1500 micrograms/day or about 500 to about 1100 micrograms per day). Letrozole may also be delivered to a subject at a target range of about 100 to about 10,000 micrograms/day (*e.g.*, about 1000 to about 5000 micrograms/day or about 1500 to about 3500 micrograms/day).

Table 4 provides four examples of differing doses of risperidone including the calculated target elution rates and proposed maximum and minimum values for average daily elution rate.

**Table 4**

| API | Oral Dosage | Bioavailability | Target Elution Rate | Min Average Daily Elution Rate | Max Average Daily Elution Rate |
|---|---|---|---|---|---|
| Risperidone | 2 mg/day | 70% | 1400 μg/day | 700 μg/day | 2100 μg/day |
| Risperidone | 4 mg/day | 70% | 2800 μg/day | 2100 | 3500 |
| | | | | μg/day | μg/day |
| Risperidone | 6 mg/day | 70% | 4200 μg/day | 3500 μg/day | 4900 μg/day |
| Risperidone | 8 mg/day | 70% | 5600 μg/day | 4900 μg/day | 6300 μg/day |

In one embodiment of the present invention, risperidone is delivered to the subject in a wide therapeutic window, for example, at a target range of about 100 to about 10,000 micrograms/day. For a 2 mg/day dosage, the target range may be from about 700 to about 2100 μg/day, about 900 to about 1900 μg/day, about 1100 to about 1700 μg/day, about 1200 to about 1600 μg/day, or about 1300 to about 1500 μg/day. For a 4 mg/day dosage, the target range may be from about 2100 to about 3500 μg/day, about 2300 to about 3300 μg/day, about 2500 μg/day to about 3100 μg/day, about 2600 μg/day to about 3000 μg/day, or about 2700 μg/day to about 2900 μg/day. For a 6 mg/day dosage, the target range may be from about 3500 to about 4900 μg/day, about 3700 to about 4700 μg/day, about 3900 μg/day to about 4500 μg/day, about 4000 μg/day to about 4400 μg/day, or about 4100 μg/day to about 4300 μg/day. For an 8 mg/day dosage, the target range may be from about 4900 to about 6300 μg/day, about 5100 to about 6100 μg/day, about 5300 μg/day to about 5900 μg/day, about 5400 μg/day to about 5800 μg/day, or about 5500 μg/day to about 5700 μg/day.

The testing method set forth in the examples to determine the elution rates for the respective actives included placing the implants in an elution bath consisting of 50 mL 0.9% saline at 37 °C. Weekly exchanges of the elution media were analyzed by HPLC for the durations given.

The drug delivery composition is long lasting. In other words, the API is delivered to the subject (*e.g.*, at a pseudo-zero order rate) for an extended period of time. For example, the API is delivered to the subject for at least about one month (about one month or greater), at least about three months (about three months or greater), at least about six months (about six months or greater), at least about one year (about one year or greater), or any period of time within those ranges. A duration of one year may be preferred because women patients typically have an annual examination with the doctor. Accordingly, the implant could be removed and/or replaced on an annual basis which coincides with pre-existing annual visits.

Prior to implantation, the drug delivery composition may undergo any suitable processing, such as sterilization (such as by gamma radiation), heat treatment, molding, and the like. Additionally, the drug delivery composition may be conditioned or primed by techniques known in the art. For example, the drug delivery composition may be place in a medium (*e.g.*, an aqueous medium, such as saline). The medium, priming temperature, and time period of priming can be controlled to optimize release of the active upon implantation.

Efficacy of Treatment for Estrogen-Related Disorders

The methods of treatment described herein may treat, delay onset, or inhibit recurrence of the disease or condition. A pharmaceutically effective or therapeutic amount of active should be administered sufficient to affect or produce the desired therapy. For the case of aromatase inhibitors as the active, releasing an amount of aromatase inhibitor effective to inhibit or slow onset or recurrence of an estrogen-related disorder, such as breast cancer (*e.g.*, lower estrogen levels), is desired. A doctor would be able to determine the efficacy of the treatment (i.e., know the aromatase inhibitor was working to treat breast cancer) using techniques known to one of ordinary skill in the art. Aromatase inhibitors may be particularly effective for treating breast cancer in post-menopausal female human. This may be especially true for a post-menopausal female human that has undergone at least one treatment for breast cancer prior to receiving the aromatase inhibitor. The doctor could determine the therapeutic effectiveness of the aromatase inhibitor in treating the estrogen-related disorder (*e.g.,* breast cancer), for example, by assessing *in vitro* (*e.g.,* cell cultures) or *in vivo* (*e.g.,* within the body of a subject) methods known in the art, which measure the extent to which serum estradiol concentrations (*e.g.*, estrogen levels) in a subject are lowered.
For example, one method for assessing the effectiveness of aromatase inhibitor(s) in the treatment of an estrogen-related disorder, such as breast cancer, comprises measuring mean serum concentrations of estradiol in a subject over time. In other words, the effectiveness of the aromatase inhibitor in lowering a subject's serum estradiol concentrations may comprise a reduction in mean serum concentration by at least about 30%, at least about 50%, or at least about 70% within about 24 hours, or within several days (*e.g.,* about 7 days or about 14 days) after implantation, and continuing, for example, throughout the time that the implant resides in the patient. Additionally, the subject's serum estradiol concentrations preferably comprise a reduction in mean serum concentration by at least about 30%, at least about 50%, or at least about 70% for the duration of the implant.
A method for assessing the effectiveness of aromatase inhibitor(s) in the treatment of short stature in children or adolescents may comprise examining the bone age of a subject according to known methods in order to predict the subject's adult height prior to treatment with the aromatase inhibitor. Following treatment with the aromatase inhibitor, the bone age of the subject may be examined again to determine if the predicted adult height had increased, which would indicate that the aromatase inhibitor had been effective in increasing the subject's projected adult height. Another method may comprise monitoring the subject's bone age acceleration before, during, and after treatment with an aromatase inhibitor.
It would also be appreciated by one of ordinary skill in the art that the treatment regime for treating an estrogen-related disorder, such as breast cancer, with an aromatase inhibitor (or anastrozole, specifically), may depend on a variety of factors, including the type, age, weight, sex, diet and medical condition of the patient, and pharmacological considerations such as activity, efficacy, pharmacokinetic and toxicology profiles of the particular active employed. Thus, the treatment regime actually employed may vary widely from subject to subject.

Treatment of Estrogen-Related Disorders with Anastrozole

Although embodiments of the invention have been described with respect to aromatase inhibitors generally, the treatment of estrogen-related disorders or estrogen-receptor disorders (especially breast cancer) may be particularly effective with anastrozole selected as the active or one of the active pharmaceutical agents. As depicted in Figure 4, the elution rates of anastrozole are at a pseudo-zero order rate for over 400 days. Similarly, Figure 8 depicts *in-vitro* elution rates of anastrozole from a composition comprising a polyether-based polyurethane excipient at a pseudo-zero order elution for over one year. Figure 9 shows *in-vivo* plasma concentrations (ng/mL) for over 280 days. Figure 9 also includes the plasma concentration for an oral dose of anastrozole (ARIMIDEX™ is an oral form of anastrozole sold by Astra Zeneca).
In one embodiment according to the present invention, a method of treating an estrogen-related disorder (such as breast cancer) includes implanting a reservoir-based drug delivery composition into a subject to systemically deliver a therapeutically effective amount of anastrozole to the subject for a period of time of at least one month. For example, the delivery may occur at a pseudo-zero order rate. The reservoir-based drug delivery composition may comprise at least one discrete solid dosage form surrounded by an excipient comprising at least one polymer, and the at least one discrete solid dosage form comprises 75-97 wt% (*e.g.*, about 88 wt%) anastrozole or a pharmaceutically effective salt thereof based on the total weight of the at least one discrete solid dosage form and 1-25 wt% (*e.g.*, about 10 wt%) of at least one sorption enhancer based on the at least one discrete solid dosage form. In one embodiment, the polymer is a TECOFLEX® polymer (*e.g.*, TECOFLEX® EG-93A polyurethane).
A doctor would be able to determine the efficacy of the treatment (*e.g.*, know that anastrozole was working to treat breast cancer) using techniques known to one of ordinary skill in the art as discussed above. For example, serum estradiol concentrations may be checked to determine the efficacy of treatment. The therapeutic effectiveness of anastrozole may be assessed *in vitro* or *in vivo* to measure the amount the serum estradiol concentrations in a subject are

lowered. For example, the mean serum concentrations of estradiol in a subject may be assessed over time, and the effectiveness of anastrozole in lowering a subject's serum estradiol concentrations may comprise a reduction in mean serum concentration by at least about 30%, at least about 50%, or at least about 70% within a limited period after implantation and throughout the life of the implanted composition.

Efficacy of Treatment for Psychotic Disorders

The methods of treatment described herein may treat, delay onset, or inhibit recurrence of the disease or condition. A pharmaceutically effective or therapeutic amount of active should be administered sufficient to affect or produce the desired therapy. For the case of risperidone, releasing an amount of risperidone effective to inhibit, stabilize or slow onset or recurrence of psychotic conditions, such as schizophrenia, or symptoms thereof is desired. A doctor would be able to determine the efficacy of the treatment (i.e., know the risperidone was working to treat the psychotic disorder) using techniques known to one of ordinary skill in the art.

For example, after a subject has begun a regimen of risperidone, a clinician may use a rating scale which assesses the psychiatric symptoms of schizophrenia, for example, in order to determine whether there has been an improvement in those symptoms over time. The Brief Psychiatric Rating Scale (BPRS) is a multi-item inventory of general psychopathology which may be used to evaluate the effects of risperidone treatment, for example, in a schizophrenia patient. The BPRS psychosis cluster (conceptual disorganization, hallucinatory behavior, suspiciousness, and unusual thought content) is considered a particularly useful subset for assessing schizophrenic patients. Another traditional assessment, the Clinical Global Impression (CGI), reflects the impression of a skilled observer that is fully familiar with the manifestations of the psychotic disorder (*e.g.*, schizophrenia), about the overall clinical state of the patient. In addition, the Positive and Negative Syndrome Scale (PANSS) and the Scale for Assessing Negative Symptoms (SANS) may be employed. Improvement in a subject's symptoms, as measured by a clinician according to any of the aforementioned assessments, or other assessments used in the art to evaluate the symptoms of a psychotic disorder, can be used to indicate whether the amount of risperidone being used is effective. For example, the effectiveness of risperidone in treating a subject's psychotic (*e.g.*, schizophrenia) symptoms may comprise an improvement of at least about 10%, at least about 20%, or at least about 30% in the patient's BPRS, CGI, PANSS, and/or SANS score over a period of between about 4 weeks to about 8 weeks following the start of a risperidone regimen (*e.g.,* following implantation).

Similarly, several methods may be used to assess the effectiveness of risperidone in the treatment of bipolar mania. For example, after a subject has begun a regimen of risperidone, a clinician may use a rating scale which assesses the psychiatric symptoms of bipolar mania, in order to determine whether there has been an improvement in those symptoms over time. One rating instrument that may be used for assessing manic symptoms is the Young Mania Rating Scale (YMRS), an 11-item clinician-rated scale traditionally used to assess the degree of manic symptomatology (irritability, disruptive/aggressive behavior, sleep, elevated mood, speech, increased activity, sexual interest, language/thought disorder, thought content, appearance, and insight) in a range from 0 (no manic features) to 60 (maximum score). Improvement in a subject's symptoms, as measured by a clinician according to YMRS, or other assessments used in the art to evaluate the symptoms of bipolar mania, can be used to indicate whether the amount of risperidone being used is effective to treat bipolar mania. For example, the effectiveness of risperidone in treating a subject's bipolar mania symptoms may comprise an improvement of at least about 10%, at least about 20%, or at least about 30% in the patient's YMRS score over a period of about 3 weeks to about 8 weeks following the start of a risperidone regimen (e.g., following implantation).

Several methods may also be used to assess the effectiveness of risperidone in the treatment of autism. For example, after a subject has begun a regimen of risperidone, a clinician may use a rating scale which assesses the symptoms of autism, in order to determine whether there has been an improvement in those symptoms over time. Rating instruments that may be used for assessing the symptoms of autism include the Irritability subscale of the Aberrant Behavior Checklist (ABC-I) and the Clinical Global Impression - Change (CGI-C) scale. The ABC-I subscale, for example, measures emotional and behavioral symptoms of autism, including aggression towards others, deliberate self-injuriousness, temper tantrums, and quickly changing moods. Improvement in a subject's symptoms, as measured by a clinician according to ABC-I, CGI-C, or other assessments used in the art to evaluate the symptoms of autism, can be used to indicate whether the amount of risperidone being used is effective to treat autism. For example, the effectiveness of risperidone in treating a subject's autism symptoms may comprise an improvement of at least about 10%, at least about 20%, or at least about 30% in the patient's ABC-I or CGI-C score over a period of about 4 weeks to about 12 weeks following the start of a risperidone regimen (e.g., following implantation).

Risperidone's therapeutic activity may be mediated through a combination of dopamine Type 2 (D2) and serotonin Type 2 (SHT2) receptor antagonism. Thus, the therapeutic effectiveness of risperidone in treating a psychiatric/psychotic disorder may alternatively be assessed by *in vitro* or *in vivo* methods known in the art which measure the effect of risperidone on dopamine Type 2 (D2) and/or serotonin Type 2 (SHT2) receptors.

It would also be appreciated by one of ordinary skill in the art that the treatment regime for treating a psychotic

disorder with risperidone may depend on a variety of factors, including the type, age, weight, sex, diet and medical condition of the patient, and pharmacological considerations such as activity, efficacy, pharmacokinetic and toxicology profiles of the particular active employed. Thus, the treatment regime actually employed may vary widely from subject to subject.

Subcutaneous Delivery Systems and Kits

In one aspect of the present invention, a subcutaneous delivery system comprises an elastomeric reservoir implant comprising at least one discrete solid dosage form surrounded by a polymeric rate-controlling excipient. The at least one discrete solid dosage form comprises at least one API (*e.g.*, aromatase inhibitor). The subcutaneous delivery system provides for release of the API at an elution rate suitable to provide a therapeutically effective amount of the API to a subject at a pseudo-zero order rate for a period of time of at least one month. In another aspect of the present invention, a kit for subcutaneously placing a drug delivery composition comprises a reservoir-based drug delivery composition comprising a polymeric rate-controlling excipient defining a reservoir containing at least one discrete solid dosage form comprising at least one API; and an implanter for inserting the reservoir-based drug delivery composition beneath the skin.

The drug delivery composition may be implanted into the subject in any suitable area of the subject using any suitable means and techniques known to one of ordinary skill in the art. For example, the composition may be implanted subcutaneously, e.g., at the back of the upper arm, by directly depositing in or underneath the skin, a subcutaneous fat layer, or intramuscularly.

The drug delivery composition may be delivered subcutaneously using any suitable equipment or techniques, e.g., an implanter known to one ordinary skill in the art. The kits may comprise the drug delivery composition pre-loaded into the implanter or the drug delivery composition may be loaded by the doctor or other user. The implanter may be an implantation device, such as a syringe, cannula, trocar or catheter, that may be inserted into an incision made at the delivery site of the subject. Suitable implantation devices and implantation methods include the trocar and methods disclosed in US 7,214,206 and US 7,510,549, the disclosures of which are herein incorporated by reference in their entirety, for all purposes. Other suitable methods for implanting or otherwise positioning the compositions into the body, *e.g.*, by a doctor, are well known in the art. Removal and/or replacement may also be accomplished using suitable tools and methods known in the art. Kits may also comprise other equipment well known in the art, such as scalpels, clamps, suturing tools, hydration fluid, and the like.

Implantable Drug Delivery Compositions with Polymer Excipient(s)

Although embodiments of the invention have been described above with respect to aromatase inhibitors and risperidone generally, the method of delivering a therapeutically effective amount of an active pharmaceutical ingredient from an implantable drug delivery composition may be expanded to other suitable active pharmaceutical ingredients and certain methods of treatment based on the selection of certain parameters. Without wishing to be bound to a particular theory, it is believed that by selecting specific polymers with certain contents or ratios of hard to soft segments, certain desired elution rates may be achieved. Moreover, by adding certain sorption enhancers in certain amounts with the API to the discrete solid dosage formulations within the reservoir, the elution rates may be further changed or modulated (*e.g.*, "tuned" or "dialed in") from the drug delivery composition to desired, pharmaceutically efficacious values.

According to one aspect of the present invention, a method of delivering a therapeutically effective amount of an active pharmaceutical ingredient from an implantable drug delivery composition comprises implanting a reservoir-based drug delivery composition into a subject to systemically deliver a therapeutically effective amount of an active pharmaceutical ingredient to the subject at a pseudo-zero order rate for a period of time of at least one month. The drug delivery composition comprises at least one discrete solid dosage form surrounded by an excipient comprising at least one polymer, and the at least one discrete solid dosage form comprises the active pharmaceutical ingredient or a pharmaceutically acceptable salt thereof. The polymer comprises a substantially non-porous, elastomeric polymer comprising soft and hard segments, and the relative content of the soft and hard segments provide an elution rate within a target range between a maximum and minimum value of a desired average daily elution rate for the active pharmaceutical ingredient.

According to one embodiment of the present invention, a drug delivery composition includes a rate-controlling excipient defining a reservoir which contains at least one discrete solid dosage form comprising an active pharmaceutical ingredient or a pharmaceutically acceptable salt thereof. The rate-controlling excipient comprises a substantially non-porous, elastomeric polymer comprising soft and hard segments selected based on the relative content of soft and hard segments of the polymer to obtain an elution rate within a target range of average daily elution rate for the active pharmaceutical ingredient. The at least one discrete solid dosage form comprises at least one sorption enhancer in an amount effective to modulate the average daily elution rate of the active pharmaceutical ingredient to provide for release

of the active pharmaceutical ingredient at pseudo-zero order within the target range at the therapeutically effective amount for a period of time of at least one month. The amount of sorption enhancer may be directly proportional to the average daily elution rate.

According to another embodiment of the present invention, a method of choosing an implantable drug delivery composition comprises selecting a rate-controlling excipient comprising a substantially non-porous, elastomeric polymer comprising soft and hard segments for defining a reservoir based on the relative content of soft and hard segments of the polymer to adjust the elution rate within a target range of average daily elution rate for an active pharmaceutical ingredient; and selecting and formulating the active pharmaceutical ingredient or a pharmaceutically acceptable salt thereof and at least one sorption enhancer in order to modulate the elution rate to achieve a therapeutically effective amount of the active pharmaceutical ingredient at pseudo-zero order for a period of time of at least one month, wherein the amount of sorption enhancer may be directly proportional to the average daily elution rate.

Polymer Selection

The excipient comprises at least one polymer having soft and hard segments. As used herein, the term "segment" may refer to any portion of the polymer including a monomer unit, or a block of the polymer, or a sequence of the polymer, etc. "Soft segments" may include a soft phase of the polymer, which is amorphous with a glass transition temperature below the use temperature (*e.g.*, rubbery). "Hard segments" may include a hard phase of the polymer that is crystalline at the use temperature or amorphous with a glass transition temperature above the use temperature (*e.g.*, glassy). The use temperature may include a range of temperatures including room temperature (about 20-25°C) and body temperature (about 37°C). Without wishing to be bound to a particular theory, the soft segment may provide for the greatest impact on sorption onto the excipient and the hard segment may impact diffusion across or through the excipient. See *e.g.*, Figure 1 showing sorption 112 of the API from the reservoir into the excipient 110 and desorption 114 of the API from the excipient into the subject. Any suitable polymer comprising hard and soft segments may be selected by one of ordinary skill in the art, as long as the polymer allows for delivery of a therapeutically effective amount of the API to the subject at a pseudo-zero order rate for the intended period of time of the implant. In one embodiment of the present invention, the selected polymer excipient is hydrophobic.

In one embodiment, the polymer is a thermoplastic elastomer or elastomeric polymer, which encompasses polymers (homopolymers, copolymers, terpolymers, oligomers, and mixtures thereof) having elastomeric properties and containing one or more elastomeric subunits (*e.g.*, an elastomeric soft segment or block). The thermoplastic elastomers may include copolymers (*e.g.*, styrenic block copolymers, polyolefin blends, elastomeric alloys, thermoplastic polyurethanes, thermoplastic copolyester, and thermoplastic polyamides) or a physical mix of polymers (*e.g.*, a plastic and a rubber), which consist of materials with both thermoplastic and elastomeric properties, for example, comprising a weaker dipole or hydrogen bond or crosslinking in one of the phases of the material. The elastomeric polymer may comprise polyurethanes, polyethers, polyamides, polycarbonates, polysilicones, or copolymers thereof. Thus, the polymer may include elastomeric polymers comprising polyurethane-based polymers, polyether-based polymers, polysilicone-based polymers, polycarbonate-based polymers, or combinations thereof. In an exemplary embodiment, the polymer comprises a polyurethane-based polymer or a polyether-block-polyamide polymer.

Suitable hard and soft segments of the polymer may be selected by one of ordinary skill in the art. It will be appreciated by one of ordinary skill in the art that although certain types of polymers are described herein, the hard and soft segments may be derived from monomers, polymers, portions of polymers, etc. In other words, the polymers listed may be changed or modified during polymerization, but those polymers or portions of those polymers in polymerized form constitute the hard and soft segments of the final polymer.

Examples of suitable soft segments include, but are not limited to, those derived from (poly)ethers, (poly)carbonates, (poly)silicones, or the like. For example, the soft segments may be derived from alkylene oxide polymers selected from the group consisting of poly(tetramethylene oxide) (PTMO), polyethylene glycol (PEG), poly(propylene oxide) (PPO), poly(hexamethylene oxide), and combinations thereof. The soft segment may also be derived from polycarbonate soft segments (obtainable from Lubrizol) or silicone soft segments (obtainable from Aortech).

Examples of suitable hard segments include, but are not limited to, those derived from polyurethanes or polyamides. For example, the hard segments may be derived from isocyanates and amides, such as nylons, nylon derivatives (such as nylon 6, nylon 11, nylon 12, etc.), carboxylic acid terminated amide blocks, and the like.

The polymer may be formed by any suitable means or techniques known to one of ordinary skill in the art. For example, the polymer may be formed from monomers, polymer precursors, pre-polymers, polymers, etc. Polymer precursors may include monomeric as well as oligomeric substances capable of being reacted or cured to form polymers. The polymers may be synthesized using any suitable constituents.

In one embodiment of the present invention, the polymer comprises polyurethanes (*e.g.*, comprising a urethane linkage, -RNHCOOR'-). Polyurethanes may include polyether-based polyurethanes, polycarbonate-based polyurethanes, polyamide-based polyurethanes, polysilicone-based polyurethanes, or the like, as discussed in detail above.

Polyurethanes may contain both soft segments and hard segments. The soft segments may be derived from polyols including polyether polyols, polycarbonate-based polyols, and the like. For example, soft segments may be derived from polyether polyols, such as polyalkylene glycols (*e.g.*, polyethylene glycols, polypropylene glycols, polybutylene glycols), poly(ethylene oxide) polyols (*e.g.*, polyoxyethylene diols and triols), polyoxypropylene diols and triols, and the like. Soft segments may be derived from polyols, such as 1,4-butanediol, 1,6-hexanediol, 1,12-dodecanediol, and the like. An elution rate for a composition comprising a polycarbonate soft segment polyurethane is provided in Figure 11. The soft segment derived from the polyols may be represented by the following formulas or mixtures thereof, for example:

$O\text{-}(CH_2\text{-}CH_2\text{-}CH_2\text{-}CH_2)_x\text{-}O\text{-}$ (Formula 1)

$\text{-}[O\text{-}(CH_2)_n]_x\text{-}O\text{-}$ (Formula 2)

$O\text{-}[(CH_2)_6\text{-}CO_3]_n\text{-}(CH_2)\text{-}O\text{-}$ (Formula 3)

The hard segments may be derived from isocyanates, such as aliphatic and cycloaliphatic isocyanates, such as 1,6-hexamethylene diisocyanate (HDI), 1-isocyanato-3-isocyanatomethyl-3,5,5-trimethyl-cyclohexane (isophorone diisocyanate, IPDI), and 4,4'-diisocyanato dicyclohexylmethane (H12MDI).

In another embodiment of the present invention, the polymer may comprise a polyether-based polyurethane. For example, the polymer may be an aliphatic polyether-based polyurethane comprising poly(tetramethylene oxide) as the soft segment and polymerized 4,4'-diisocyanato dicyclohexylmethane (H12MDI) and 1,4-butanediol as the hard segment. A suitable polymer includes TECOFLEX®, an aliphatic block copolymer with a hard segment consisting of polymerized 4,4'-diisocyanato dicyclohexylmethane (H12MDI) and 1,4-butanediol, and a soft segment consisting of the macrodiol poly(tetramethylene oxide).

In another embodiment of the present invention, the polymer comprises polyether-amides (*e.g.*, thermoplastic poly(ether-block-amide)s, *e.g.*, PEBA, PEB, TPE-A, and commercially known as PEBAX® polyether-amides). The hard segment may comprise the polyamide blocks (*e.g.*, carboxylic acid terminated amide blocks, such as dicarboxylic blocks) and the soft segments may comprise the polyether blocks (*e.g.*, a diol, such as polyoxyalkylene glycols). The general structural formula of these block copolymers may be depicted as follows:

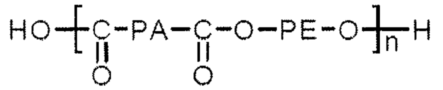

(Formula 4)

where PA represents the hard segment and PE represents the soft segment. The polyamide block may include various amides including nylons (such as nylon 6, nylon 11, nylon 12, etc.). The polyether block may also include various polyethers, such as poly(tetramethylene oxide) (PTMO), polyethylene glycol (PEG), poly(propylene oxide) (PPO), poly(hexamethylene oxide), polyethylene oxide (PEO), and the like. The ratio of polyether to polyamide blocks may vary from 80:20 to 20:80 (PE:PA). As the amount of polyether increases, a more flexible, softer material may result.

In one embodiment, the elastomeric polymer is selected from the group consisting of TECOFLEX® polyurethanes, CARBOTHANE® polyurethanes, PEBAX® polyether-amides, and combinations thereof. For example, the elastomeric polymer may include TECOFLEX® EG-93A polyurethane, TECOFLEX® EG-80A polyurethane, TECOFLEX® EG-85A polyurethane, PEBAX® 2533 polyether-amide, PEBAX® 3533 polyether-amide, CARBOTHANE® PC-3585A polyurethane, and combinations thereof.

The relative content of the soft and hard segments may provide an elution rate within a target range of average daily elution rate for the active pharmaceutical ingredient. The relative content of the soft and hard segments refers to the amount or content of soft segments to hard segments in the polymer. The relative content may also be defined as a ratio of soft segment to hard segments (*e.g.*, at least about 2:1 or at least about 4:1 of soft to hard segments). For example, the soft content may be 50% or more, 60% or more, 70% or more, or 80% or more relative to the hard content. In one embodiment, the relative content is about 70% soft segments and about 30% hard segments or at least about 2.3:1 soft: hard (e.g., PEBAX® 2533 polyether-amide). In another embodiment, the relative content is about 80% soft segments and about 20% hard segments or at least about 4:1 soft:hard (*e.g.*, PEBAX® 3533 polyether-amide).

The ratio of soft to hard segments may vary depending on the desired elution rate. Without wishing to be bound to a particular theory, it is believed that the soft segments may contribute to the sorption of the API into the excipient and/or the hard segment may contribute to the rate of diffusion (*e.g.*, how fast the active diffuses through the excipient). The rate of diffusion through the excipient probably does not matter much, however, once the implant reaches steady

state (*e.g.*, a constant or near constant elution rate). Thus, it may be desirable to have a higher ratio of soft segments relative to hard segments (*e.g.*, at least about 2:1, at least about 3:1, or at least about 4:1). The relative content of the soft and hard segments may also be considered directly proportional on the molecular weights of both the soft and hard segments. In other words, for a given ratio, a higher molecular weight polymer for the soft segment results in a higher relative content of soft segments to hard segments.

The molecular weights of each of the soft and hard segments may be selected depending on the specific soft and hard segments selected. In particular, the size (*e.g.*, molecular weight) of the soft segment may impact the elution rate. For example, the soft block (*e.g.*, polyether) molecular weights may range from about 1000-12,000 daltons (daltons may be used interchangeably with g/mol for molecular weight). For the case of PTMO as the soft segment, the molecular weights may range from about 1000-3000 daltons. In some cases, a higher molecular weight may be preferred (*e.g.*, about 2000-2900 daltons) in order to elevate elution, as compared to less than about 1000 daltons. For the case of PPO as the soft segment, the molecular weight may range from about 2000-12,0000 daltons, and again a higher molecular weight may be preferred to elevate elution rates. For the case of polyether-block amides, the molecular weight of the polyether block may vary from about 400 to about 3000 daltons and that of the polyamide block may vary from about 500 to about 5000 daltons. Without wishing to be bound to a particular theory, it is believed that by increasing the molecular weight of soft segments in the polymer, the content of hard segments is reduced providing for better dissolution and diffusion of the API through the excipient.

The Shore D hardness or Shore hardness of the polymer segments may also have an impact on the elution rates. In some cases, the Shore hardness may be inversely proportional to the elution rate (*e.g.,* a higher Shore hardness results in a lower elution rate). For example, in the case of polyether-block amides, a Shore hardness of 35 provides a lower elution rate as compared to a Shore hardness of 25. This is depicted, for example, in Figure 12, which shows elution rates for an anastrozole composition comprising PEBAX® 2533 polyether-amide (a Shore hardness of 25) and PEBAX® 3533 polyether-amide (a Shore hardness of 35).

In one embodiment of the present invention, the excipient is substantially or completely non-porous, in that the polymer has a porosity or void percentage less than about 10%, about 5%, or about 1%, for example. In particular, the excipient is substantially non-porous in that there are no physical pores or macropores which would allow for egress of the API from the drug delivery composition. In another embodiment, the excipient is practically insoluble in water, which equates to one gram in > 10,000 mL of water. In another embodiment of the present invention, the drug delivery composition does not require erosion or degradation of the excipient *in vivo* in order to release the API in a therapeutically effective amount. Alternatively, the excipient is not substantially erodible and/or not substantially degradable *in vivo* for the intended life of the implantable composition (*e.g.*, the API is not released due to erosion or degradation of the material *in vivo*).

The rate-controlling excipient may comprise a substantially non-porous, elastomeric polymer comprising soft and hard segments selected based on the relative content of soft and hard segments of the polymer to obtain an elution rate within a target range of average daily elution rate for the active pharmaceutical ingredient. A therapeutically effective amount of the API is delivered to the subject at a pseudo-zero order rate within a target range between a maximum and minimum value of a desired average daily elution rate for the active pharmaceutical ingredient. Pseudo-zero order refers to a zero-order, near-zero order, substantially zero order, or controlled or sustained release of the API. The composition may initially release an amount of the API that produces the desired therapeutic effect, and gradually and continually release other amounts of the API to maintain the level of therapeutic effect over the intended duration of treatment (*e.g.*, about one year).

As previously noted, the excipient defines the shape of the reservoir, which may be of any suitable size and shape. In an exemplary embodiment, the excipient is substantially cylindrically shaped. An embodiment of a cylindrically shaped excipient is depicted, for example, in Figure 2. The reservoir may be of any suitable size depending on the active and location of delivery, *e.g.*, a ratio of about 1:1.5 to 1:5 diameter to length.

The wall thickness of the excipient may also be selected to provide for the desired elution rate. The wall thickness may be inversely proportional to elution rate. Thus, a larger wall thickness may result in a lower elution rate. The excipient may form a wall having an average thickness of about 0.05 to about 0.5 mm, or about 0.1 mm to about 0.3 mm (*e.g.*, about 0.1 mm, about 0.2 mm, or about 0.3 mm). Figure 21 shows an example of anastrozole elution rates from implants having varying wall thicknesses (i.e., 0.15 mm, 0.2 mm, and 0.25 mm, respectively).

The polymers may be processed using any suitable techniques, such as extrusion, injection molding, compression molding, spin-casting. In one embodiment, a method of making an implantable drug delivery composition includes: (a) selecting a substantially non-porous elastomeric polymer comprising soft and hard segments based on the relative content and molecular weights of the soft and hard segments of the polymer to provide an elution rate within a target range of average daily elution rate for an active pharmaceutical ingredient; (b) forming a hollow tube from the elastomeric polymer (see *e.g.*, Figure 2); (c) selecting and formulating the active pharmaceutical ingredient or a pharmaceutically acceptable salt thereof and at least one sorption enhancer in order to produce an elution rate at a therapeutically effective amount of the active pharmaceutical ingredient at pseudo-zero order for a period of time of at least one month, wherein

the amount of sorption enhancer is directly proportional to the average daily elution rate; (d) loading at least one discrete solid dosage form comprising the active pharmaceutical ingredient and the at least one sorption enhancer into the tube; and (e) sealing both ends of the tube to form a sealed cylindrical reservoir-based drug delivery composition. The tube may be sealed using any suitable means or techniques known in the art. For example, the ends may be plugged, filled with additional polymers, heat sealed, or the like. The tubes should be permanently sealed such that the discrete solid dosage forms may not be removed. Also, the ends should be suitably sealed such that there are no holes or openings that would allow egress of the active once implanted.

Active Pharmaceutical Ingredients

Suitable active pharmaceutical ingredients in accordance with the present invention include active pharmaceutical ingredients in oral dosage forms where compliance is at issue, long term treatment is needed, and/or a steady dose (*e.g.*, zero order) is required, for example, to minimize side effects. In other words, suitable actives may be selected for the treatment of diseases and conditions that are long lasting (*e.g.*, requiring treatment for many weeks, months or even years). Diseases and conditions may include, but are not limited to, the treatment of estrogen related disorders (*e.g.*, breast cancer, short stature in children or adolescents), psychotic disorders (*e.g.*, schizophrenia, Bipolar disorder), benign prostatic hyperplasia, overactive bladder, Parkinson's disease, muscle relaxer, smoking cessation, Alzheimer's, Sickle cell anemia, pulmonary arterial hypertension, autoimmune diseases (*e.g.*, multiple sclerosis, Crohn's disease, Lupus), and the like.

Suitable actives may be selected based on traditional oral dosage forms, which require continued dosage over a long period of time, especially where the patient may not be compliant in taking the medications. For example, the active pharmaceutical ingredient may be selected from the group consisting of anastrozole, exemestane, dutasteride, oxybutynin, letrozole, selegiline, tolterodine, tizanidine, varenicline, rivastigmine, rasagiline, asenapine, paliperidone, aripiprazole, rotigotine, folic acid, vardenafil, fingolimod, laquinimod, risperidone, nicergoline, guanfacine, and pharmaceutically acceptable salts thereof (*e.g.*, HCl, tartrate, mesylate, maleate, palmitate, and the like). The active pharmaceutical ingredient may alternatively be selected from the group consisting of anastrozole, exemestane, dutasteride, oxybutynin, letrozole, selegiline, tolterodine, varenicline, rivastigmine, asenapine, paliperidone, aripiprazole, rotigotine, folic acid, vardenafil, fingolimod, laquinimod, risperidone, nicergoline, guanfacine, and pharmaceutically acceptable salts thereof (*e.g.,* HCl, tartrate, mesylate, maleate, palmitate, and the like). For example, pharmaceutically acceptable salts may include, but are not limited to, oxybutynin HCl, selegiline HCl, tolterodine tartrate, rivastigmine tartrate, and asenapine maleate. In one embodiment of the present invention, the selected API is hydrophobic.

According to an embodiment of the present invention, the active pharmaceutical ingredient is fingolimod free base. As described in the examples below, fingolimod free base demonstrated higher elution rates *in vitro,* in comparison to fingolimod HCl. According to one embodiment, the at least one discrete solid dosage form comprises 75-97 wt% (e.g., about 88%) fingolimod free base based on the total weight of the at least one discrete solid dosage form; 1-25 wt% (e.g., about 10%) of at least one sorption enhancer based on the total weight of the at least one discrete solid dosage form; and 0-5 wt% (e.g., about 2%) lubricant based on the total weight of the at least one discrete solid dosage form.

According to another embodiment of the present invention, the active pharmaceutical ingredient is varenicline free base. As described in the examples below, varenicline free base demonstrated higher and more stable elution rates *in vitro,* in comparison to varenicline tartrate. According to one embodiment, the at least one discrete solid dosage form comprises 75-97 wt% (e.g., about 88%) varenicline free base based on the total weight of the at least one discrete solid dosage form; 1-25 wt% (e.g., about 10%) of at least one sorption enhancer based on the total weight of the at least one discrete solid dosage form; and 0-5 wt% (e.g., about 2%) lubricant based on the total weight of the at least one discrete solid dosage form.

According to another embodiment of the present invention, the active pharmaceutical ingredient is selected from the group consisting of oxybutynin HCl and oxybutynin free base. As described in the examples below, both oxybutynin HCl and oxybutynin free base demonstrated suitable elution rates *in vitro.* It was also observed that oxybutynin HCl and oxybutynin free base demonstrated suitable elution rates *in vitro* depending on the type of polymer used. For example, oxybutynin HCl had suitable release rates from TECOFLEX® EG-80A and TECOFLEX® EG-85A implants, whereas oxybutynin free base had suitable release rates from TECOFLEX® EG-93A, TECOPHILIC® HP-60D-05, and TECOPHILIC® HP-60D-10 implants. According to one embodiment, the at least one discrete solid dosage form comprises 75-97 wt% (e.g., about 88%) oxybutinin free base or oxybutinin HCl based on the total weight of the at least one discrete solid dosage form; 1-25 wt% (e.g., about 10%) of at least one sorption enhancer based on the total weight of the at least one discrete solid dosage form; and 0-5 wt% (e.g., about 2%) lubricant based on the total weight of the at least one discrete solid dosage form.

For the treatment of estrogen-related disorders, the active may include aromatase inhibitors, such as anastrozole, letrozole, exemestane, etc. For the treatment of psychotic disorders including schizophrenia, the active may include risperidone, asenapine (*e.g.*, asenapine maleate), paliperidone, etc. For the treatment of Bipolar disorder, the active

may include risperidone, aripiprazole, etc. For the treatment of benign prostatic hyperplasia, the active may include dutasteride, etc. For the treatment of overactive bladder, the active may include oxybutynin HCl, oxybutynin base, tolterodine (*e.g.*, tolterodine tartrate), etc. For the treatment of Parkinson's disease, the active may include monoamine oxidase B inhibitors, such as selegiline (*e.g.*, selegiline HCl), or rotigotine, etc. For providing a muscle relaxer, the active may include tizanidine, etc. For smoking cessation, the active may include varenicline, etc. For the treatment of Alzheimer's, the active may include rivastigmine (*e.g.*, rivastigmine tartrate), etc. For the treatment of sickle cell anemia, the active may include folic acid, etc. For the treatment of pulmonary arterial hypertension, the active may include vardenafil, etc. For the treatment of autoimmune diseases including multiple sclerosis, Crohn's disease, or Lupus, the active may include fingolimod, laquinimod, etc.

Sorption Enhancer(s) and the Discrete Dosage Form

In another aspect of the present invention, the at least one discrete solid dosage form, within the reservoir, may also comprise at least one sorption enhancer in an amount effective to modulate the average daily elution rate of the active pharmaceutical ingredient to provide for release of the active pharmaceutical ingredient at pseudo-zero order within the target range at the therapeutically effective amount for a period of time of at least one month. As used herein, the terms "modulate" or "modulation" may be used to describe a change in the activity of the drug delivery composition. This may equate to a change in elution rate (*e.g.*, an increase or a decrease in a given elution rate or range).

Sorption enhancers may include compounds which improve the release of the API from the drug delivery composition. Without wishing to be bound to a particular theory, the sorption enhancers may improve release of the API from the drug delivery composition by drawing water or other fluids into the reservoir from the subject, disintegrating or breaking apart the discrete solid dosage form(s), and/or allowing the API to come into contact or remain in contact the inner walls of the excipient. Such a mechanism may be depicted, for example, in Figure 1.

The amount of the sorption enhancer is not particularly limited, but, when present, is preferably on the order of about 1-25 wt% of the solid dosage form, more preferably about 5-20 wt% of the solid dosage form, and more preferably about 10 wt%. The amount of sorption enhancer may be directly proportional to the elution rate. In other words, a higher weight percent of sorption enhancer in the composition may result in a higher average elution rate than a smaller weight percentage. Thus, it may be preferable to include a higher weight percent of sorption enhancer to give a higher elution rate (*e.g.*, about 8-25 wt% or about 10-20 wt%). Figure 13 depicts elution rates for an anastrozole composition comprising 0% sorption enhancer and 10% sorption enhancer (*e.g.*, croscarmellose), respectively. As shown in Figure 13, the anastrozole composition comprising 10% sorption enhancer provided a pseudo-zero order elution rate for over 80 days.

Any suitable sorption enhancer(s) may be selected by one of ordinary skill in the art. Particularly suitable sorption enhancer(s) may include, for example, negatively-charged polymers, such as croscarmellose sodium, sodium carboxymethyl starch, sodium starch glycolate, sodium acrylic acid derivatives (e.g., sodium polyacrylate), cross-linked polyacrylic acid (e.g., CARBOPOL®), chondroitin sulfate, poly-glutamic acid, poly-aspartic acid, sodium carboxymethyl cellulose, neutral polymers, such as polyethylene glycol, polyethylene oxide, polyvinylpyrrolidone, and combinations thereof. In an exemplary embodiment, the sorption enhancer is croscarmellose sodium. The amount of the sorption enhancer is not particularly limited, but, when present, is preferably on the order of about 1-25 wt% of the solid dosage form, about 2-20 wt% of the solid dosage form, about 2-12 wt% of the solid dosage form, about 5-10 wt% of the solid dosage form (*e.g.*, about 5 wt% or about 10 wt% of the solid dosage form). The selection of the specific sorption enhancer may have an impact on the elution rate. Figure 14 depicts elution rates for an anastrozole composition comprising 10% sorption enhancer of three different types: sodium carboxymethyl starch (EXPLOTAB® obtainable from JRS PHARMA Gmbh & Co.KG with offices in Rosenberg, Germany), sodium starch glycolate (VIVASTAR® P also obtainable from obtainable from JRS PHARMA GmbH & Co.KG), and polyvinylpyrrolidone (POLYPLASDONE® ULTRA 10 available from Ashland Inc. with offices in Wayne, New Jersey), respectively. As shown in Figure 14, the anastrozole compositions comprising 10% sodium carboxymethyl starch or sodium starch glycolate provided a pseudo-zero order elution rate for over 80 days.

In one embodiment of the present invention, the at least one discrete solid dosage form comprises: 75-97 wt% API based on the total weight of the at least one discrete solid dosage form; 1-25 wt% of at least one sorption enhancer based on the total weight of the at least one discrete solid dosage form; and 0-5 wt% lubricant based on the total weight of the at least one discrete solid dosage form. For example, 85-95 wt% (*e.g.*, about 88 wt%) API based on the total weight of the at least one discrete solid dosage form; 5-20 wt% (*e.g.*, about 10 wt%) of at least one sorption enhancer (*e.g.*, croscarmellose sodium) based on the total weight of the at least one discrete solid dosage form; and 0-5 wt% (*e.g.,* 2%) lubricant (*e.g.*, stearic acid) based on the total weight of the at least one discrete solid dosage form. In another example, the at least one discrete solid dosage form comprises about 89.25% API, about 10% of at least one sorption enhancer (*e.g.*, croscarmellose sodium), and about 0.75% lubricant (*e.g.*, magnesium stearate). Preferably, each component of the drug delivery composition is provided in an amount effective for the treatment of the disease or condition being treated.

The therapeutically effective amount of the API may be delivered to the subject at a target range of average daily elution rate for the API. The target elution rate (mg/day) is based on the oral dose rate multiplied by the fractional oral bioavailability. The elution rate may be an average rate, *e.g.*, based on the mean average for a given period of time, such as a day (i.e., average daily elution rate). The average daily elution rate of the active pharmaceutical ingredient may vary in direct proportion to the amount of sorption enhancer in the drug delivery composition (*e.g.*, more sorption enhancer may provide for a higher average daily elution rate).

As previously discussed, the minimum value(s) for the average daily elution rate may be correlated to the trough value for an oral dosage version of the API (*e.g.,* based on the blood/plasma concentrations for oral formulations). Similarly, the maximum value(s) may be correlated to the peak value for an oral dosage version of the API (*e.g.*, the maximum blood/plasma concentration when an oral dosage is first administered or a pharmaceutically toxic amount). In other words, the target range is between maximum and minimum elution rates, respectively, which may be determined based on blood/plasma concentrations for equivalent oral dosage forms containing the same active. Table 5 provides examples of suitable APIs including the calculated target elution rate and maximum and minimum target values.

**Table 5**

| API | Oral Dosage | Bioavailability | Target Elution Rate | Min Elution Rate | Max Elution Rate |
|---|---|---|---|---|---|
| Anastrozole | 1 mg/day | 80-85% | 800 µg/day | 100 µg/day | 1,500 µg/day |
| Risperidone | 2, 4, 6, & 8 mg/day | ~ 70% | 1400-5600 µg/day | 1000 µg/day | 6000 µg/day |
| Dutasteride | 0.5 mg/day | ~ 60% | 300 µg/day | 100 µg/day | 1,000 µg/day |
| Oxybutynin HCl | 5, 10, 15, 20 mg/day | ~ 6% | 300, 600 & 900 µg/day | 100 µg/day | 1,500 µg/day |
| Oxybutynin Base | 5, 10, 15, 20 mg/day | ~ 6% | 300, 600 & 900 µg/day | 100 µg/day | 1,500 µg/day |
| Letrozole | 2.5 mg/day | 99% | 2,000 - 2,500 µg/day | 100 µg/day | 10,000 µg/day |
| Selegiline HCl | Buccal 1.25 mg/day | 90% | 1,100 - 1,400 µg/day | 100 µq/day | 3,000 µg/day |
| Tolterodine Tartrate | 1 & 2 mg/day | ~ 75% | 750 & 1,500 µg/day | 100 µg/day | 5,000 µg/day |
| Rivastigmine Tartrate | 2x per day 1.5 - 6 mg/day | 35% | 1,000 - 4,200 µg/day | 100 µg/day | 10,000 µg/day |
| Asenapine Maleate | Sublingual 5 & 10 mg, 2x daily | 35% | 1,750 - 3,500 µg/day | 100 µg/day | 10,000 µg/day |
| Paliperidone | 1.5; 3; 6 & 9 mg/day | 28% | 425; 850; 1,700 & 2,550 µg/day | 100 µg/day | 10,000 µg/day |
| Aripiprazole | 2, 5, 10, 15, 20 & 30 mg/day | 85% | 1,700-12,750 µg/day | 1000 µg/day | 15,000 µg/day |
| Rotigotine | 2, 4 & 6 mg transdermal | Transdermal 35% | 700; 1,400 & 2,100 µg/day | 100 µg/day | 5,000 µg/day |
| Folic acid | 1 mg/day | 60% | 600 µg/day | 100 µg/day | 1,500 µg/day |
| Vardenafil HCl | 5 mg 2x day | 15% | 1,500 µg/day | 100 µg/day | 5,000 µg/day |
| Fingolimod HCl | 0.5 mg/day | 93% | 450 µg/day | 100 µg/day | 2,000 µg/day |

The number and shape of the discrete dosage form(s) may be optimized to provide for the desired elution rates. For example, the discrete solid dosage forms may be of suitable shape to not fill the entire cavity of the reservoir. In one embodiment, the at least one discrete dosage form is substantially spherical in shape in that the solid dosage forms are spherical or nearly spherical. For example, the shape of the dosage form may not deviate from a perfect sphere by more than about 10%. The number of discrete dosage forms may be selected to provide a given elution rate. The discrete solid dosage forms may comprise more than one pellet (*e.g.*, 2-9 pellets). The number of discrete solid dosage forms may be directly proportional or related to the elution rate. In other words, a higher number of dosage forms may result in a higher average elution rate than a smaller number of dosage forms. Thus, it may be preferable to include more discrete solid dosage forms to give a higher elution rate (*e.g.*, 7-9 pellets). See *e.g.*, Figure 10 showing elution rates (μg/day) for anastrozole with reservoirs containing 7 or 9 pellets, where a composition containing 9 pellets produced a higher elution profile than a composition containing 7.

Subcutaneous Delivery of Risperidone *In Vivo*

Methods for effectively dosing antipsychotic drugs involve a great deal of variability and unpredictability in individual response. There are many adverse side effects associated with antipsychotic drugs, such as extrapyramidal symptoms, so that dosing strategies are often based on the avoidance of these effects. This often leads to extensive periods of trial and error, as well as patient suffering, while clinicians search for optimum dosing strategies, particularly for oral dosage forms. Moreover, the mechanisms of action of antipsychotic drugs are not entirely understood.

The applicants have discovered that when a drug delivery composition comprising risperidone or a pharmaceutically acceptable salt thereof is implanted into a subject in accordance with embodiments of the present invention, the resulting active moiety plasma concentration profile in the subject is significantly different from the active moiety plasma concentration profile achieved by an oral dose of risperidone in the same subject or in one or more different subjects. For example, the active moiety plasma concentration profile achieved with an implantable drug delivery composition in accordance with embodiments of the present invention can be compared to the plasma concentration profile achieved with one or more oral doses of risperidone as measured in the same subject, in a different subject, or as measured in two or more different subjects. For plasma concentration profiles achieved with one or more oral doses of risperidone as measured in two or more different subjects, the plasma concentration profile can be represented, e.g., as an average of one or more measurements. Despite the difference in plasma concentration profiles, the implantable risperidone compositions of the present invention remain therapeutically effective in treating one or more symptoms of a psychotic disorder.

As used herein, an "active moiety plasma concentration" of risperidone refers to the summed plasma concentrations of risperidone and its active metabolite 9-OH-risperidone. A "peak" plasma concentration refers to the highest plasma concentration observed over a specified period of time. A "trough" plasma concentration refers to the lowest plasma concentration observed over a specified period of time.

As described, for example, in Example 33 below, it has been observed that implantable compositions of the present invention are effective to provide a much smaller difference between the peak and trough active moiety plasma concentrations of risperidone over an extended period of time (e.g., over at least 30 days, at least 60 days, or at least 90 days, at least six months or at least one year) when compared to the difference between peak and trough active moiety plasma concentrations provided by a daily oral dose (e.g., 4 mg) of risperidone over one day. It has also been discovered that implantable compositions of the present invention may provide a peak active moiety plasma concentration over an extended period of time that is substantially equivalent to, or not significantly greater than, the trough active moiety plasma concentration achieved by the oral dose over one day.

Unlike the significant peak in an active moiety plasma concentration provided by an oral dosage form within hours of administration (*see, e.g.,* Figure 38, which shows that a peak active moiety plasma concentration of about 50 ng/mL was observed within about 3 hours following administration), implantable compositions of the present invention provide active moiety plasma concentrations that generally remain stable over time without a significant peak after the implant is administered (*see, e.g.,* Figure 39, which shows that a peak active moiety plasma concentration of only about 20 ng/mL was not observed until about 4 weeks following administration). Although the implantable risperidone compositions of the present invention do not provide a peak active moiety plasma concentration immediately following administration (*e.g.*, within 3 hours, within 6 hours, within 12 hours, within 18 hours, or within 24 hours), and the peak active moiety plasma concentrations are lower in comparison to those provided by oral dosage forms, the compositions of the present invention remain therapeutically effective over time.

According to an embodiment of the present invention, an implantable reservoir-based drug delivery composition comprises a formulation comprising risperidone or a pharmaceutically acceptable salt thereof, wherein the composition yields (i.e., is effective to provide) a therapeutically effective systemic active moiety plasma concentration for at least one month (*e.g.*, at least one month, at least three months, at least six months, at least one year, etc.) when subcutaneously administered to a subject, wherein the trough active moiety plasma concentration is not less than about 50% of the peak

active moiety plasma concentration over the at least one month (*e.g.*, at least one month, at least three months, at least six months, at least one year, etc.). According to particular embodiments, the trough active moiety plasma concentration may be not less than about 60%, or not less than about 70% of the peak active moiety plasma concentration over at least one month. For example, if a peak active moiety plasma concentration over at least one month is about 20 ng/mL, the active moiety concentration is maintained between about 10 ng/mL (about 50% of 20 ng/mL) to about 20 ng/mL over the course of the at least one month (i.e., the trough active moiety concentration does not drop below about 10 ng/mL). According to another example, if a peak active moiety plasma concentration over at least one month is about 30 ng/mL, the active moiety concentration is maintained between about 15 ng/mL (about 50% of 30 ng/mL) to about 30 ng/mL over the at least one month. According to another example, if a peak active moiety plasma concentration over at least one month is about 40 ng/mL, the active moiety concentration is maintained between about 20 ng/mL (about 50% of 40 ng/mL) to about 40 ng/mL over the at least one month.

According to particular embodiments, the formulation comprising risperidone or a pharmaceutically acceptable salt thereof comprises at least one discrete solid dosage form. The at least one discrete solid dosage form may comprise, for example, 75-97 wt% of the risperidone or pharmaceutically acceptable salt thereof based on the total weight of the at least one discrete solid dosage form; 1-25 wt% of at least one sorption enhancer based on the total weight of the at least one discrete solid dosage form; and 0-5 wt% lubricant based on the total weight of the at least one discrete solid dosage form. For example, the at least one discrete solid dosage form may comprise about 88 wt% risperidone or pharmaceutically acceptable salt thereof, about 10 wt% croscarmellose sodium, and about 2 wt% stearic acid based on the total weight of the at least one discrete solid dosage form. According to another example, the at least one discrete solid dosage form may comprise about 89.25 wt% risperidone or pharmaceutically acceptable salt thereof, about 10 wt% croscarmellose sodium, and about 0.75 wt% magnesium stearate based on the total weight of the at least one discrete solid dosage form.

Another embodiment of the present invention provides a method of treating one or more symptoms of a psychotic disorder comprising implanting a reservoir-based drug delivery composition into a subject, wherein the composition comprises a formulation comprising risperidone or a pharmaceutically acceptable salt thereof, wherein the composition yields a therapeutically effective systemic active moiety plasma concentration for at least one month (*e.g.*, at least one month, at least three months, at least six months, at least one year, etc.), wherein the trough active moiety plasma concentration is not less than about 50% of the peak active moiety plasma concentration over the at least one month (*e.g.*, at least one month, at least three months, at least six months, at least one year, etc.). Stated another way, a method for subcutaneously delivering a therapeutically effective amount of risperidone to a subject comprises providing the subject with a therapeutically effective systemic risperidone active moiety plasma concentration for at least one month, wherein the trough active moiety plasma concentration is not less than about 50% of the peak active moiety plasma concentration over the at least one month.

For example, as discussed above, the trough active moiety plasma concentration may be not less than about 60% of the peak active moiety plasma concentration over at least one month. Alternatively, the trough active moiety plasma concentration may be not less than about 70% of the peak active moiety plasma concentration over at least one month. Stated another way, the composition yields a peak-to-trough active moiety plasma concentration ratio in a subject that does not exceed about 2:1 over at least one month (*e.g.*, at least one month, at least three months, at least six months, at least one year, etc.). For example, the composition may yield a peak-to-trough active moiety plasma concentration ratio between about 1.4:1 to about 2:1 over at least one month.

According to another embodiment of the present invention, an implantable reservoir-based drug delivery composition comprises a formulation comprising risperidone or a pharmaceutically acceptable salt thereof, wherein the composition yields (i.e., is effective to provide) a therapeutically effective systemic active moiety plasma concentration for at least one month (*e.g.*, at least one month, at least three months, at least six months, at least one year, etc.) when subcutaneously administered to a subject, wherein the peak active moiety plasma concentration over the at least one month (*e.g.*, at least one month, at least three months, at least six months, at least one year, etc.) is not more than about 1.5 times the trough active moiety plasma concentration achieved by a once-daily oral dose (e.g., a 4 mg dose) of risperidone. For example, if the trough active moiety plasma concentration achieved by a once-daily oral dose of risperidone is about 20 ng/mL, then the peak active moiety plasma concentration achieved by the implantable composition is not more than about 1.5 x 20 ng/mL = 30 ng/mL over the at least one month. As another example, if the trough active moiety plasma concentration achieved by a once-daily oral dose of risperidone is about 30 ng/mL, then the peak active moiety plasma concentration achieved by the implantable composition is not more than about 1.5 x 30 ng/mL = 45 ng/mL over the at least one month.

Another embodiment of the present invention provides a method of treating one or more symptoms of a psychotic disorder comprising implanting a reservoir-based drug delivery composition into a subject, wherein the composition comprises a formulation comprising risperidone or a pharmaceutically acceptable salt thereof, wherein the composition yields (i.e., is effective to provide) a therapeutically effective systemic active moiety plasma concentration for at least one month (*e.g.*, at least one month, at least three months, at least six months, at least one year, etc.) when subcutaneously

administered to the subject, wherein the peak active moiety plasma concentration over the at least one month (*e.g.*, at least one month, at least three months, at least six months, at least one year, etc.) is not more than about 1.5 times the trough active moiety plasma concentration achieved by a once-daily oral dose (e.g., a 4 mg dose) of risperidone. Stated another way, a method for subcutaneously delivering a therapeutically effective amount of risperidone to a subject comprises providing the subject with a therapeutically effective systemic risperidone active moiety plasma concentration for at least one month, wherein the peak active moiety plasma concentration over the at least one month is not more than 50% of the trough active moiety plasma concentration in the subject after receiving a once-daily oral dose of risperidone.

For example, the composition may achieve a peak active moiety plasma concentration over the at least one month that is not more than about 1.25 times, or not more than about 1.1 times the trough active moiety plasma concentration achieved by a once-daily oral dose of risperidone. Alternatively, the composition may achieve a peak active moiety plasma concentration over the at least one month that is equivalent, or substantially equivalent, to the trough active moiety plasma concentration achieved by a once-daily oral dose of risperidone.

According to another embodiment of the present invention, an implantable reservoir-based drug delivery composition comprises a formulation comprising risperidone or a pharmaceutically acceptable salt thereof, wherein the composition yields (i.e., is effective to provide) a therapeutically effective systemic active moiety plasma concentration for at least one month (*e.g.*, at least one month, at least three months, at least six months, at least one year, etc.) when subcutaneously administered to a subject, wherein the peak active moiety plasma concentration over the at least one month (*e.g.*, at least one month, at least three months, at least six months, at least one year, etc.) is not more than about 50% of the peak active moiety plasma concentration achieved by a once-daily oral dose (e.g., a 4 mg dose) of risperidone. For example, if the peak active moiety plasma concentration achieved by a once-daily oral dose of risperidone is about 50 ng/mL, then the peak active moiety plasma concentration achieved by the implantable composition over the at least one month is not more than about 25 ng/mL. In another embodiment, the peak active moiety plasma concentration over the at least one month may be not more than about 40% of the peak active moiety plasma concentration achieved by the once-daily oral dose (e.g., a 4 mg dose) of risperidone. For example, if the peak active moiety plasma concentration achieved by a once-daily oral dose of risperidone is about 50 ng/mL, then the peak active moiety plasma concentration achieved by the implantable composition over the at least one month is not more than about 20 ng/mL.

According to other embodiments, the peak active moiety plasma concentration over the at least one month (*e.g.*, at least one month, at least three months, at least six months, at least one year, etc.) is not more than about 60% of the peak active moiety plasma concentration achieved by a once-daily oral dose (e.g., a 4 mg dose) of risperidone (*e.g.*, if the peak active moiety plasma concentration achieved by a once-daily oral dose of risperidone is about 50 ng/mL, then the peak active moiety plasma concentration achieved by the implantable composition over the at least one month is not more than about 30 ng/mL); or not more than about 70% of the peak active moiety plasma concentration achieved by a once-daily oral dose (e.g., a 4 mg dose) of risperidone (*e.g.*, if the peak active moiety plasma concentration achieved by a once-daily oral dose of risperidone is about 50 ng/mL, then the peak active moiety plasma concentration achieved by the implantable composition over the at least one month is not more than about 35 ng/mL). According to particular embodiments, the peak active moiety plasma concentration over the at least one month (*e.g.*, at least one month, at least three months, at least six months, at least one year, etc.) is between about 40% to about 70% (e.g., about 50% to about 60%) of the peak active moiety plasma concentration achieved by a once-daily oral dose (e.g., a 4 mg dose) of risperidone.

Another embodiment of the present invention provides a method of treating one or more symptoms of a psychotic disorder comprising implanting a reservoir-based drug delivery composition into a subject, wherein the composition comprises a formulation comprising risperidone or a pharmaceutically acceptable salt thereof, wherein the composition yields (i.e., is effective to provide) a therapeutically effective systemic active moiety plasma concentration for at least one month (*e.g.*, at least one month, at least three months, at least six months, at least one year, etc.), wherein the peak active moiety plasma concentration over the at least one month (*e.g.*, at least one month, at least three months, at least six months, at least one year, etc.) is not more than about 50% of the peak active moiety plasma concentration achieved by a once-daily oral dose (e.g., a 4 mg dose) of risperidone. Stated another way, a method for subcutaneously delivering a therapeutically effective amount of risperidone to a subject comprises providing the subject with a therapeutically effective systemic active moiety plasma concentration for at least one month, wherein the peak active moiety plasma concentration in the subject over the at least one month is not more than about 50% of the peak active moiety plasma concentration in the subject after receiving a once-daily oral dose of risperidone.

According to another embodiment of the present invention, an implantable reservoir-based drug delivery composition comprises a formulation comprising risperidone or a pharmaceutically acceptable salt thereof, wherein the composition yields (i.e., is effective to provide) a therapeutically effective systemic active moiety plasma concentration for at least one month (*e.g.*, at least one month, at least three months, at least six months, at least one year, etc.) when subcutaneously administered to a subject, wherein the difference between peak and trough active moiety plasma concentrations over the at least one month (e.g., over the at least one month, at least two months, or at least three months)

is at least 2 times less than (i.e., is not more than about 50% of) the difference between peak and trough active moiety plasma concentrations achieved by a once-daily oral dose (e.g., a 4 mg dose) of risperidone. For example, if the difference between peak and trough active moiety plasma concentrations achieved by a once-daily oral dose of risperidone is about 30 ng/mL, then the difference between peak and trough active moiety plasma concentrations achieved by the implantable composition over the at least one month is not more than about 15 ng/mL.

Another embodiment of the present invention provides a method of treating one or more symptoms of a psychotic disorder comprising implanting a reservoir-based drug delivery composition into a subject, wherein the composition comprises a formulation comprising risperidone or a pharmaceutically acceptable salt thereof, wherein the composition yields (i.e., is effective to provide) a therapeutically effective systemic active moiety plasma concentration for at least one month (*e.g.*, at least one month, at least three months, at least six months, at least one year, etc.) when subcutaneously administered to a subject, wherein the difference between peak and trough active moiety plasma concentrations over the at least one month (*e.g.*, at least one month, at least three months, at least six months, at least one year, etc.) is at least 2 times less than (i.e., is not more than about 50% of) the difference between peak and trough active moiety plasma concentrations achieved by a once-daily oral dose (e.g., a 4 mg dose) of risperidone (in the same subject). Stated another way, a method for subcutaneously delivering a therapeutically effective amount of risperidone to a subject comprises providing the subject with a therapeutically effective systemic risperidone active moiety plasma concentration for at least one month, wherein the difference in peak to trough active moiety plasma concentrations in the subject over the at least one month is at least 2 times less (i.e., is not more than about 50% of) than the difference in peak to trough active moiety plasma concentrations in the subject after receiving a once-daily oral dose of risperidone.

For example, the difference between peak and trough active moiety plasma concentrations over at least one month may be at least 3 times less (e.g., about 3.75 times less) than the difference between peak and trough active moiety plasma concentrations achieved by a once-daily oral dose (e.g., a 4 mg dose) of risperidone. For example, if the difference between peak and trough active moiety plasma concentrations achieved by a once-daily oral dose of risperidone is about 30 ng/mL, then the difference between peak and trough active moiety plasma concentrations achieved by the implantable composition over the at least one month is not more than about 10 ng/mL (at least 3 times less than about 30 ng/mL). Alternatively, the difference between peak and trough active moiety plasma concentrations over at least one month may be at least 4 times or at least 5 times (e.g., about 6 times) less than the difference between peak and trough active moiety plasma concentrations achieved by a once-daily oral dose (e.g., a 4 mg dose) of risperidone.

Drug Delivery Compositions, Subcutaneous Delivery Systems, and Kits

As previously noted, the drug delivery composition is long lasting, and the API may be delivered to the subject at a pseudo-zero order rate for an extended period of time (*e.g.*, at least about one month (about one month or greater), at least about three months (about three months or greater), at least about six months (about six months or greater), at least about one year (about one year or greater), or any period of time within those ranges).

According to one embodiment of the present invention, a subcutaneous delivery system for releasing an active pharmaceutical ingredient at a pseudo-zero order comprises an elastomeric reservoir implant comprising a rate-controlling excipient defining a reservoir. The rate-controlling excipient comprises a substantially non-porous elastomeric polymer having a relative content of hard segments and soft segments to provide an elution rate within a target range of average daily elution rate for the active pharmaceutical ingredient. The reservoir containing at least one discrete solid dosage form comprising the active pharmaceutical ingredient or a pharmaceutically acceptable salt thereof and an effective amount of at least one sorption enhancer to modulate the elution rate of the active pharmaceutical ingredient for release of a therapeutically effective amount of the active pharmaceutical ingredient within the target range at pseudo-zero order for a period of time of at least one month. The amount of sorption enhancer may be directly proportional to the average daily elution rate.

The drug delivery composition may be implanted into the subject in any suitable area of the subject using any suitable means and techniques known to one of ordinary skill in the art. For example, the composition may be implanted subcutaneously, *e.g.*, at the back of the upper arm or in the upper back (*e.g.*, scapular region), by directly depositing in or underneath the skin, a subcutaneous fat layer, or intramuscularly.

According to another embodiment of the present invention, a kit for subcutaneously placing a drug delivery composition includes a reservoir-based drug delivery composition comprising a rate-controlling excipient defining a reservoir containing at least one discrete solid dosage form and an implanter for inserting the reservoir-based drug delivery composition beneath the skin, and optionally instructions for implantation and explantation of the drug delivery composition. The rate-controlling excipient comprises a substantially non-porous, elastomeric polymer comprising soft and hard segments and the relative content of soft and hard segments of the polymer are selected to obtain an elution rate within a target range of average daily elution rate for the active pharmaceutical ingredient. The at least one discrete solid dosage form comprises an active pharmaceutical ingredient or a pharmaceutically acceptable salt thereof and at least one sorption enhancer in an amount effective to modulate the elution rate of the active pharmaceutical ingredient

to provide for release of the active pharmaceutical ingredient at pseudo-zero order within the target range at the therapeutically effective amount for a period of time of at least one month, and the amount of sorption enhancer may be directly proportional to the average daily elution rate.

The drug delivery composition may be delivered subcutaneously using any suitable equipment or techniques, *e.g.*, an implanter known to one ordinary skill in the art. The kits may comprise the drug delivery composition pre-loaded into the implanter or the drug delivery composition may be loaded by the doctor or other user. The implanter may be an implantation device, such as a syringe, cannula, trocar or catheter, that may be inserted into an incision made at the delivery site of the subject. Suitable implantation devices and implantation methods include the trocar and methods disclosed in US 7,214,206 and US 7,510,549, the disclosures of which are herein incorporated by reference in their entirety, for all purposes. Other suitable methods for implanting or otherwise positioning the compositions into the body, *e.g.*, by a doctor, are well known in the art. Removal and/or replacement may also be accomplished using suitable tools and methods known in the art. Kits may also comprise other equipment well known in the art, such as scalpels, clamps, suturing tools, hydration fluid, and the like.

Embodiments of Kits and Methods of Use Thereof

As used herein, the terms "proximal" and "distal" refer respectively to the directions closer to and further from the surgeon implanting the drug-eluting implant. For purposes of clarity, the distal portion of the insertion instrument is inserted into a subject and the proximal portion of the instrument remains outside the subject. For frame of reference in the figures, arrows marked "P" refer generally to the proximal direction and arrows marked "D" refer generally to the distal direction relative to the orientation of the items depicted in the figures.

Referring to Figure 41, a kit 10 for subcutaneously placing a drug-eluting implant in a subject is shown in accordance with one exemplary embodiment of the invention. Kit 10 includes a drug-eluting implant 100 and an insertion instrument 200 for subcutaneously placing the drug-eluting implant in a subject. Insertion instrument 200 is packaged with implant 100 pre-loaded into the insertion instrument 200. Although insertion instrument 200 is shown with a single drug-eluting implant 100, the instrument may be pre-loaded with two or more drug-eluting implants to be implanted into a subject. In addition, one or more drug-eluting implants 100 may be provided in kit 10 that are packaged separately from insertion instrument 200.

Referring to Figures 42-50, insertion instrument 200 includes a cannula 210 having a hollow shaft 230 where the cannula 210 connects to a front hub portion 223 of a handle portion 224 of the insertion instrument 200. The cannula and hence the hollow shaft 230 has a longitudinal axis 240 and forms an interior bore or lumen 232 that extends through the hollow shaft. The cannula 210 has a sharp distal end 234 that may be covered by a protective sheath 231, shown in Figure 42, when insertion instrument 200 is not in use. Insertion instrument 200 also includes a stop rod 250 capable of extending through (i) a rear hub portion 220 of the handle portion 224, (ii) the handle portion 224, (iii) the front hub portion 223 of the handle portion 224, and (iv) into hollow shaft 230. Cannula 210 is slidably displaceable over stop rod 250, as will be described in more detail.

In accordance with embodiments of the invention, the handle portion 224 may be formed with a number of different constructs. For example, handle portion 224 may be constructed from two injection molded portions 220a and 220b. Portions 220a and 220b may connect to one another with, for example, a plurality of pins (not shown) that mate with a corresponding plurality of sockets 228 (shown in Figure 50). When portions 220a and 220b are connected with one another, they collectively form the rear hub portion 220 and the front hub portion 223 of the handle portion 224, and the handle portion 224. As will be readily apparent to those skilled in the art, other constructions are possible for handle portion 224. Front hub portion 223 is adapted to receive the cannula 210 and stop rod 250 therein. Handle portion 224 is offset to one side of longitudinal axis 240 of hollow shaft 230, forming a lateral extension that can be gripped by the user. A pair of flanges 221 project outwardly from handle portion 224 for engagement with a user's fingers.

Distal end 234 of hollow passage 230 provides a passageway into lumen 232. Lumen 232 is adapted to receive and store drug-eluting implant 100 inside hollow shaft 230. Drug-eluting implant 100 can be loaded into lumen 232 by inserting the implant through open distal end 234 and into hollow shaft 230. In this arrangement, drug-eluting implant 100 can be pre-loaded into insertion instrument 200 by the manufacturer after the instrument 200 is assembled. Alternatively, drug-eluting implant 100 can be loaded into insertion instrument 200 by the user.

Referring to Figure 50, insertion instrument 200 is shown in a ready-to-use condition, with drug-eluting implant 100 pre-loaded into hollow shaft 230 of the cannula 210. Stop rod 250 includes a proximal end 252 and a distal end 254. Proximal end 252 of stop rod 250 includes a knob or handle portion 256. Distal end 254 of stop rod 250 includes an abutment face 259. Abutment face 259 is disposed within hollow shaft 230 in close proximity to drug-eluting implant 100.

Cannula 210 is slidably displaceable over stop rod 250, as noted above. Insertion instrument 200 has two settings, one which allows axial displacement of the cannula 210 over stop rod 250, and one that prevents axial displacement. The settings are controlled by the relative orientation of stop rod 250 with respect to cannula 210. Stop rod 250 is axially rotatable relative to longitudinal axis 240 of hollow shaft 230 between an unlocked orientation and a locked

orientation. In the unlocked orientation, cannula 210, front hub 223 and rear hub 220 are permitted to slide over stop rod 250. In the locked orientation, cannula 210, front hub 223 and rear hub 220 are prevented from sliding over stop rod 250.

Stop rod 250 includes a first locking feature defined by two longitudinal grooves 236 as best seen in Figure 42A. Grooves 236 extend along a portion of the length of stop rod 250. Handle portion 224 includes a second locking feature defined by a pair of projections 216 located on rear hub 220 as best seen in Figure 50. Each projection 216 extends radially inwardly toward horizontal axis 240 of the hollow shaft 230. When stop rod 250 is rotated into the locked orientation, grooves 236 are not in radial alignment with projections 216. As such, projections 216 engage stop rod 250, preventing cannula 210 from sliding over the stop rod toward proximal end 252 of the stop rod. When stop rod 250 is rotated to the unlocked orientation, grooves 236 are positioned in radial alignment with projections 216. Each groove 236 is sized to receive one of the projections 216. Therefore, in the unlocked position, each projection 216 is received within a groove 236 thereby permitting the cannula 210 to slide over stop rod 250 toward proximal end 252 of the stop rod 250. Stop rod 250 may include spaced markings thereon to indicate the distance that the cannula 210 has been moved proximally with respect to the proximal end 252 of the stop rod 250.

Insertion instrument 200 is packaged in the kit 10 with the drug-eluting implant 100 pre-loaded into the cannula 210. In alternative embodiments, the kit may be provided with an insertion instrument 200 and a drug-eluting implant 100, with the implant packaged separately from the instrument (i.e. the instrument is contained in one package in the kit, and the implant is contained in a separate package in the kit outside of the package containing the instrument). This packaging option allows a user to remove the drug-eluting implant from its packaging, inspect the implant, and load the implant into the instrument immediately before inserting the implant into the patient. This option also provides the user with the flexibility to substitute the implant provided in the kit with another implant that may be more suitable. Separate packaging may be used with kits that contain multiple implants having different properties. In such kits, the different implants may be individually packaged, and the user may select and open the appropriate implant, and load that implant into the instrument.

Kits in accordance with the invention may contain one or more implants that differ from one another in terms of the drug composition they contain, or other characteristic. For example, kit 10 is provided with a single drug-eluting implant 100. Implant 100 consists of a polymeric rate-controlling excipient, the excipient defining a reservoir containing at least one discrete solid dosage form. Other kit embodiments may be provided with two or more implants consisting of polymeric rate-controlling excipients. Although the figures schematically show a single implant 100 pre-loaded in insertion instrument 200, other embodiments in accordance with the invention may feature insertion instruments pre-loaded with two or more implants 100. Kits in accordance with embodiments of the invention may be provided with an insertion instrument pre-loaded with one or more implants, and one or more separately packaged implants that are not pre-loaded in the insertion instrument. Any number, type or combination of implants and instruments, whether packaged together or separately, may be provided in kits in accordance with embodiments of the invention. Thus, multiple implants having different therapeutic effects may be implanted in a single delivery procedure.

It is desirable in some instances to prepare a subcutaneous cavity beneath the cutis, prior to inserting insertion instrument 200 into the subject. The subcutaneous cavity provides a pocket that is large enough to receive the full length of the hollow shaft of the cannula, making it easier to deposit the implant in the proper location. For this reason, kits in accordance with embodiments of the invention may optionally include a separate instrument for preparing a subcutaneous cavity in a subject. Referring to Figure 51, an alternate kit 10’ in accordance with embodiments of the invention is shown. Kit 10’ includes the same insertion instrument 200 pre-loaded with a drug-eluting implant 100 as shown in prior figures. Kit 10’ also includes a second instrument, referred to as a tunneling instrument 300, for preparing a subcutaneous cavity in a subject. In addition, kit 10’ includes another drug-eluting implant 100’ that is packaged separately from the instruments.

Referring to Figures 52-60, tunneling instrument 300 has an elongated profile characterized by a horizontal axis H that is parallel to an insertion direction I, and a vertical axis V that is normal to the horizontal axis. Tunneling instrument 300 includes a blade 310 and a handle 350 attached to the blade. Blade 310 has a proximal end 312 and a distal end 314. Handle 350 also has a proximal end 352 and a distal end 354. In the present embodiment, distal end 354 of handle 350 is attached to proximal end 312 of blade 310 by a pair of screws 311. As will be readily apparent to those skilled in the art, blade 310 may be attached to handle 350 by any other means known in the art. When blade 310 is viewed from a side, as shown in Figure 52, the vertical height or dimension of the blade 310 with respect to vertical axis V gradually increases from distal end 314 toward the proximal end 312. Blade 310 includes a superior surface 316 and an inferior surface 318 opposite the superior surface. Inferior surface 318 extends between the proximal and distal ends 312, 314 of blade 310 and includes a substantially flat portion 322 that extends parallel to horizontal axis H. Superior surface 316 of blade 310 forms an inclined surface 324. Inclined surface 324 extends at an acute angle $\theta$ (as best seen in Figure 53) with respect to flat portion 322. Referring to Figure 56, blade 310 has a tapered profile with a maximum width at proximal end 312. The width of blade 310 tapers to a minimum width at the distal end 314. Each side of blade 310 follows a gradual curve. Blade 310 may be covered by a protective sheath 315, as shown in Figure 55, when tunneling instrument 300 is not in use.

Handle 350 includes a base portion 356 and an elongated gripping portion 358 extending from the base portion.

Base portion 356 has a superior surface 362 and an inferior surface 364 opposite the superior surface. Inferior surface 364 extends substantially coplanar with flat portion 322 of blade 310 to form a substantially continuous surface between the blade 310 and base portion 356. Gripping portion 358 extends upwardly from base portion 356 with respect to vertical axis V, and features a superior surface 366 and an inferior surface 368. An overmolded grip 372 extends over superior surface 366 of gripping portion 358 and superior surface 362 of base portion 362. Overmolded grip 372 may be formed of rubber or other material that provides a soft cushioned area to grip the instrument.

A method for subcutaneously placing a drug-eluting implant in a subject in accordance with embodiments of the invention will now be described with reference to the instruments in kit 10'. In this example, the method is used to subcutaneously place the implant in the arm of a human subject. The method begins by positioning the patient so that the surgeon has access to the location into which the implant is to be placed. For example, the patient may be positioned lying down on his or her back, with one arm flexed and turned to give the surgeon access to the inner aspect of the upper arm. The insertion site is then located on the upper arm. One possible insertion site is located approximately halfway between the patient's shoulder and elbow, and in the crease between the bicep and triceps. Once the insertion site is selected, the area around the site is swabbed and a local anesthetic is administered. Using a sterile scalpel, the surgeon makes an incision at the insertion site in a direction transverse to the long axis of the upper arm. The length of the incision should be as short as possible, but long enough to allow insertion of blade 310 of tunneling instrument 300 into the incision and under the skin. In alternate embodiments, the drug-eluting implant may be placed without the aid of a tunneling instrument. In such cases, the length of the incision should be as short as possible, but long enough to allow insertion of the cannula 210 of the insertion instrument 200 into the incision and under the skin.

For cases when a tunneling instrument 300 is used, the tunneling instrument 300 is removed from its packaging (if not already done) and placed in proximity to the incision, with flat portion 322 of blade 310 resting on or positioned just above the skin, and distal end 314 of the blade aligned with the incision. Inferior surface 364 of base portion 356 of handle 350 should also be resting on or positioned just above the skin, so that flat portion 322 of blade 310 is substantially parallel to the long axis of the patient's arm. Distal end 314 of blade 310 is then inserted through the incision and advanced into the patient's arm in a direction substantially parallel to the long axis of the arm, with the blade advancing immediately beneath the cutis and into the subcutaneous tissue. As blade 310 is advanced into the arm, the portion of the blade that enters the arm becomes gradually wider and wider in the horizontal and vertical directions due to the geometry of the blade 310 discussed above to expand the cavity created by the blade, forming a pocket or tunnel by blunt dissection. During insertion, the surgeon preferably maintains the insertion path just beneath the cutis and visibly raises the skin with blade 310 until a subcutaneous tunnel of sufficient length and width is created. Blade 310 is then removed from the patient's arm. For single-use kits, tunneling instrument 300 may be discarded.

Insertion instrument 200 is then removed from its packaging (if not already done). As noted above, insertion instrument 200 is packaged in kit 10' with drug-eluting implant 100 pre-loaded into cannula 210. Insertion instrument 200 is preferably packaged with stop rod 250 withdrawn from handle portion 224 and in the locked position as shown in Figure 41. Prior to use, the surgeon may wish to check that insertion instrument 200 is set with stop rod 250 rotated to the locked position, so as to prevent cannula 210 from being inadvertently advanced over the stop rod 250. The surgeon can determine if stop rod 250 is locked in a number of ways. For example, the surgeon can try sliding the cannula 210 over stop rod 250 to see if the stop rod is locked or unlocked. In addition, or as an alternative, the surgeon can check visible markings on insertion instrument 200 to determine whether stop rod 250 is locked or unlocked. In the illustrated example, rear hub portion 220 has a first indicia 222 in the form of a small horizontal line (as best seen in Figures 46 and 47). Stop rod 250 has a second indicia 251 and a third indicia 253 in the form of two horizontal lines that are radially offset from one another on the perimeter of the stop rod (as best seen in Figure 46). Stop rod 250 is rotatable relative to hub 220 to a first orientation that aligns the second indicia 251 with the first indicia 222. This first orientation corresponds to the locked position. Stop rod 250 is also rotatable relative to the hub 220 to a second orientation that aligns the third indicia 253 with the first indicia 222. This second orientation corresponds to the unlocked position. In preferred embodiments, the instrument includes a mechanism that provides tactile feedback to the surgeon when the stop rod 250 is rotated to the locked and unlocked positions. For example, the instrument may include an internal spring latch that engages a detent inside the hub to make an audible click after the stop rod is rotated to the locked position and/or unlocked position. The second and third indicia may also be color coded (e.g. green and red lines) to suggest which orientation is the unlocked position and which orientation is the locked position.

Once the locked position is confirmed, distal end 234 of cannula 210 is inserted into the incision and advanced into the subcutaneous tissue. Cannula 210 is advanced into the tunnel until a distal end 229 of hub 220 reaches the incision. At this stage, the hollow shaft 230 and hence, the implant 100, is positioned in the tunnel. Stop rod 250 is then rotated to the unlocked position in preparation for withdrawing cannula 210 from the incision. The unlocked position can be confirmed by an audible click, or by visual reference using the first indicia 222 and third indicia 253. The surgeon applies a gentle downward pressure on top of stop rod 250, preferably at or near proximal end 252, so as to fix the position of the stop rod relative to the patient's arm. Once stop rod 250 is fixed, the surgeon holds the stop rod 250 in the fixed position with one hand, and grasps the handle portion 224 of the insertion instrument 200 with the other hand.

The surgeon then applies a pulling force on handle portion 224 in a direction away from the incision to withdraw cannula 210 out of the incision. This may be performed in a single rapid motion to withdraw cannula 210 from the tunnel while leaving implant 100 in place in the tunnel. Depending on the length of implant 100 relative to the length of cannula 210 and other factors, the implant may be completely released from the hollow shaft 230 when the cannula 210 is partially removed from the incision (i.e. when a portion of the cannula 210 is withdrawn from the tunnel, while the remaining portion of the cannula 210 still remains in the tunnel). In other scenarios, implant 100 may be completely released from hollow shaft 230 only after the entire cannula 210 is completely removed from the incision (i.e. no portion of the cannula 210 remains in the tunnel).

Depending on factors such as friction, implant 100 may travel a small distance with cannula 210 as the cannula is withdrawn from the tunnel. In the event that implant 100 travels with cannula 210, the implant may travel far enough to contact abutment face 259 of stop rod 250. Abutment face 259 remains fixed inside the tunnel as cannula 210 is withdrawn, preventing the implant from being pulled out of the tunnel as the cannula 210 is withdrawn and removed from the incision.

In another embodiment, the implant 100 may be delivered as follows. Once the locked position is confirmed, distal end 234 of cannula 210 is inserted into the incision and advanced into the subcutaneous tissue. Cannula 210 is advanced into the tunnel until the distal end 234 of the cannula 210 is at the desired location of implant delivery in the tunnel. At this stage, the stop rod 250 is then rotated to the unlocked position in preparation for advancing the implant 100 toward the distal end 234 of the cannula 210. Similar to the previous embodiment, the unlocked position can be confirmed by an audible click, or by visual reference using the first indicia 222 and third indicia 253. The surgeon next pushes the stop rod 250 distally thereby advancing the implant 100 in the hollow shaft 230 toward the distal end 234 of the cannula 210. Once the implant is at the distal end 234, the surgeon then applies a gentle downward pressure on top of stop rod 250, preferably at or near proximal end 252, so as to fix the position of the stop rod relative to the patient's arm. Once stop rod 250 is fixed, the surgeon holds the stop rod 250 in the fixed position with one hand, and grasps the handle portion 224 of the insertion instrument 200 with the other hand. The surgeon then applies a pulling force on handle portion 224 in a direction away from the incision to withdraw cannula 210 out of the incision. Moving the handle portion 224 and hence, the cannula 210 in this manner while holding the stop rod 250 and hence, the implant 100, stationary, causes the implant 100 to be delivered out of the hollow shaft 230 and into the subject.

Once cannula 210 is withdrawn from the tunnel, the surgeon can check the position of implant 100 inside the tunnel. The surgeon can confirm proper placement of implant 100 by palpation and inspection of the incision. After correct placement is confirmed, the surgeon or other medical professional should cover the insertion site with sterile gauze, apply pressure to the insertion site, and follow any other post-operative procedures that are required.

To remove implant 100, an incision is made transverse to the long axis of the upper arm adjacent to one end of the implant. The incision should be of a size adequate to allow the tips of a hemostat to enter the tunnel. The tips of the hemostat are inserted into the incision and positioned on opposite sides of implant 100 in a position to grasp the implant. Implant 100 is then grasped and carefully pulled out of the pocket. After implant 100 is removed, the surgeon or other medical professional should cover the insertion site with sterile gauze, apply pressure to the insertion site, and follow any other post-operative procedures that are required.

Many elements shown in the illustrated embodiments are ornamental elements. The appearance of each ornamental element is not dictated by any function that the feature may perform. Rather, the appearance of each ornamental feature is selected based on aesthetic considerations. These ornamental elements may have a wide variety of shapes, colors, dimensions and surface textures that are selected individually, or in combination, to achieve a desired product appearance. For example, the shape, contours and relative dimensions of flanges 221 on insertion instrument 200 need not be as shown in Figures 41-49, which show the flanges as crescent-shaped elements. Flanges 221 may be larger or smaller, and/or have other shapes such as triangular or rectangular shapes, without changing any functional aspects of insertion instrument 200. Other ornamental aspects of insertion instrument 200 include, but are not limited to, the circular perimeter of handle portion 224 (which can be any shape), the common border between the circular perimeter of the handle portion and the perimeter of each flange, the rounded transitions between the handle portion and front hub 223, the off-centered axial position of the handle portion with respect to the front hub 223, and the differences in length and diameter among the various parts of the hub and stop rod. The tunneling tool 300 also has many ornamental features, including but not limited to the compound curvatures on superior surface 366 of gripping portion 358, the compound curvatures on inferior surface 368 of the gripping portion, the hourglass shaped profile of the gripping portion (Figure 56), the curved sides and rounded corners of overmolded grip 372 (Figures 52 and 53), the U-shape of base section 356 (Figures 54-56), and the contrasting surface texture between overmolded grip 372 and gripping portion 358. These ornamental aspects of the embodiments, which are only some of the ornamental aspects shown on the embodiments, do not influence the utilitarian aspects of the instruments or the functional purposes of any parts, and therefore may be replaced by an infinite number of other ornamental designs.

EXAMPLES

Embodiments of the present invention may be further understood by reference to the Examples provided below.

**Example 1: Anastrozole + Sorption Enhancer**

The follow general procedure was followed for the manufacture of an implant containing an API (Anastrozole, in this Example). Tubing was received in continuous length rolls and was cut to an appropriate starting length using a single-edged razor blade (or suitably sized scalpel). One end of each tubing section was thermally sealed, imparting a semi-spherical closure on the tip of the tubing section.

A discrete solid dosage form was prepared as follows. Anastrozole (88 wt%), croscarmellose sodium (10 wt%), and stearic acid (2 wt%) were premixed in a Turbula blender, slugged, milled, passed through a 500 micron sieve, and collected on a 212 micron sieve to achieve a particle size of between 212 and 500 microns. The anastrozole blend was compacted using a single punch tablet press.

Anastrozole pellets were then manually placed inside each sealed section of tubing. Each pellet-containing tubing section was sealed as described above, which imparted a semi-spherical seal on the open tip of the tubing section and completely sealed the implant. Sterilization was accomplished by gamma irradiation of the implants.

**Example 2: Anastrozole + Polyether-based Polyurethane**

A drug implant was manufactured as described in Example 1 using an aliphatic, polyether-based polyurethane (TECOFLEX® EG-93A polyurethane) as the tubing material. The implant dimensions were: a total length of the implant of about 45 mm, an outer diameter (O.D.) of 4.0 mm, an inner diameter (I.D.) of 3.6 mm, and a wall thickness of 0.2 mm. Nine pellets of the anastrozole blend were then placed inside the tubing. A total of about 370 mg anastrozole per implant was used. The implants were sterilized by gamma irradiation and placed in an elution bath of 0.9% saline (50 mL) at 37 °C. Regular exchanges of the elution media were analyzed by HPLC for over 400 days. Figure 4 depicts the elution rate of anastrozole from an aliphatic, polyether-based urethane implant over about 400 days.

**Example 3: Anastrozole + Polycarbonate-based Polyurethane**

A drug implant was manufactured as described in Example 1 using an aliphatic, polycarbonate-based polyurethane (CARBOTHANE® PC-3585A polyurethane) as the tubing material. The implant dimensions were: a total length of the implant of about 45 mm, an outer diameter (OD) of 4.0 mm, an inner diameter (ID) of 3.6 mm, and a wall thickness of 0.2 mm. A total of about 370 mg anastrozole per implant was used for the anastrozole blend. The implants were sterilized by gamma irradiation and placed in an elution bath of 0.9% saline (50 mL) at 37 °C. Regular exchanges of the elution media were analyzed by HPLC for about 100 days. Figure 5 depicts the elution rate of anastrozole from an aliphatic, polycarbonate-based urethane implant over about 100 days.

**Example 4: *In vivo* Implant Testing**

Three beagle dogs were dosed orally with ARIMIDEX® anastrozole for 2 weeks to establish peak and trough levels of anastrozole in beagles. These curves are shown in Figure 6 on the left side of the graph. Peak levels were about 37 ng/mL and trough levels were about 7 ng/mL. After a 1 week wash-out period, the same beagles, serving as their own controls, were then implanted with an anastrozole implant made according to Example 2, containing about 375 mg anastrozole, made with TECOFLEX® EG-93A polyurethane material.

Analysis of plasma samples out to 335 days of implantation indicated that anastrozole was eluted from the anastrozole implant. After an initial lag time of about 1 week, plasma levels of anastrozole were consistently above trough levels for the evaluated period as shown in Figure 6. At the conclusion of the experiment, the implants were removed and anastrozole levels returned to undetectable levels within one week.

**Example 5: Sorption Enhancer (Croscarmellose Sodium) + Polycarbonate-based Polyurethane Excipient**

Implants with varying amounts of croscarmellose sodium were prepared according to Example 1 using an aliphatic, polycarbonate-based polyurethane (CARBOTHANE® PC-3585A polyurethane) as the tubing material. In implant D, the croscarmellose sodium was omitted and replaced by additional anastrozole. Thus, the composition of implant D was 98% anastrozole, instead of 88%. Implant E contained 10 wt% croscarmellose sodium, as described in Example 1.

Implants D and E were then tested for *in vitro* elution, as described in Example 2. On average, less than 200 μg/day anastrozole eluted from implant D. In comparison, implant E eluted on average more than 500 μg/day anastrozole

after 20 days of implantation. The results are graphically illustrated in Figure 7. Thus, Figure 7 depicts the *in vitro* elution of anastrozole from aliphatic, polycarbonate-based urethane implants containing 0 and 10wt% croscarmellose sodium.

**Example 6: Varying the Sorption Enhancer**

Implants were prepared according to Example 1, but with the following sorption enhancers at 10 wt% in place of the 10 wt% croscarmellose sodium: sodium carboxymethyl starch (EXPLOTAB®), sodium starch glycolate (VIVASTAR® P), and polyvinylpyrrolidone (POLYPLASDONE® ULTRA 10), respectively. Figure 14 depicts elution rates for an anastrozole composition comprising 10% sorption enhancer of the three different types. As shown in Figure 14, the anastrozole compositions comprising 10% sodium carboxymethyl starch or sodium starch glycolate provided a pseudo-zero order elution rate for over 80 days.

Additional variations of the sorption enhancers may also be tested including types of sodium starch glycolate (*e.g.*, VIVASTAR® PSF and EXPLOTAB® CLV), and polyvinylpyrrolidone (*e.g.*, POLYPLASDONE® ULTRA, POLYPLASDONE® XL, and POLYPLASDONE® XL 10).

**Examples 7: Polyurethanes with polyether soft segment for release of Letrozole**

The drug implants were manufactured as described in Example 1, but using TECOFLEX® EG-80A polyurethane and TECOFLEX® EG-85A polyurethane, polyurethanes with a polyether soft segment, as the tubing material and letrozole, as the aromatase inhibitor. The implant dimensions were a total length of the implant of about 45 mm, an OD of 4.0 mm, an ID of 3.6 mm, and a wall thickness of 0.2 mm. A total of about 370 mg letrozole was loaded into the implants with 10 wt% croscarmellose and 2 wt% stearic acid. The implants were sterilized by gamma irradiation and placed in an elution bath consisting of 50 mL 0.9% saline at 37 °C. Weekly exchanges of the elution media were analyzed by HPLC for over 120 days. The graph is shown in Figure 15.

**Example 8: Polyurethane with polyrcarbonate soft segment for release of Anastrozole**

The drug implant was manufactured as described in Example 1 using CARBOTHANE® MPD00311D polyurethane, a polyurethane with a polycarbonate soft segment, as the tubing material and anastrozole, as an aromatase inhibitor. The implant dimensions were a total length of the implant of about 45 mm, an OD of 4.0 mm, an ID of 3.6 mm, and a wall thickness of 0.2 mm. A total of about 370 mg anastrozole was loaded into the implant with 10 wt% croscarmellose and 2 wt% stearic acid. The implant was sterilized by gamma irradiation and placed in an elution bath consisting of 50 mL 0.9% saline at 37 °C. Weekly exchanges of the elution media were analyzed by HPLC for over 100 days. The graph is shown in Figure 16.

**Example 9: Polyurethane with polysilicone soft segment for release of Anastrozole**

The drug implant was manufactured as described in Example 1 using ELAST-EON™ polyurethane, a polyurethane with a polysilicone soft segment, as the tubing material and anastrozole, as an aromatase inhibitor. The implant dimensions were a total length of the implant of about 45 mm, an OD of 4.0 mm, an ID of 3.6 mm, and a wall thickness of 0.2 mm. A total of about 370 mg of anastrozole was loaded into the implant with 10 wt% croscarmellose and 2 wt% stearic acid. The implant was sterilized by gamma irradiation and placed in an elution bath consisting of 50 mL 0.9% saline at 37 °C. Weekly exchanges of the elution media were analyzed by HPLC for about 50 days. The graph is shown in Figure 17.

**Example 10: Polyamide with polyether soft segment for release of Anastrozole**

The drug implants were manufactured as described in Example 1 using two types of PEBAX® polyether-amide, a polyamide with a polyether soft segment having different Shore hardness values, as the tubing material and anastrozole, as an aromatase inhibitor. The first two numbers denote the Shore hardness (i.e., PEBAX® 2533 polyether-amide has a Shore hardness of 25 and PEBAX® 3533 polyether-amide has a Shore hardness of 35). The implant dimensions were a total length of the implant of about 25 mm, an OD of 4.0 mm, an ID of 3.6 mm, and a wall thickness of 0.2 mm. A total of about 200 mg of anastrozole was loaded into the implants with 10 wt% croscarmellose and 2 wt% stearic acid. The implants were sterilized by gamma irradiation and placed in an elution bath consisting of 50 mL 0.9% saline at 37 °C. Weekly exchanges of the elution media were analyzed by HPLC for over 11 weeks. The graph is shown in Figure 12.

**Example 11: Influence of API Load on drug delivery**

Implants were manufactured as described in Example 1 with TECOFLEX® EG-93A polyurethane tubing, but in this series of implants the amount of the API (anastrozole) was varied from about 125 mg to about 540 mg per implant. The implants were sized accordingly to accommodate the number of pellets. The implants were sterilized by gamma irradiation and placed in an elution bath consisting of 50 mL 0.9% saline at 37 °C. Weekly exchanges of the elution media were analyzed by HPLC for over 70 days indicate a linear release of anastrozole with increased load into the implant. The graph is shown in Figure 18.

**Example 12: Variation of the Sorption Enhancer**

Implants were manufactured as described in Example 1 with TECOFLEX® EG-93A polyurethane tubing and using anastrozole, as the aromatase inhibitor. The two types of sorption enhancers were sodium carboxymethyl starch (EXPLOTAB®) and sodium starch glycolate (VIVASTAR® P). The implant dimensions were a total length of the implant of about 45 mm, an OD of 4.0 mm, an ID of 3.6 mm, and a wall thickness of 0.2 mm. A total of about 370 mg of anastrozole was loaded into the implants with 10 wt% sorption enhancer and 2 wt% stearic acid. The implants were sterilized by gamma irradiation and placed in an elution bath consisting of 50 mL 0.9% saline at 37 °C. Weekly exchanges of the elution media were analyzed by HPLC for over 16 weeks. The graph is shown in Figure 19.

**Example 13: Influence of sorption enhancer concentration on drug delivery from polyurethane with polycarbonate soft segment**

Implants were manufactured as described in Example 1 with CARBOTHANE PC-3585A tubing and anastrozole as the aromatase inhibitor. In one implant series, the excipient sodium croscarmellose was omitted and replaced by more drug substance anastrozole so that the implant's final composition was 98% anastrozole instead of 88% as in Example 1. Results are shown in Figure 20.

**Example 14: Influence of the wall thickness on anastrozole elution rates**

The drug implants were manufactured as described in Example 1 using TECOFLEX® EG-93A polyurethane, a polyurethane with a polyether soft segment, as the tubing material and anastrozole as an aromatase inhibitor. The implant dimensions were a total length of the implant of about 45 mm, an OD of 4.0 mm and, the wall thickness was varied from 0.15 mm to 0.25 mm. A total of about 370 mg anastrozole was loaded into the implant, with 10% croscarmellose and 2 % stearic acid. The implants were sterilized by gamma irradiation and placed in an elution bath consisting of 50 mL 0.9% saline at 37 °C. Weekly exchanges of the elution media were analyzed by HPLC for over 14 weeks. The graph is shown in Figure 21.

**Example 15: Influence of sorption enhancer concentration on elution rates of anastrozole**

The drug implants were manufactured as described in Example 1 using TECOFLEX® EG-93A polyurethane, a polyurethane with a polyether soft segment, as the tubing material and anastrozole as an aromatase inhibitor. The implant dimensions were a total length of the implant of about 45 mm, an OD of 4.0 mm, and a wall thickness of 0.2 mm. A total of about 370 mg anastrozole was loaded into the implant, with 2 % stearic acid and varying concentrations of croscarmellose from 10% to 5% and 0%. The implants were sterilized by gamma irradiation and placed in an elution bath consisting of 50 mL 0.9% saline at 37 °C. Weekly exchanges of the elution media were analyzed by HPLC for over 11 weeks. The graph is shown in Figure 22.

**Example 16: Risperidone with and without Sorption Enhancer**

The follow general procedure was followed for the manufacture of an implant containing an API (Risperidone, in this Example). Tubing was received in continuous length rolls and was cut to an appropriate starting length using a single-edged razor blade (or suitably sized scalpel). One end of each tubing section was thermally sealed and a semi-spherical closure was imparted on the tip of the tubing section. TECOFLEX® EG-80A polyurethane was used in this example as the polymer excipient.

A discrete solid dosage form was prepared as follows. Risperidone (88 wt%), croscarmellose sodium (10 wt%), and stearic acid (2 wt%) were premixed in a Turbula blender, slugged, milled, passed through a 500 micron sieve, and collected on a 212 micron sieve to achieve a particle size of between 212 and 500 microns. For implant A, a total of about 370 mg risperidone was loaded into the implant with 10% croscarmellose and 2% stearic acid. For elution comparison, implant B was also prepared without the croscarmellose (i.e., 98 wt% risperidone), with a total amount of risperidone of about 400 mg.

The risperidone blend was compacted using a single punch tablet press. Risperidone pellets were then manually placed inside each sealed section of tubing. Each pellet-containing tubing section was sealed and a semi-spherical seal was imparted on the open tip of the tubing section and completely sealed the implant.

The implant dimensions were a total length of about 45 mm, an outer diameter (O.D.) of 4.0 mm, an inner diameter (I.D.) of 3.6 mm, and a wall thickness of 0.2 mm. The implants were sterilized by gamma irradiation and placed in an elution bath of 0.9% saline (200 mL) at 37°C. Weekly exchanges of the elution media were analyzed by HPLC and the results are shown in Figure 23. It is noteworthy that significantly higher daily elution rates were achieved with a sorption enhancer, in comparison to an implant without the sorption enhancer.

## Example 17: Anastrozole Implants With Croscarmellose Sodium

Drug containing pellets were manufactured as described in Example 1, and the polymeric sorption enhancer croscarmellose sodium was added to the drug blend. Nine pellets of a drug blend with various concentrations of anastrozole and croscarmellose, with the remainder being 2 % stearic acid as lubricant, were placed into a polyurethane tubing (TECOFLEX® EG-93A). The implants were sterilized by gamma irradiation and placed in an elution batch consisting of 50 mL saline at 37 °C. Weekly exchanges of the elution media were analyzed by HPLC for 13 weeks. The graph is shown in Figure 24. As can be seen in the Figure 24, even the smallest percentage of the polymeric sorption enhancer increased the drug release compared to no added sorption enhancer (see Figure 22 for comparison). At the smallest concentration of croscarmellose of 2 %, elution was about 250 μg/day, while the highest concentration of croscarmellose of 10% achieved an elution rate of about 850 μg/day, enabling the control of the drug release through the addition of various levels of sorption enhancer.

## Example 18: Anastrozole Implant With Sodium Polyacrylate

Drug containing pellets were manufactured as described in Example 1, and the polymeric sorption enhancer sodium polyacrylate was added to the drug blend. Nine pellets of a drug blend with various concentrations of anastrozole and sodium polyacrylate, with the remainder being 2 % stearic acid as lubricant, were placed into a polyurethane tubing (TECOFLEX® EG-93A). The implants were sterilized by gamma irradiation and placed in an elution batch consisting of 50 mL saline at 37 °C. Weekly exchanges of the elution media were analyzed by HPLC for 17 weeks. The graph is shown in Figure 25. As can be seen in the Figure 25, the polymeric sorption enhancer sodium polyacrylate increased the drug release compared to no added sorption enhancer (see Figure 22 for comparison). Increasing the amount of sodium polyacrylate from 10% of the drug blend to 14% of the drug blend increased the drug release rate.

## Example 19: Risperidone Implants With Croscarmellose Sodium

Drug containing pellets were manufactured as described in Example 1, and the polymeric sorption enhancer croscarmellose sodium was added to the drug blend. Nine pellets of a drug blend with various concentrations of risperidone and croscarmellose, with the remainder being 2 % stearic acid as lubricant, were placed into a polyurethane tubing (TECOFLEX® EG-80A). The implants were sterilized by gamma irradiation and placed in an elution batch consisting of 500 mL saline at 37 °C. Weekly exchanges of the elution media were analyzed by HPLC for 11 weeks. The graph is shown in Figure 26. As can be seen in the Figure 26, even the smallest percentage of the polymeric sorption enhancer increased the drug release compared to no added sorption enhancer (see Figure 23 for comparison). At the smallest concentration of croscarmellose of 2 %, elution was about 1,450 μg/day at week 2, before slowly declining to about 850 μg/day at week 11, while the highest concentration of croscarmellose at 12% achieved an elution rate of about 1,800 μg/day at week 2, before slowly declining to about 1,400 μg/day at week 11, enabling the control of the drug release through the addition of various levels of sorption enhancers.

## Example 20: Risperidone Implant with Polyethylene Oxide, Sodium Polyacrylate or Chondroitin Sulfate

Drug containing pellets were manufactured as described in Example 1 and the polymeric sorption enhancers polyethylene oxide, sodium polyacrylate or chondroitin sulfate were added to the drug blend. Nine pellets of a drug blend with 88% risperidone and 10% polymeric sorption enhancer, with the remainder being 2 % stearic acid as lubricant, were placed into a polyurethane tubing (TECOFLEX® EG-80A). The total risperidone load was 390 mg. The implants were sterilized by gamma irradiation and placed in an elution batch consisting of 500 mL saline at 37 °C. Weekly exchanges of the elution media were analyzed by HPLC for 7 weeks. The graph is shown in Figure 27. As can be seen in the Figure 27, the polymeric sorption enhancer increased the drug release compared to no added sorption enhancer

(see Figure 23 for comparison). Different sorption enhancers demonstrated different enhancement of the elution rate allowing for control of the drug release.

**Example 21: Risperidone Implant with Sodium Carboxymethyl Cellulose**

Drug containing pellets were manufactured as described in Example 1, and the polymeric sorption enhancer sodium carboxymethyl cellulose was added to the drug blend. The sodium carboxymethyl cellulose had a degree of substitution of about 0.7 and a low viscosity (Aqualon® Sodium carboxymethyl cellulose, 7L2). Nine pellets of a drug blend with 88% risperidone and 10% sodium carboxymethyl cellulose, with the remainder being 2 % stearic acid as lubricant, were placed into a polyurethane tubing (TECOFLEX® EG-80A). The total risperidone load was 390 mg. The implants were sterilized by gamma irradiation and placed in an elution batch consisting of 500 mL saline at 37 °C. Weekly exchanges of the elution media were analyzed by HPLC for 10 weeks. The graph is shown in Figure 28. As can be seen in the Figure 28, the polymeric sorption enhancer increased the drug release compared to no added sorption enhancer (see Figure 23 for comparison) and the elution profile is similar to croscarmellose sodium.

**Example 22: Drug Implants Using Polyether-Block-Amide (PEBAX®) Polymers**

The follow general procedure was followed for testing various API's with polyether-block-amide polymers. Tubing was received in continuous length rolls and was cut to an appropriate starting length using a single-edged razor blade (or suitably sized scalpel). One end of each tubing section was thermally sealed imparting a semi-spherical closure on the tip of the tubing section.

The API and a sorption enhancer, e.g. croscarmellose sodium, were premixed in a Turbula blender. Stearic acid as a lubricant is added and the mixture again mixed in a Turbula blender. The standard final drug blend was 88% API, 10% sorption enhancer, and 2% stearic acid powder.

The API blend was compacted using a single punch tablet press. Drug pellets were manually placed inside each sealed section of tubing. The open section of each pellet-containing tubing section was then sealed into a semi-spherical seal. Sterilization was accomplished by gamma irradiation of the implants.

**Example 23: Guanfacine Free Base Release from PEBAX® Implants**

The drug implants were manufactured as described in Example 26 using PEBAX® 2533 and PEBAX® 3533 as the tubing material and guanfacine free base as an API. The implant dimensions were a total length of the implant of about 50 mm, an OD of 4.0 mm, an ID of 3.6 mm and a wall thickness of 0.2 mm. A total of about 375 mg guanfacine were loaded into the implant with 10% croscarmellose and 2 % stearic acid. The implant were sterilized by gamma irradiation and placed in an elution batch consisting of 200 mL 0.9% saline at 37 °C. Weekly exchanges of the elution media were analyzed by HPLC for over 50 days. The graph is shown in Figure 29.

**Example 24: Paliperidone Release from PEBAX® Implants**

The drug implants were manufactured as described in Example 26 using PEBAX® 2533 and PEBAX® 3533 as the tubing material and paliperidone as an API. The implant dimensions were a total length of the implant of about 40 mm, an OD of 4.0 mm, an ID of 3.6 mm and a wall thickness of 0.2 mm. A total of about 250 mg paliperidone were loaded into the implant with 10% croscarmellose and 2 % stearic acid. The implant were sterilized by gamma irradiation and placed in an elution batch consisting of 200 mL 0.9% saline at 37 °C. Weekly exchanges of the elution media were analyzed by HPLC for over 70 and 110 days, respectively. The graph is shown in Figure 30.

**Example 25: Letrozole Release from PEBAX® Implants**

The drug implants were manufactured as described in Example 26 using PEBAX® 2533 and PEBAX® 3533 as the tubing material and letrozole as an API. The implant dimensions were a total length of the implant of about 50 mm, an OD of 4.0 mm, an ID of 3.6 mm and a wall thickness of 0.2 mm. A total of about 350 mg letrozole were loaded into the implant with 15% croscarmellose and 2 % stearic acid, a variation to the standard blend. The implant were sterilized by gamma irradiation and placed in an elution batch consisting of 600 mL 0.9% saline at 37 °C. Weekly exchanges of the elution media were analyzed by HPLC for over 70 days, respectively. The graph is shown in Figure 31.

**Example 26: Delivery of Fingolimod, Varenicline, and Oxybutynin from Polyurethane Implants**

The follow general procedure was followed for testing different base and salt forms of the API's fingolimod,

varenicline, and oxybutynin with various polyurethane implants. Tubing was received in continuous length rolls and was cut to an appropriate starting length using a single-edged razor blade (or suitably sized scalpel). One end of each tubing section was thermally sealed imparting a semi-spherical closure on the tip of the tubing section.

The API and an sorption enhancer, e.g. croscarmellose sodium, were premixed in a Turbula blender. A lubricant was added and the mixture again mixed in a Turbula blender. The standard drug blend was 88% API, 10% sorption enhancer, and 2% lubricant.

The drug blend was compacted using a single punch tablet press. Drug pellets were manually placed inside each sealed section of tubing. The open section of each pellet-containing tubing section was then sealed into a semi-spherical seal. Sterilization was accomplished by gamma irradiation of the implants.

**Example 27: Fingolimod Delivery from Polyurethane Implants**

The drug implants were manufactured as described in Example 26 using TECOFLEX® EG-80A polyurethane or TECOFLEX® EG-93A polyurethane as the tubing materials and either fingolimod hydrochloride or fingolimod free base as the API. The implant dimensions were a total length of the implant of about 40 mm, an OD of 4.0 mm, an ID of 3.6 mm and a wall thickness of 0.2 mm. A total of about 250 mg fingolimod were loaded into the implants with 10% croscarmellose and 2 % stearic acid. The implants were sterilized by gamma irradiation and placed in an elution batch consisting of 800 mL 0.9% saline at 37 °C. Weekly exchanges of the elution media were analyzed by HPLC for up to 30 weeks. The graph is shown in Figure 32. The drug release from the implants was about 7-fold higher with the fingolimod free base from the same polymers as compared to the fingolimod hydrochloride.

**Example 28: Varenicline Tartrate Delivery from Polyurethane Implants**

The drug implants were manufactured as described in Example 26 using TECOFLEX® EG-80A, TECOFLEX® EG-85A, TECOFLEX® EG-93A, TECOFLEX® EG-100A, TECOFLEX® EG-65D, or TECOFLEX® EG-68D polyurethanes as the tubing materials and varenicline tartrate as the API. The implant dimensions were a total length of the implant of about 50 mm, an OD of 4.0 mm, an ID of 3.6 mm and a wall thickness of 0.2 mm. A total of about 350 mg varenicline tartrate were loaded into each implant with 10% croscarmellose and 2 % stearic acid. The implants were sterilized by gamma irradiation and placed in an elution batch consisting of 200 mL 0.9% saline at 37 °C. Weekly exchanges of the elution media were analyzed by HPLC for up to 11 weeks. The graph is shown in Figure 33. Either no drug was released from the implants using varenicline tartrate (for the TECOFLEX® EG-93A, TECOFLEX® EG-100A, TECOFLEX® EG-65D, and TECOFLEX® EG-68D implants), or only after a long incubation phase, the drug was released but without any control over the release, as indicated by steadily increasing release rates for TECOFLEX® EG-80A and TECOFLEX® EG-85A.

**Example 29: Varenicline Free Base Delivery from Polyurethane Implants**

The drug implant was manufactured as described in Example 26 using TECOFLEX® EG-93A polyurethane as the tubing material and varenicline free base as the API. The implant dimensions were a total length of the implant of about 40 mm, an OD of 4.0 mm, an ID of 3.6 mm and a wall thickness of 0.2 mm. A total of about 250 mg varenicline free base were loaded into the implant with 10% croscarmellose and 2 % stearic acid. The implant was sterilized by gamma irradiation and placed in an elution batch consisting of 100 mL 0.9% saline at 37 °C. Weekly exchanges of the elution media were analyzed by HPLC for up to 11 weeks. The graph is shown in Figure 34. In contrast to the tartrate salt of varenicline, in which no drug was released from TECOFLEX® EG-93A, the free base form of varenicline was released at about 1,750 μg/day.

**Example 30: Oxybutynin Hydrochloride Delivery from Polyurethane Implants**

The drug implants were manufactured as described in Example 26 using TECOFLEX® EG-80A, TECOFLEX® EG-85A, TECOFLEX® EG-93A, TECOPHILIC® HP-60D-05, and TECOPHILIC® HP-60D-10 polyurethanes as the tubing materials and oxybutinin hydrochloride as the API. The implant dimensions were a total length of the implant of about 50 mm, an OD of 4.0 mm, an ID of 3.6 mm and a wall thickness of 0.2 mm. A total of about 375 mg oxybutynin hydrochloride were loaded into the implants with 10% croscarmellose and 2 % stearic acid. The implants were sterilized by gamma irradiation and placed in an elution batch consisting of 0.9% saline at 37 °C. Weekly exchanges of the elution media were analyzed by HPLC. The graph is shown in Figure 35. Oxybutynin HCl failed to elute from the TECOFLEX® EG-93A, TECOPHILIC® HP-60D-05, and TECOPHILIC® HP-60D-10 implants (data not shown), whereas the TECOFLEX® EG-80A and TECOFLEX® EG-85A implants demonstrated suitable release rates.

**Example 31: Oxybutynin Free Base Delivery from Polyurethane Implants**

The drug implants were manufactured as described in Example 26 using TECOFLEX® EG-80A, TECOFLEX® EG-85A, TECOFLEX® EG-93A, TECOPHILIC® HP-60D-05, and TECOPHILIC® HP-60D-10 polyurethanes as the tubing materials and oxybutinin free base as the API. The implant dimensions were a total length of the implant of about 50 mm, an OD of 4.0 mm, an ID of 3.6 mm and a wall thickness of 0.2 mm. A total of about 370 mg oxybutynin free base were loaded into the implants with 10% croscarmellose and 2 % stearic acid. The implants were sterilized by gamma irradiation and placed in an elution batch consisting of 0.9% saline at 37 °C. Weekly exchanges of the elution media were analyzed by HPLC. The graph is shown in Figure 36. Contrary to oxybutynin HCl, oxybutynin free base essentially streamed out of the TECOFLEX® EG-80A and TECOFLEX® EG-85A implants as if there was no rate-controlling membrane (data not shown), whereas the TECOFLEX® EG-93A, TECOPHILIC® HP-60D-05, and TECOPHILIC® HP-60D-10 implants provided suitable and controllable release rates.

**Example 32: Risperidone Implant Embodiment**

The following general procedure was followed for the manufacture of an implant containing risperidone as the API. Tubing was received in continuous length rolls and was cut to an appropriate starting length using a single-edged razor blade (or suitably sized scalpel). One end of each tubing section was thermally sealed imparting a semi-spherical closure on the tip of the tubing section.

Risperidone and croscarmellose sodium were premixed in a Turbula blender. Magnesium stearate as a lubricant was added and the mixture again mixed in a Turbula blender. The final drug blend was 89.25% risperidone, 10% croscarmellose sodium, and 0.75% magnesium stearate.

The risperidone blend was compacted using a single punch tablet press with each tablet weighing about 67 mg. Eight risperidone drug pellets were manually placed inside each sealed section of tubing for a total of about 480 mg risperidone per implant. The open section of each pellet-containing tubing section was then sealed into a semi-spherical seal. Sterilization was accomplished by gamma irradiation of the implant. The implant was placed in an elution batch consisting of 0.9% saline at 37 °C. Weekly exchanges of the elution media were analyzed by HPLC. The graph is shown in Figure 37.

**Example 33: Risperidone Implant *In Vivo***

The following general procedure was followed for a clinical study of a risperidone implant in humans. Six schizophrenic subjects were stabilized on a daily 4 mg oral risperidone dose. Prior to implant insertion, each subject's trough risperidone, 9-OH-risperidone, and active moiety plasma concentration (i.e., summed concentrations of risperidone + 9-OH-risperidone) following a 4 mg oral dose of risperidone were assessed; a full 24 hr concentration-time profile was also obtained on Day 1 following a 4 mg oral dose of risperidone to access the subject's risperidone, OH-risperidone and active moiety Cmax values. A graph of the mean risperidone active moiety plasma concentration (ng/mL) for the six subjects over 24 hours, following oral administration of 4 mg risperidone, is shown in Figure 38.

The risperidone implant used in this study was manufactured by the following general procedure. Tubing was received in continuous length rolls and was cut to an appropriate starting length using a single-edged razor blade (or suitably sized scalpel). One end of each tubing section was thermally sealed by the following process: One tubing section was loaded into an appropriately-adjusted holding fixture of a sealing machine and a sealing cycle was initiated as described above in Example 1, which imparted a semi-spherical closure on the tip of the tubing section. TECOFLEX® EG-80A polyurethane was used as the polymer excipient.

A discrete solid dosage form was prepared by premixing risperidone (88 wt%), croscarmellose sodium (10 wt%), and stearic acid (2 wt%) in a Turbula blender. The drug blend was compacted using a single punch tablet press. Drug pellets were manually placed inside each sealed section of tubing. The open section of each pellet-containing tubing section was then sealed into a semi-spherical seal. Sterilization was accomplished by gamma irradiation of the implants. A total of about 375 mg risperidone were loaded into the implant with 10% croscarmellose and 2% stearic acid.

On the day of implantation, each subject was given a 2 mg oral dose of risperidone to compensate for any possible lag time in risperidone release from the implant. Following implantation, blood samples were obtained daily for the first 2 weeks and weekly thereafter until explantation for bioanalysis. The timed average concentrations ($C_{avg}$) of risperidone, 9-OH-risperdone and active moiety following implantation were assessed from day 3 after implantation to immediately prior to explanation. The implant remained in situ for 30 days in the first subject, while for the remaining 5 subjects the implants remained in situ for 90 days. On the day of explantation, the subjects were administered a 2 mg oral dose of risperidone, and starting one day post-explant returned to their original oral dosing regimen of 4 mg risperidone. The residual risperidone remaining in the implants after explantation was assessed, and based on the difference between the starting and residual risperidone contents in the implant, the average daily release rate of risperidone was

estimated to be about 1.45 mg/day, which correlated well to the 1.3-1.5 mg/day elution rate previously measured *in vitro* and the 1.5 mg/day measured *in vivo* in dogs over 28 weeks. A graph of the mean risperidone, 9-OH-risperidone and total active moiety plasma concentrations (i.e., risperidone + 9-OH-risperidone) (ng/mL) for the six subjects from Day 3 to Day 90, following implantation, is shown in Figure 39. A comparison of the graph in Figure 38 (mean risperidone active moiety plasma concentration over 24 hours following oral administration of 4 mg risperidone) and the active moiety graph in Figure 39 (mean active moiety plasma concentration from Day 3 to Day 90 following implantation) is shown in Figure 40.

As shown in the graphs of Figures 38, 39, and 40, the active moiety plasma concentrations for the implant ranged from about 12 ng/mL trough to about 20 ng/mL peak, for a difference of about 8 ng/mL from peak to trough. Thus, the active moiety plasma concentration was not less than about 12/20 = 60% of the peak active moiety plasma concentration while the implants were in situ. The peak to trough ratio following implantation was about 20/12 = 1.7. The active moiety plasma concentrations in the implant ranged from about 12 ng/mL trough to about 17 ng/mL peak over 60 days (from about Day 30 to about Day 90), for a difference of about 5 ng/mL from peak to trough over 60 days. Thus, the active moiety plasma concentration was within about 12/17 = 70% of the peak active moiety plasma concentration over about 30 days to about 60 days. The peak to trough ratio over about 30 days to about 60 days was about 17/12 = 1.4. On the contrary, active moiety plasma concentrations for the 4 mg oral dose ranged from about 20 ng/mL trough to about 50 ng/mL peak over 1 day, for a difference of about 30 ng/mL from peak to trough over 1 day. Thus, the trough active moiety plasma concentration was about 20/50 = 40% of the peak active moiety plasma concentration over 1 day. The peak to trough ratio over one day was about 50/20 = 2.5.

The peak active moiety plasma concentration for the implant over about 90 days (about 20 ng/mL) was approximately equivalent to the trough active moiety plasma concentration for the oral dose over 1 day (about 20 ng/mL). The peak active moiety plasma concentration for the implant over about 90 days (about 20 ng/mL) was about 40% of the peak active moiety plasma concentration for the oral dose over 1 day (50 ng/mL).

The difference in peak to trough plasma levels for the implant over about 30 to about 60 days (about 5 ng/mL) was about 6 times less than the difference in peak to trough plasma levels for the oral dose over 1 day (30 ng/mL). The difference in peak to trough plasma levels for the implant over about 90 days (about 8 ng/mL) was about 3.75 times less than the difference in peak to trough plasma levels for the oral dose over 1 day (30 ng/mL).

### Example 34: Anastrozole Implant *In Vivo*

The following general procedure was followed for a clinical study of an anastrozole implant in humans. A Phase I clinical study was conducted with open-label, two arm, fixed dose, repeated dose design in up to 12 post-menopausal women, in order to investigate safety and tolerability pharmacokinetics and pharmacodynamics of anastrozole following administration for 2-4 weeks of an oral dosage form of anastrozole (i.e., an oral tablet that contained 1 mg anastrozole, taken once daily), or following administration of an anastrozole subcutaneous implant for 6 weeks in accordance with embodiments of the present invention. Six subjects were enrolled in Cohort 1 and six subjects were enrolled in Cohort 2. The subjects were randomized to receive either an oral dosage form of anastrozole or an anastrozole subcutaneous implant first. The subjects then switched treatments after a 2 week washout. Dense pharmacokinetic and pharmacodynamic data were collected at scheduled times. Anastrozole and estrogen data were evaluated using NONMEM™ (NONlinear Mixed Effects Modeling™) Version 7 level 2.0, which is owned by ICON Development Solutions. Standard model building and evaluation techniques were used.

The anastrozole implant used in this study was manufactured by the following general procedure. Tubing was received in continuous length rolls and was cut to an appropriate starting length using a single-edged razor blade (or suitably sized scalpel). One end of each tubing section was thermally sealed by the following process: One tubing section was loaded into an appropriately-adjusted holding fixture of a sealing machine and a sealing cycle was initiated as described above in Example 1, which imparted a semi-spherical closure on the tip of the tubing section. TECOFLEX® EG-93A polyurethane was used as the polymer excipient.

A discrete solid dosage form was prepared by premixing anastrozole (88 wt%), croscarmellose sodium (10 wt%), and stearic acid (2 wt%) in a Turbula blender. The drug blend was compacted using a single punch tablet press. Drug pellets were manually placed inside each sealed section of tubing. The open section of each pellet-containing tubing section was then sealed into a semi-spherical seal. Sterilization was accomplished by gamma irradiation of the implants. A total of about 375 mg anastrozole were loaded into each implant with 10% croscarmellose and 2% stearic acid. Each implant had a length of about 50 mm, an outer diameter of about 4.0 mm, an inner diameter of about 3.6 mm, and a wall thickness of about 0.2 mm.

The pharmacokinetics of anastrozole were well-described by a one compartment linear model with first order elimination. Based on PKPD modeling, the amount of time from administration to steady-state was approximately 2 weeks (see Figure 61, which shows the mean anastrozole plasma concentrations (pg/mL) over time). The effect of anastrozole on estrogen formation was well-described with an indirect effect model with sigmoidal inhibitory drug effect.

The concentration response curve for anastrozole on estrogen was shallow (Hill coefficient 0.455) with a low value for the concentration achieving half-maximal response (Anastrozole EC50 0.565 ng/L) (see Figure 62, which shows the mean estradiol plasma concentrations (pg/mL) over time). The term half-maximal effective concentration (EC50) refers to the concentration of a drug that produces 50% of the maximum response after a specified period of time. Owing to low **[0267]** EC50, estrogen reduction following administration of oral anastrozole and the anastrozole subcutaneous implant were comparable, despite lower concentrations achieved with the anastrozole subcutaneous implant. These results indicate that the anastrozole subcutaneous implant was effective in suppressing serum estradiol concentrations in human patients. Based on PKPD results, the anastrozole subcutaneous implant is expected to sustain sufficient anastrozole levels with resulting suppression of estrogen for at least 1 year, as approximately 6.8% of the anastrozole present in the anastrozole subcutaneous implant was absorbed after 6 weeks in situ (as determined based on the blood levels of anastrozole and the amount of anastrozole left in the implant once it was removed).

## Claims

**1.** A reservoir-based drug delivery composition for use in treating an estrogen-related disorder by implantation into a subject, wherein the drug delivery composition systemically delivers a therapeutically effective amount of an aromatase inhibitor to the subject for a period of time of at least one month, preferably 3 months.

**2.** A drug delivery composition for use according to claim 1, wherein the drug delivery composition comprises at least one discrete solid dosage form, preferably a substantially spherical dosage form, comprising at least one aromatase inhibitor surrounded by an excipient comprising at least one polymer, preferably a thermoplastic elastomer or an elastomeric polymer, preferably defining a reservoir which is preferably cylindrically shaped.

**3.** A drug delivery composition for use according to claim 1 or 2, wherein the therapeutically effective amount of the at least one aromatase inhibitor is delivered at a pseudo-zero order rate, preferably subcutaneously.

**4.** A drug delivery composition for use according to any of claims 1 to 3, wherein the thermoplastic elastomer comprises polyurethane-based polymers, polyether-based polymers, polysilicone-based polymers, polycarbonate-based polymers, or combinations thereof.

**5.** A drug delivery composition for use according to any of claims 1 to 4, wherein the polymer comprises a polyether-based polyurethane, preferably an aliphatic polyether-based polyurethane comprising poly(tetramethylene oxide) and polymerized 4,4'-diisocyanato dicyclohexylmethane (H12MDI) and 1,4-butanediol.

**6.** A drug delivery composition for use according to any of claims 1 to 5, wherein the excipient comprising at least one polymer forms a wall having an average thickness of about 0.05 mm to about 0.5 mm, wherein the excipient preferably is not substantially erodible and not substantially degradable *in vivo.*

**7.** A drug delivery composition for use according to any of claims 1 to 6, wherein the drug delivery composition does not require erosion or degradation of the excipient *in vivo* to release the aromatase inhibitor in the therapeutically effective amount.

**8.** A drug delivery composition for use according to any of claims 1 to 7, wherein the estrogen-related disorder is breast cancer, 9endometriosis, uterine fibroids, or short stature in a child or adolescent.

**9.** A drug delivery composition for use according to any of claims 1 to 8, wherein the therapeutically effective amount of the aromatase inhibitor is delivered to the subject at a target range of about 100 to about 10,000 micrograms/day.

**10.** A drug delivery composition for use according to any of claims 1 to 9, wherein the at least one aromatase inhibitor has a non-steroidal chemical structure, preferably is selected from the group consisting of anastrozole, letrozole, and combinations and pharmaceutically acceptable salts thereof.

**11.** A drug delivery composition for use according to any of claims 1 to 10 wherein the at least one discrete solid dosage form comprises:

75-97 wt% anastrozole or a pharmaceutically acceptable salt thereof based on the total weight of the at least one discrete solid dosage form;

1-25 wt%, preferably 2-12 wt%, of at least one sorption enhancer based on the total weight of the at least one discrete solid dosage form; and
0-5 wt% lubricant based on the total weight of the at least one discrete solid dosage form.

**12.** A drug delivery composition for use according to any of claims 1 to 11, wherein the at least one sorption enhancer is selected from the group consisting of croscarmellose sodium, sodium carboxymethyl starch, sodium starch glycolate, sodium acrylic acid derivatives, chondroitin sulfate, poly-glutamic acid, poly-aspartic acid, and combinations thereof

**13.** A drug delivery composition for use according to any of claims 1 to 12, wherein the elastomeric polymer is preferably substantially non-porous, and wherein the elastomeric polymer preferably comprises soft segments derived from polyethers, preferably from alkylene oxide polymers selected from the group consisting of poly(tetramethylene oxide) (PTMO), polyethylene glycol (PEG), poly(propylene oxide) (PPO), poly(hexamethylene oxide), and combinations thereof, polycarbonates, or polysilicones.

**14.** A drug delivery composition for use according to any of claims 1 to 12 in form of a kit, comprising an implanter for inserting the reservoir-based drug delivery composition beneath the skin

**15.** A drug delivery composition comprising:

a drug elution rate-controlling excipient comprising an elastomeric polymer, defining a reservoir, and
the reservoir contains at least one discrete solid dosage form, comprising at least one aromatase inhibitor, preferably as claimed in any of the claims 1 to 14.

**Patentansprüche**

**1.** Reservoir-basierte Arzneimittelabgabezusammensetzung zur Verwendung bei der Behandlung einer östrogenbezogenen Störung durch Implantation in ein Subjekt, wobei die Arzneimittelabgabezusammensetzung eine therapeutisch wirksame Menge eines Aromataseinhibitors über einen Zeitraum von wenigstens einem Monat, vorzugsweise 3 Monaten, systemisch an das Subjekt abgibt.

**2.** Arzneimittelabgabezusammensetzung zur Verwendung nach Anspruch 1, wobei die Arzneimittelabgabezusammensetzung wenigstens eine diskrete feste Dosierungsform, vorzugsweise eine im Wesentlichen sphärische Dosierungsform, beinhaltet, die wenigstens einen Aromataseinhibitor beinhaltet, der von einem Hilfsstoff umgeben ist, der wenigstens ein Polymer, vorzugsweise ein thermoplastisches Elastomer oder ein elastomeres Polymer, beinhaltet, und der vorzugsweise ein Reservoir definiert, das vorzugsweise zylindrisch geformt ist.

**3.** Arzneimittelabgabezusammensetzung zur Verwendung nach Anspruch 1 oder 2, wobei die therapeutisch wirksame Menge des wenigstens einen Aromataseinhibitors in einer Rate pseudo-nullter Ordnung, vorzugsweise subkutan, abgegeben wird.

**4.** Arzneimittelabgabezusammensetzung zur Verwendung nach einem der Ansprüche 1 bis 3, wobei das thermoplastische Elastomer Polyurethan-basierte Polymere, Polyetherbasierte Polymere, Polysilikon-basierte Polymere, Polycarbonat-basierte Polymere oder Kombinationen davon beinhaltet.

**5.** Arzneimittelabgabezusammensetzung zur Verwendung nach einem der Ansprüche 1 bis 4, wobei das Polymer ein Polyether-basiertes Polyurethan, vorzugsweise ein aliphatisches Polyether-basiertes Polyurethan, beinhaltet, das Poly(tetramethylenoxid) und polymerisiertes 4,4'-Diisocyanatodicyclohexylmethan (H12MDI) und 1,4-Butandiol beinhaltet.

**6.** Arzneimittelabgabezusammensetzung zur Verwendung nach einem der Ansprüche 1 bis 5, wobei der Hilfsstoff, der wenigstens ein Polymer beinhaltet, eine Wand mit einer durchschnittlichen Dicke von etwa 0,05 mm bis etwa 0,5 mm bildet, wobei der Hilfsstoff vorzugsweise *in vivo* nicht wesentlich erodierbar und nicht wesentlich abbaubar ist.

**7.** Arzneimittelabgabezusammensetzung zur Verwendung nach einem der Ansprüche 1 bis 6, wobei die Arzneimittelabgabezusammensetzung keine(n) Erosion oder Abbau des Hilfsstoffs *in vivo* erfordert, um den Aromataseinhibitor in der therapeutisch wirksamen Menge freizusetzen.

**8.** Arzneimittelabgabezusammensetzung zur Verwendung nach einem der Ansprüche 1 bis 7, wobei die östrogenbezogene Störung Brustkrebs, Endometriose, Uterusmyome oder Kleinwüchsigkeit bei einem Kind oder Jugendlichen ist.

**9.** Arzneimittelabgabezusammensetzung zur Verwendung nach einem der Ansprüche 1 bis 8, wobei die therapeutisch wirksame Menge des Aromataseinhibitors in einem Zielbereich von etwa 100 bis etwa 10.000 Mikrogramm/Tag an das Subjekt abgegeben wird.

**10.** Arzneimittelabgabezusammensetzung zur Verwendung nach einem der Ansprüche 1 bis 9, wobei der wenigstens eine Aromataseinhibitor eine nichtsteroidale chemische Struktur hat und vorzugsweise aus der Gruppe bestehend aus Anastrozol, Letrozol und Kombinationen und pharmazeutisch akzeptablen Salzen davon ausgewählt ist.

**11.** Arzneimittelabgabezusammensetzung zur Verwendung nach einem der Ansprüche 1 bis 10, wobei die wenigstens eine diskrete feste Dosierungsform Folgendes beinhaltet:

75-97 Gew.-% von Anastrozol oder einem pharmazeutisch akzeptablen Salz davon bezogen auf das Gesamtgewicht der wenigstens einen diskreten festen Dosierungsform;
1-25 Gew.-%, vorzugsweise 2-12 Gew.-%, von wenigstens einem Sorptionsverbesserer bezogen auf das Gesamtgewicht der wenigstens einen diskreten festen Dosierungsform; und
0-5 Gew.-% Schmiermittel bezogen auf das Gesamtgewicht der wenigstens einen diskreten festen Dosierungsform.

**12.** Arzneimittelabgabezusammensetzung zur Verwendung nach einem der Ansprüche 1 bis 11, wobei der wenigstens eine Sorptionsverbesserer ausgewählt ist aus der Gruppe bestehend aus Croscarmellose-Natrium, Natriumcarboxymethylstärke, Natriumstärkeglykolat, Natriumacrylsäurederivaten, Chondroitinsulfat, Polyglutaminsäure, Polyasparaginsäure und Kombinationen davon.

**13.** Arzneimittelabgabezusammensetzung zur Verwendung nach einem der Ansprüche 1 bis 12, wobei das elastomere Polymer vorzugsweise im Wesentlichen nicht porös ist und wobei das elastomere Polymer vorzugsweise weiche Segmente beinhaltet, die von Polyethern, vorzugsweise von Alkylenoxidpolymeren, abgeleitet sind, die ausgewählt sind aus der Gruppe bestehend aus Poly(tetramethylenoxid) (PTMO), Polyethylenglykol (PEG), Poly(propylenoxid) (PPO), Poly(hexamethylenoxid) und Kombinationen davon, Polycarbonaten oder Polysilikonen.

**14.** Arzneimittelabgabezusammensetzung zur Verwendung nach einem der Ansprüche 1 bis 12 in Form eines Kits, der eine Implantationsvorrichtung zum Einfügen der Reservoirbasierten Arzneimittelabgabezusammensetzung unter die Haut beinhaltet.

**15.** Arzneimittelabgabezusammensetzung, die Folgendes beinhaltet:

einen Hilfsstoff zur Regulierung der Arzneimittelelutionsrate, der ein elastomeres Polymer beinhaltet und ein Reservoir definiert, und
das Reservoir enthält wenigstens eine diskrete feste Dosierungsform, die wenigstens einen Aromataseinhibitor beinhaltet, vorzugsweise wie in einem der Ansprüche 1 bis 14 beansprucht.

**Revendications**

**1.** Composition pour la délivrance d'un médicament basée sur un réservoir pour une utilisation dans le traitement de maladies liées aux oestrogènes par implantation chez un sujet, où la composition pour la délivrance d'un médicament libère systémiquement une quantité thérapeutiquement efficace d'un inhibiteur de l'aromatase chez le sujet pendant une période d'au moins un mois, préférablement de 3 mois.

**2.** Composition pour la délivrance d'un médicament pour une utilisation selon la revendication 1, où la composition pour la délivrance d'un médicament comprend au moins une forme pharmaceutique solide discrète, préférablement une forme pharmaceutique essentiellement sphérique, comprenant au moins un inhibiteur de l'aromatase entouré par un excipient comprenant au moins un polymère, préférablement un élastomère thermoplastique ou un polymère élastomère, définissant préférablement un réservoir dont la forme est préférablement cylindrique.

**3.** Composition pour la délivrance d'un médicament pour une utilisation selon la revendication 1 ou 2, où la quantité thérapeutiquement efficace du au moins un inhibiteur de l'aromatase est libérée à une vitesse de pseudo ordre zéro, préférablement par voie sous-cutanée.

**4.** Composition pour la délivrance d'un médicament pour une utilisation selon l'une quelconque des revendications 1 à 3, où l'élastomère thermoplastique comprend des polymères à base de polyuréthane, des polymères à base de polyéther, des polymères à base de polysilicone, des polymères à base de polycarbonate ou des combinaisons de ceux-ci.

**5.** Composition pour la délivrance d'un médicament pour une utilisation selon l'une quelconque des revendications 1 à 4, où le polymère comprend un polyuréthane à base de polyéther, préférablement un polyuréthane à base de polyéther aliphatique comprenant un poly(oxyde de tétraméthylène) et le 4,4'-diisocyanatodicyclohexylméthane (H12MDI) et le 1,4-butanediol polymérisés.

**6.** Composition pour la délivrance d'un médicament pour une utilisation selon l'une quelconque des revendications 1 à 5, où l'excipient comprend au moins un polymère qui forme une cloison dont l'épaisseur moyenne va d'environ 0,05 mm à environ 0,5 mm, préférablement où l'excipient n'est essentiellement pas érodable et n'est essentiellement pas dégradable *in vivo.*

**7.** Composition pour la délivrance d'un médicament pour une utilisation selon l'une quelconque des revendications 1 à 6, où la composition pour la délivrance d'un médicament ne requiert pas une érosion ou une dégradation de l'excipient *in vivo* pour libérer l'inhibiteur de l'aromatase à la quantité thérapeutiquement efficace.

**8.** Composition pour la délivrance d'un médicament pour une utilisation selon l'une quelconque des revendications 1 à 7, où la maladie liée aux oestrogènes est le cancer du sein, l'endométriose, le fibrome utérin ou l'insuffisance staturale chez un enfant ou un adolescent.

**9.** Composition pour la délivrance d'un médicament pour une utilisation selon l'une quelconque des revendications 1 à 8, où la quantité thérapeutiquement efficace de l'inhibiteur de l'aromatase est libérée chez le sujet dans une plage cible qui va d'environ 100 à environ 10 000 microgrammes/jour.

**10.** Composition pour la délivrance d'un médicament pour une utilisation selon l'une quelconque des revendications 1 à 9, où le au moins un inhibiteur de l'aromatase a une structure chimique non stéroïdienne et est préférablement sélectionné dans le groupe consistant en l'anastrozole, le létrozole et des combinaisons et sels pharmaceutiquement acceptables de ceux-ci.

**11.** Composition pour la délivrance d'un médicament pour une utilisation selon l'une quelconque des revendications 1 à 10, où la au moins une forme pharmaceutique solide discrète comprend :

75-97 % en poids d'anastrozole ou d'un sel pharmaceutiquement acceptable de celui-ci rapportés au poids total de la au moins une forme pharmaceutique solide discrète ;
1-25 % en poids, préférablement 2-12 % en poids, d'au moins un activateur de sorption rapportés au poids total de la au moins une forme pharmaceutique solide discrète ; et
0-5 % en poids d'un lubrifiant rapportés au poids total de la au moins une forme pharmaceutique solide discrète.

**12.** Composition pour la délivrance d'un médicament pour une utilisation selon l'une quelconque des revendications 1 à 11, où le au moins un activateur de sorption est sélectionné dans le groupe consistant en la croscarmellose sodique, le carboxyméthylamidon sodique, le glycolate d'amidon sodique, des dérivés sodiques de l'acide acrylique, le sulfate de chondroïtine, l'acide poly(glutamique), l'acide poly(aspartique) et des combinaisons de ceux-ci.

**13.** Composition pour la délivrance d'un médicament pour une utilisation selon l'une quelconque des revendications 1 à 12, où le polymère élastomère est préférablement essentiellement non poreux et où le polymère élastomère comprend préférablement des segments souples dérivés de polyéthers, préférablement des polymères d'oxydes d'alkylène sélectionnés dans le groupe consistant en le poly(oxyde de tétraméthylène) (PTMO), le polyéthylène glycol (PEG), le poly(oxyde de polypropylène) (PPO), le poly(oxyde d'hexaméthylène) et des combinaisons de ceux-ci, des polycarbonates ou des polysilicones.

**14.** Composition pour la délivrance d'un médicament pour une utilisation selon l'une quelconque des revendications 1

à 12 sous forme d'un kit qui comprend un dispositif d'implantation pour l'insertion sous la peau de la composition pour la délivrance d'un médicament basée sur un réservoir.

**15.** Composition pour la délivrance d'un médicament comprenant :

un excipient contrôlant la vitesse d'élution du médicament comprenant un polymère élastomère qui définit un réservoir, et
le réservoir contient au moins une forme pharmaceutique solide discrète comprenant au moins un inhibiteur de l'aromatase, préférablement telle que définie à l'une quelconque des revendications 1 à 14.

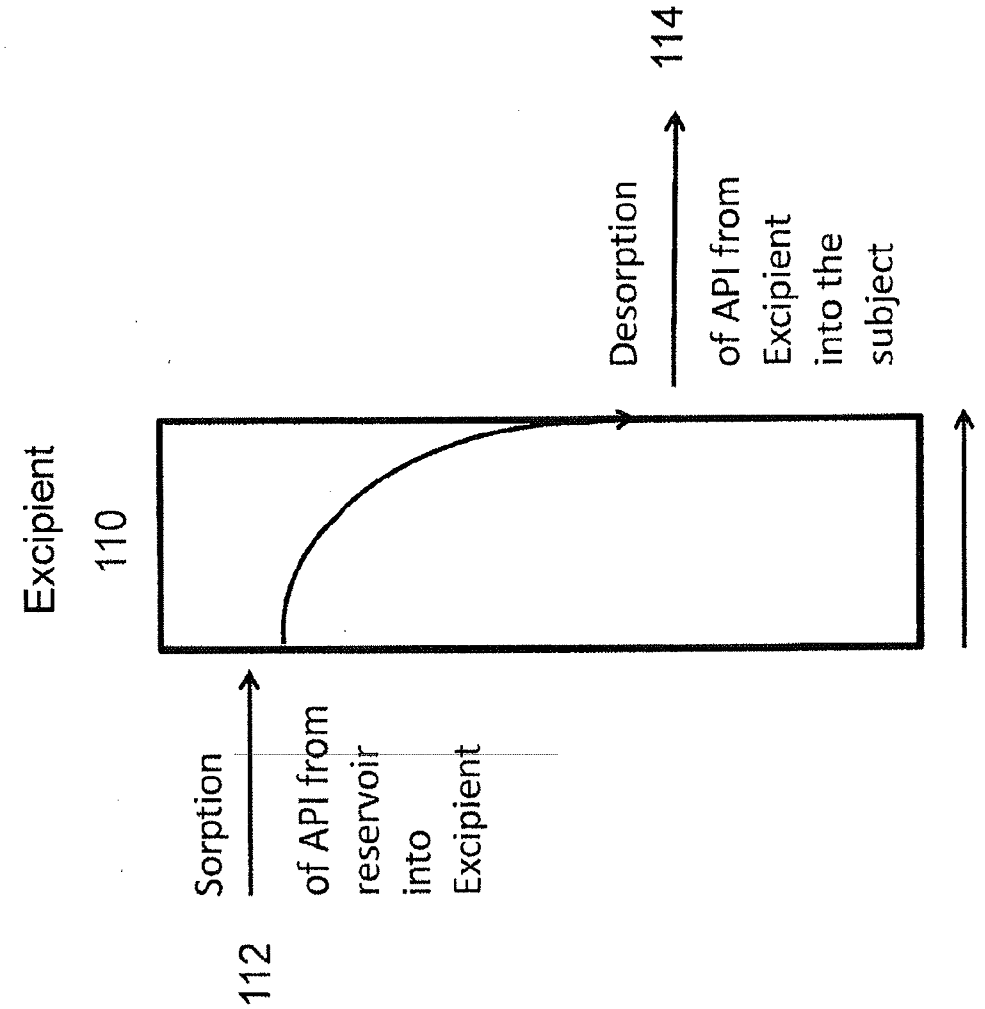
Excipient
110
112
Sorption
of API from
reservoir
into
Excipient
Desorption
of API from
Excipient
into the
subject
114

Figure 1

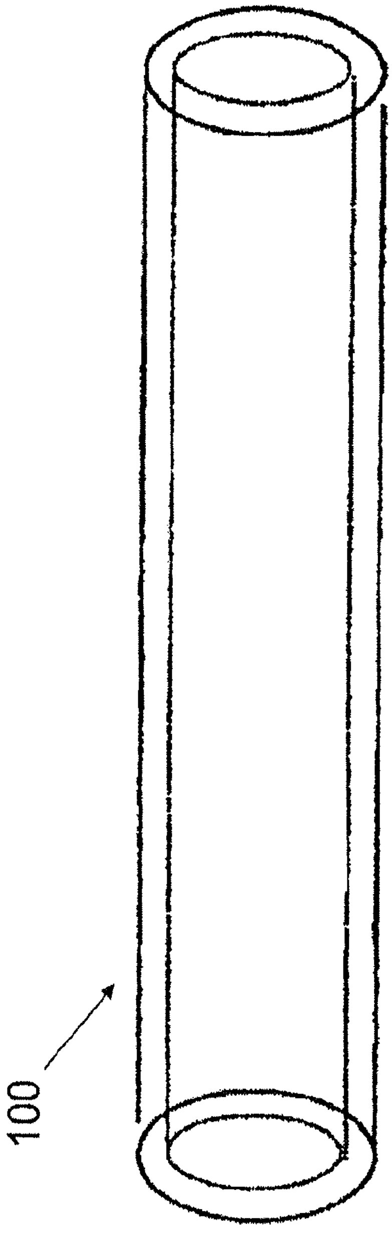


Figure 2

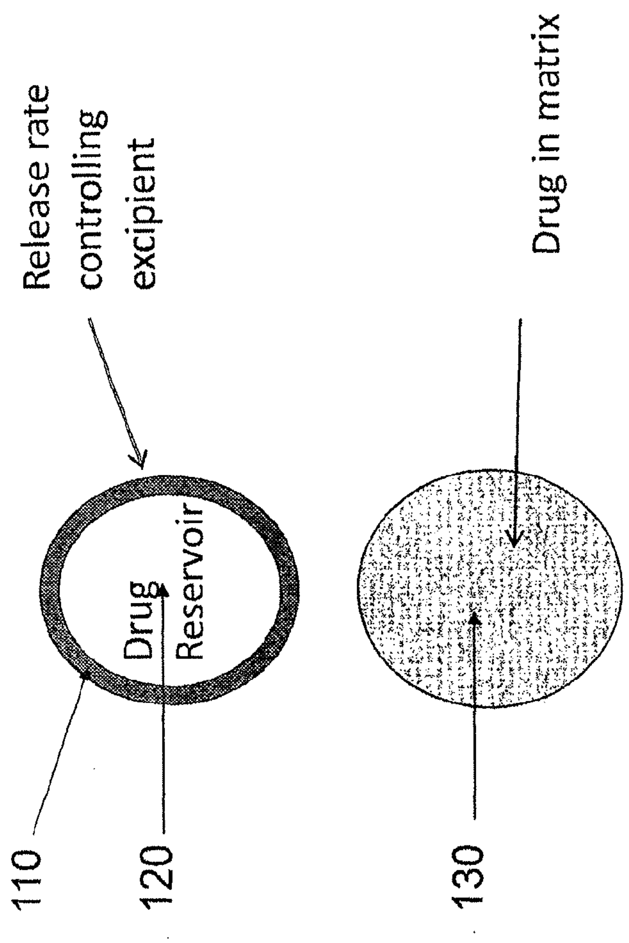
Release rate controlling excipient
Drug Reservoir
Drug in matrix
110
120
130

Figure 3

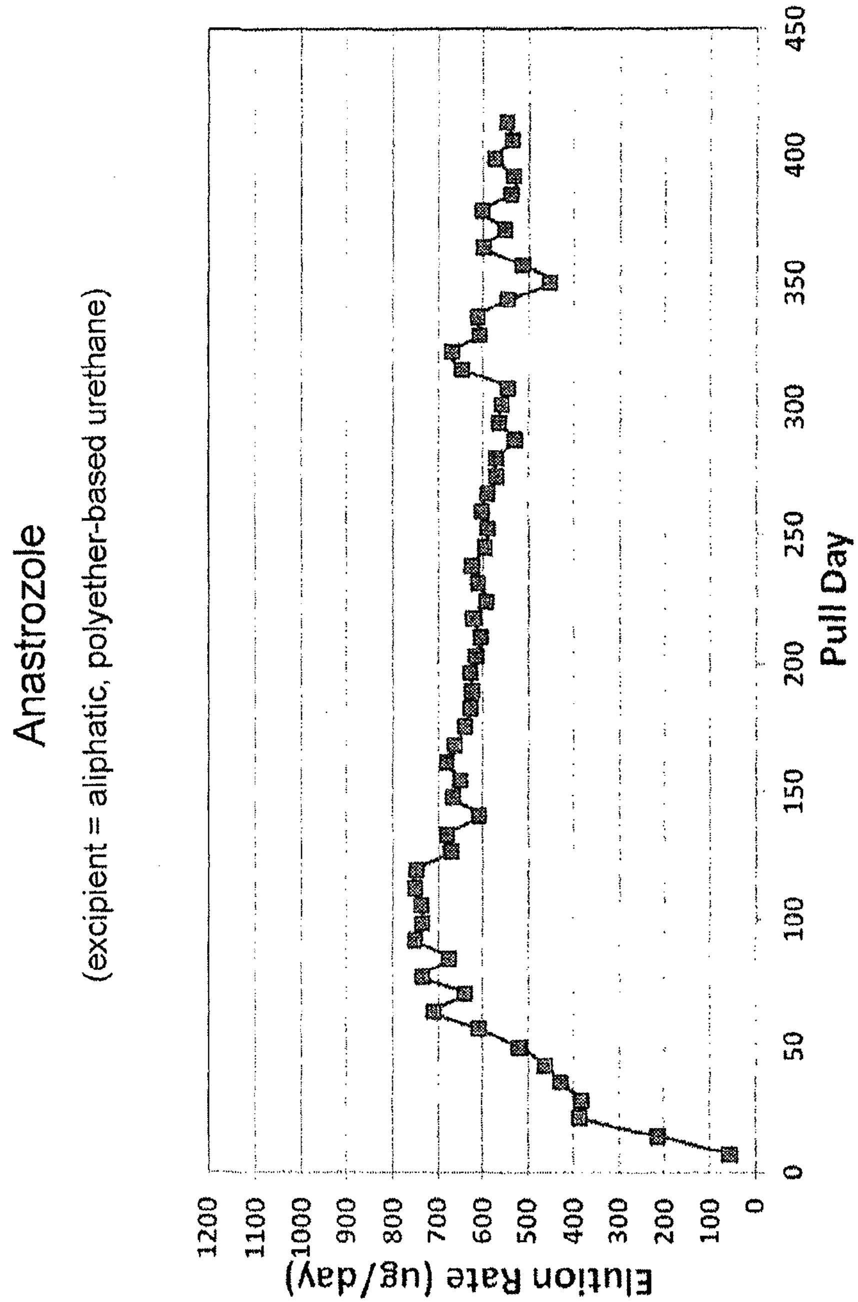
Anastrozole
(excipient = aliphatic, polyether-based urethane)
Elution Rate (ug/day)
1200
1100
1000
900
800
700
600
500
400
300
200
100
0
Pull Day
0
50
100
150
200
250
300
350
400
450

Figure 4

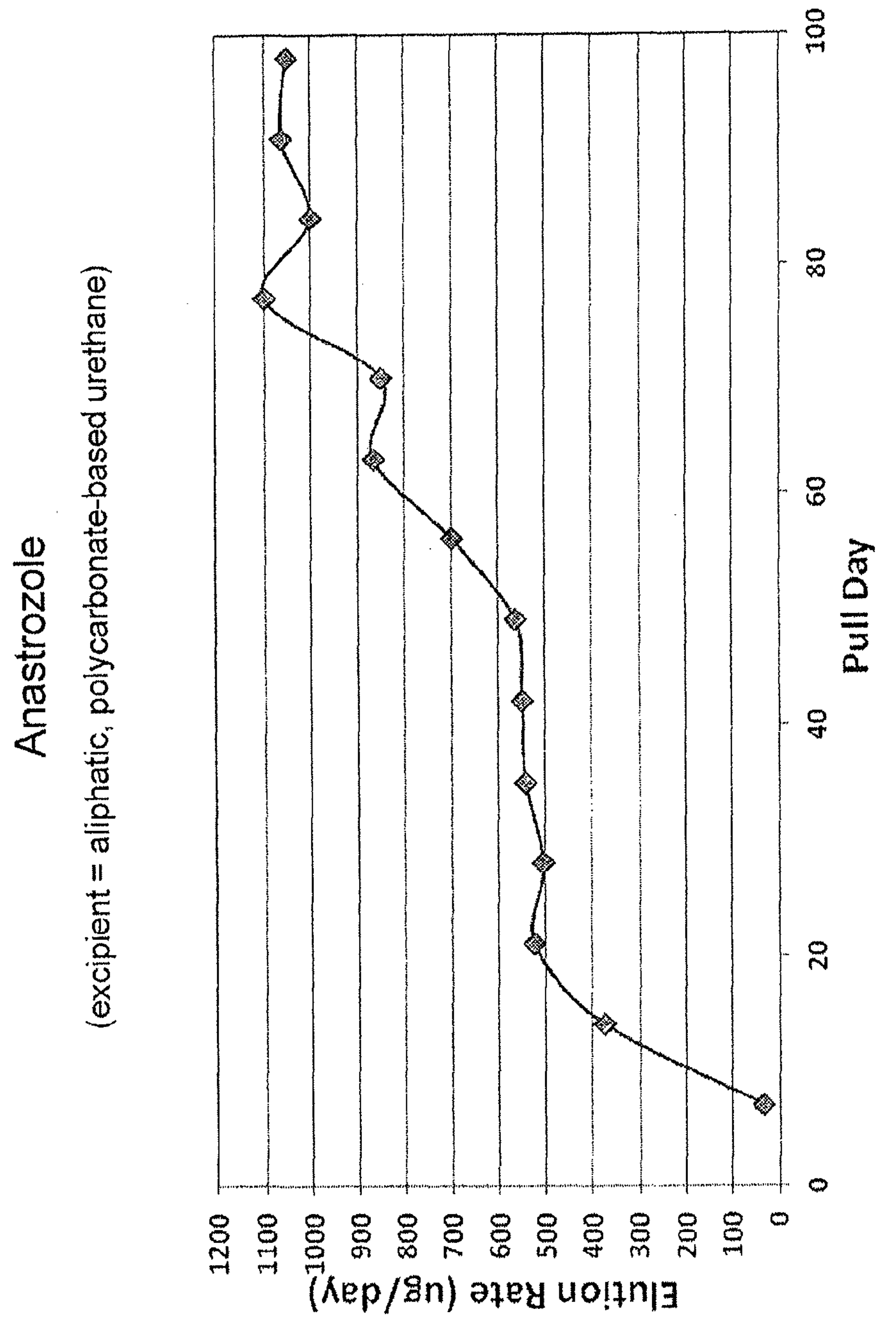
Anastrozole
(excipient = aliphatic, polycarbonate-based urethane)
Elution Rate (ug/day)
1200
1100
1000
900
800
700
600
500
400
300
200
100
0
0
20
40
60
80
100
Pull Day

Figure 5

Plasma Concentration in Beagle Dogs (oral dose and implant)

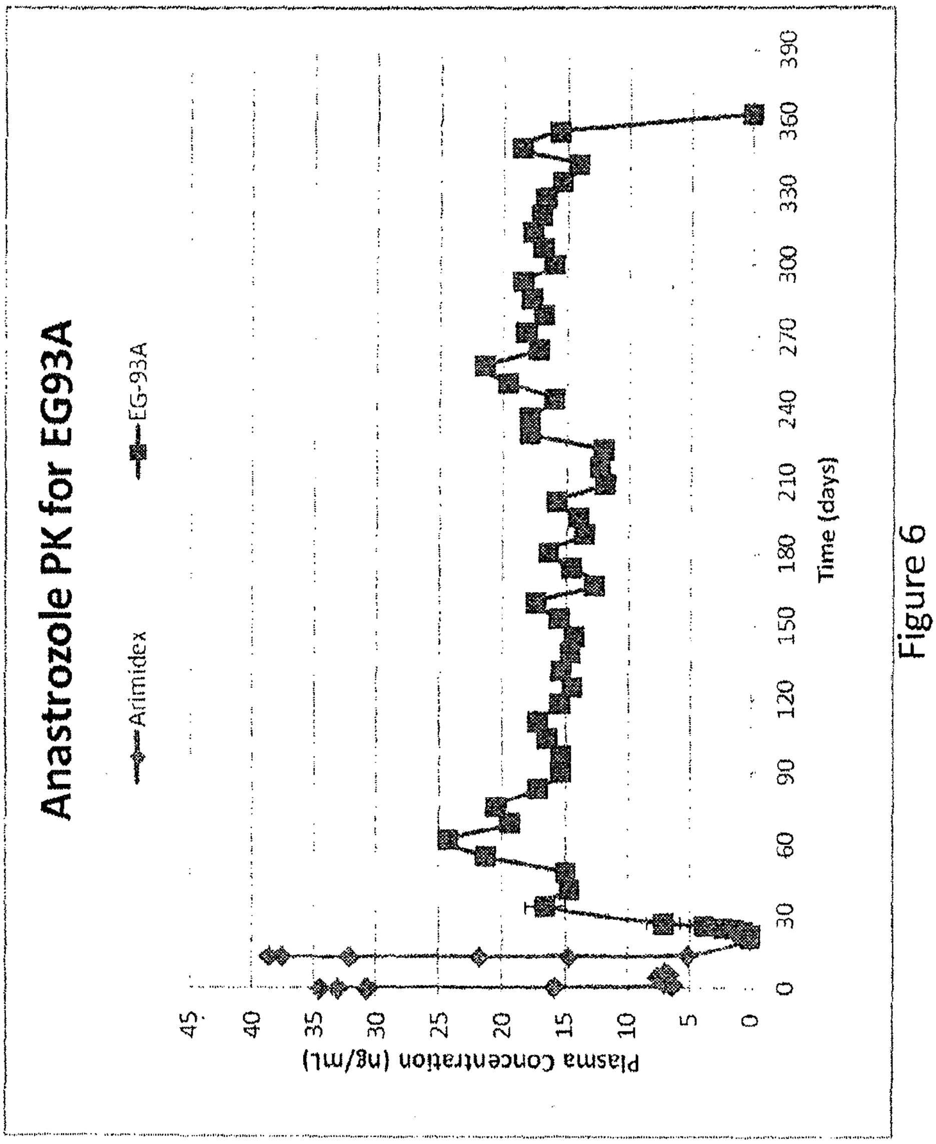


Figure 6

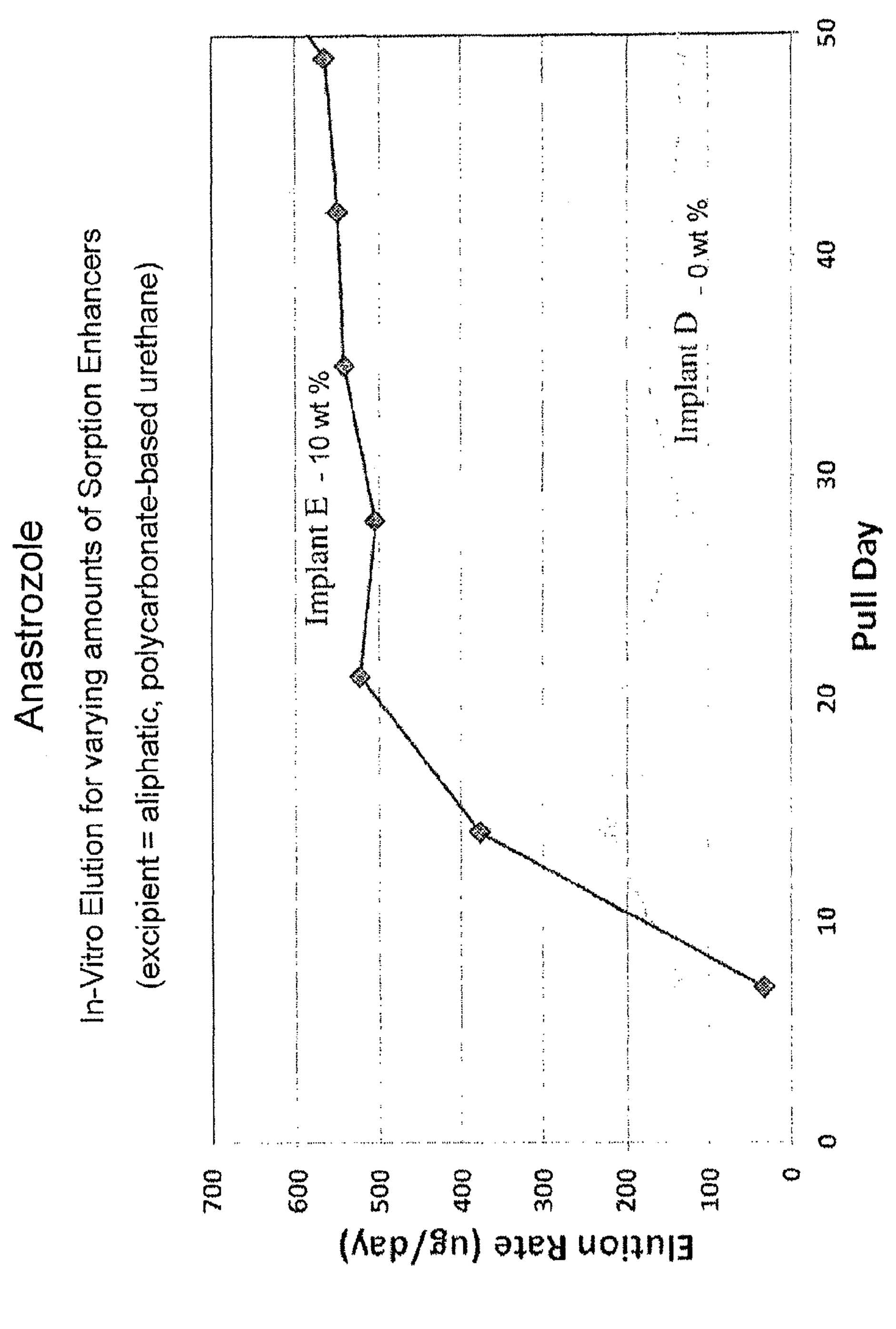
Anastrozole
In-Vitro Elution for varying amounts of Sorption Enhancers
(excipient = aliphatic, polycarbonate-based urethane)
Implant E - 10 wt %
Implant D - 0 wt %
Elution Rate (ug/day)
700
600
500
400
300
200
100
0
0
10
20
30
40
50
Pull Day

Figure 7

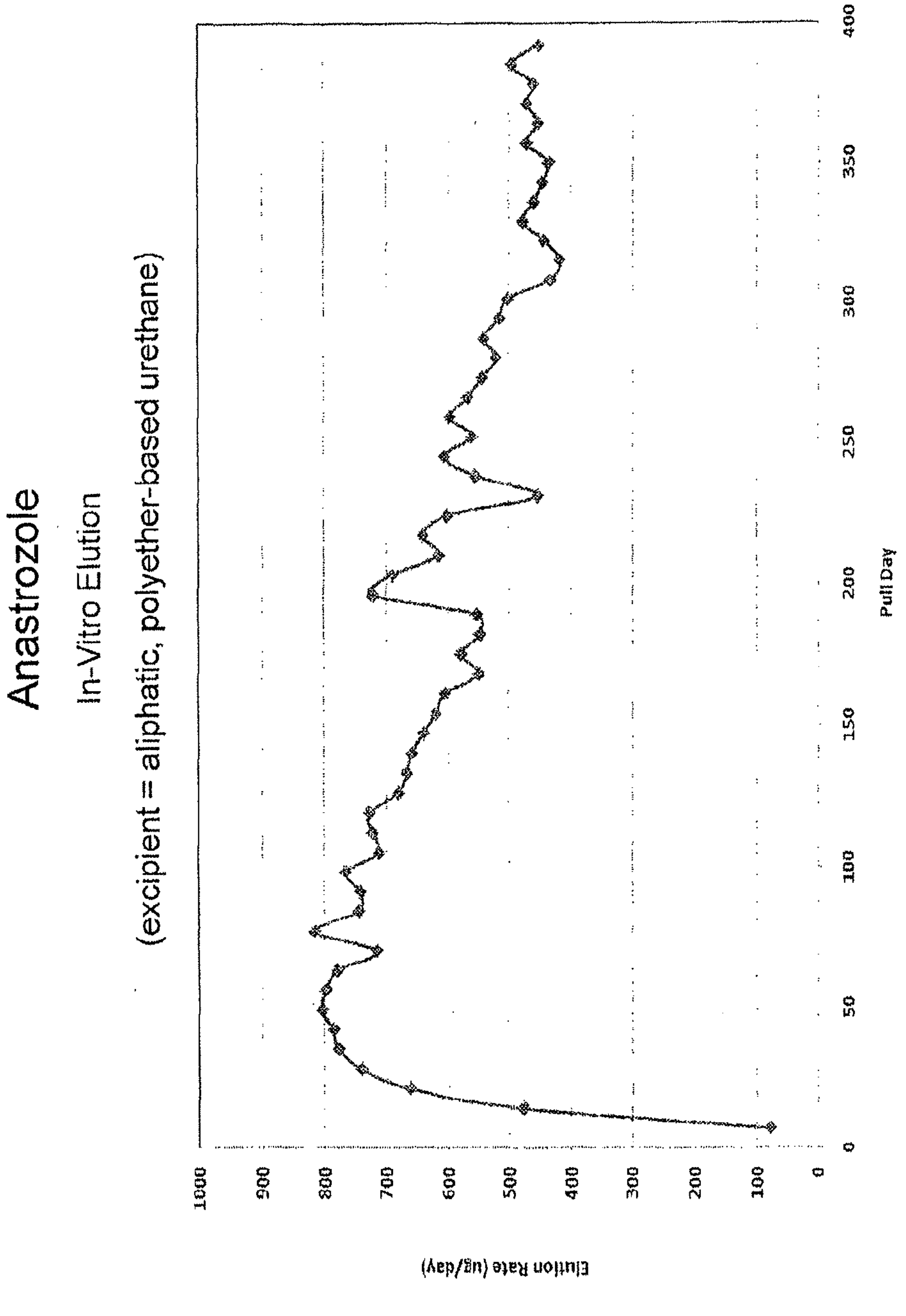
Anastrozole
In-Vitro Elution
(excipient = aliphatic, polyether-based urethane)
Elution Rate (ug/day)
1000
900
800
700
600
500
400
300
200
100
0
0
50
100
150
200
250
300
350
400
Pull Day

Figure 8

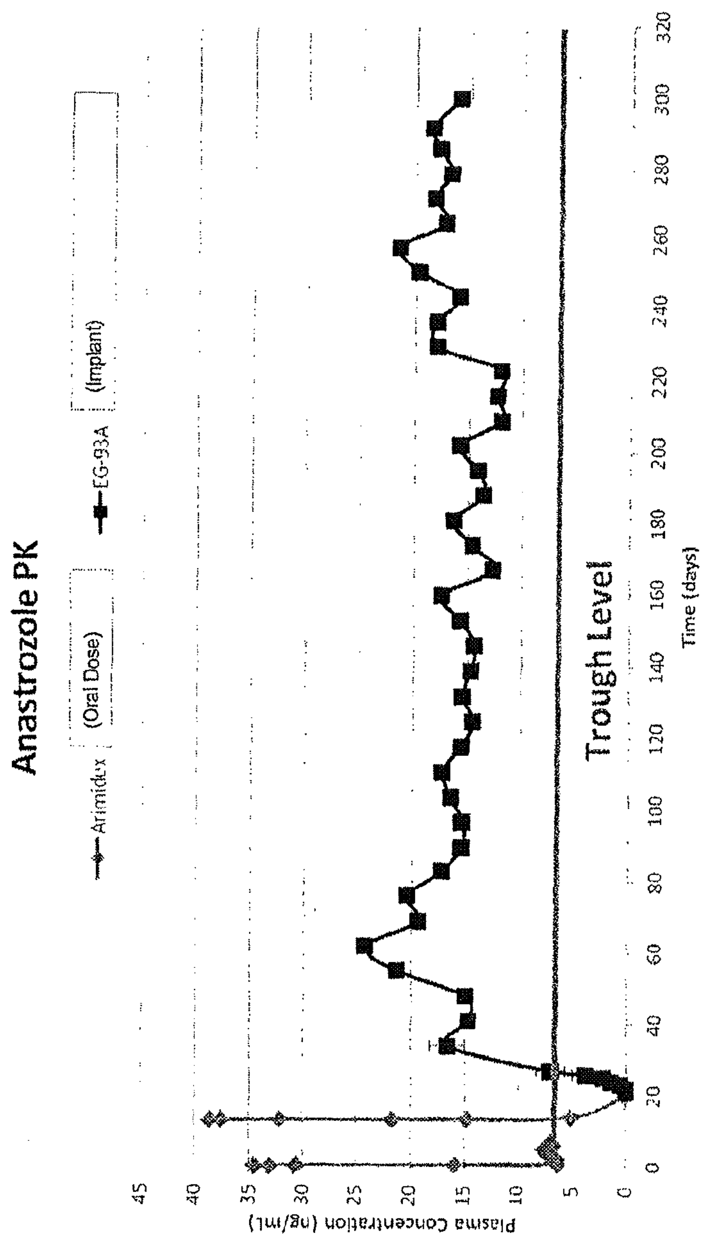
Anastrozole PK
Arimidex (Oral Dose)
EG-93A (Implant)
Trough Level
Plasma Concentration (ng/mL)
Time (days)
0
5
10
15
20
25
30
35
40
45
0
20
40
60
80
100
120
140
160
180
200
220
240
260
280
300
320

Figure 9

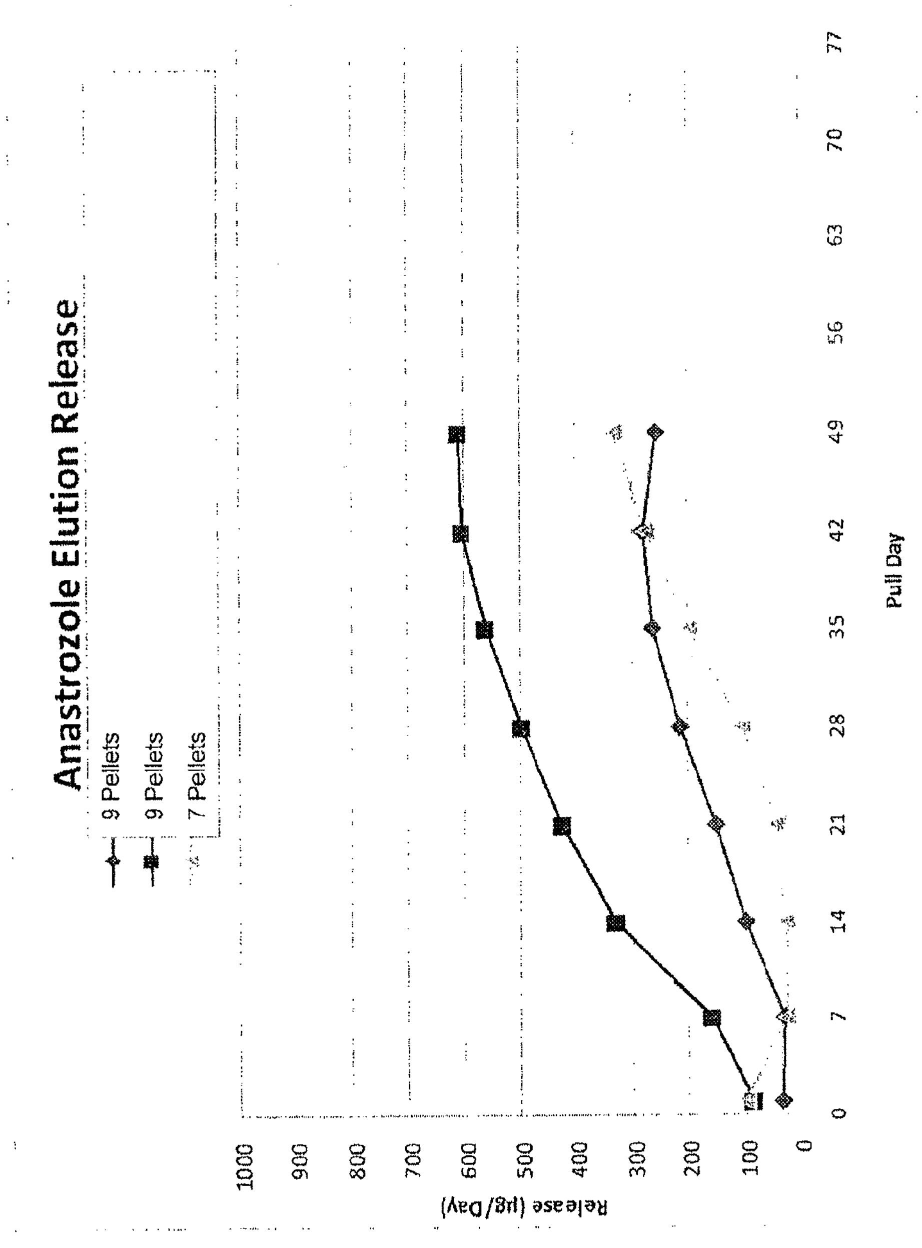

Anastrozole Elution Release
9 Pellets
9 Pellets
7 Pellets
Release (µg/Day)
1000
900
800
700
600
500
400
300
200
100
0
0
7
14
21
28
35
42
49
56
63
70
77
Pull Day


Figure 10

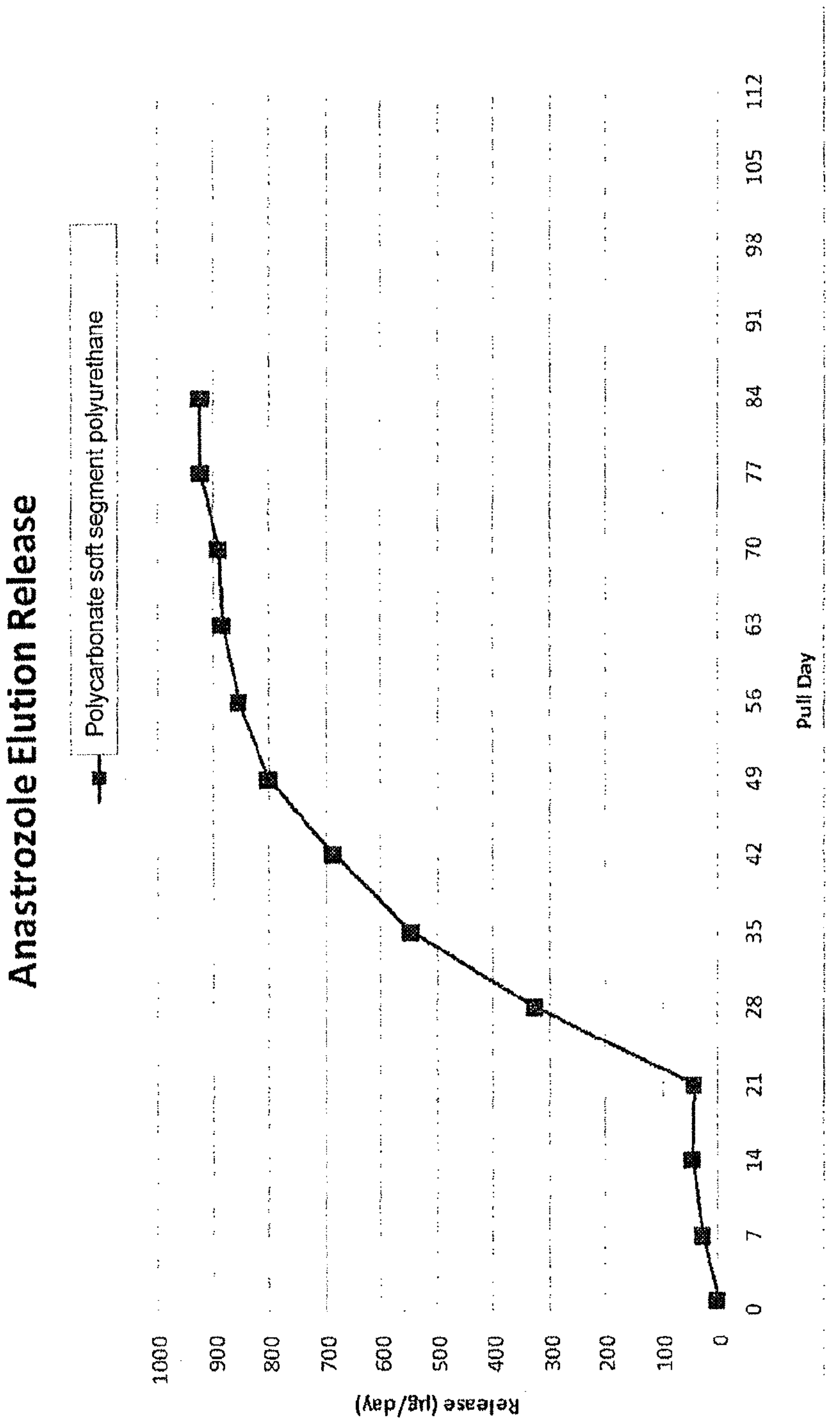
Anastrozole Elution Release
Polycarbonate soft segment polyurethane
Release (µg/day)
1000
900
800
700
600
500
400
300
200
100
0
0
7
14
21
28
35
42
49
56
63
70
77
84
91
98
105
112
Pull Day

Figure 11

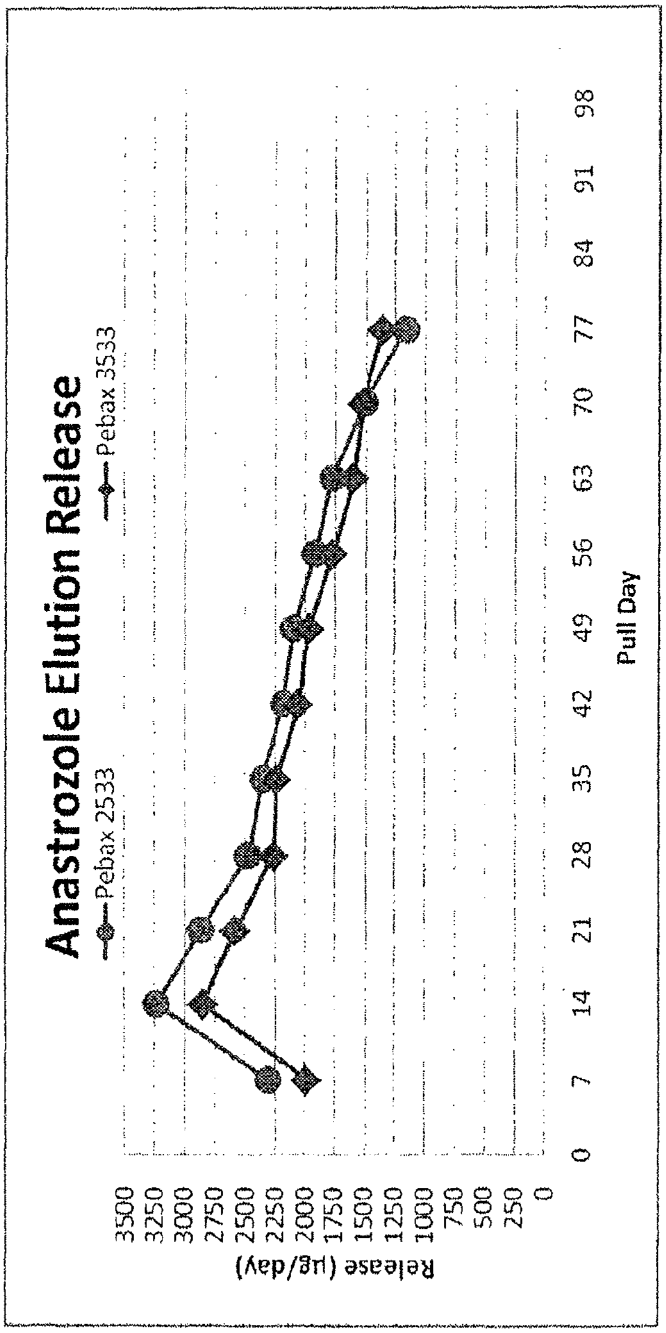
Anastrozole Elution Release
Pebax 2533
Pebax 3533
Release (µg/day)
3500
3250
3000
2750
2500
2250
2000
1750
1500
1250
1000
750
500
250
0
0
7
14
21
28
35
42
49
56
63
70
77
84
91
98
Pull Day

Figure 12

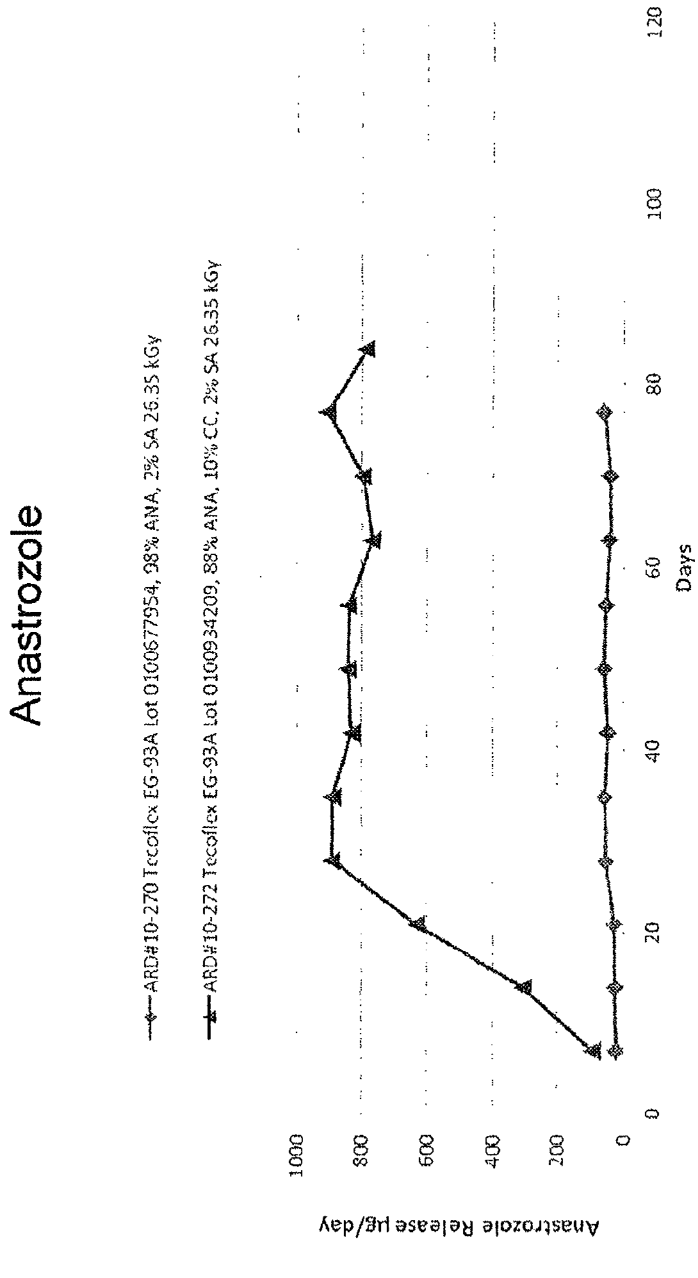
Anastrozole
ARD#10-270 Tecoflex EG-93A Lot 0100677954, 98% ANA, 2% SA 26.35 kGy
ARD#10-272 Tecoflex EG-93A Lot 0100934209, 88% ANA, 10% CC, 2% SA 26.35 kGy
Anastrozole Release µg/day
1000
800
600
400
200
0
0
20
40
60
80
100
120
Days

Figure 13

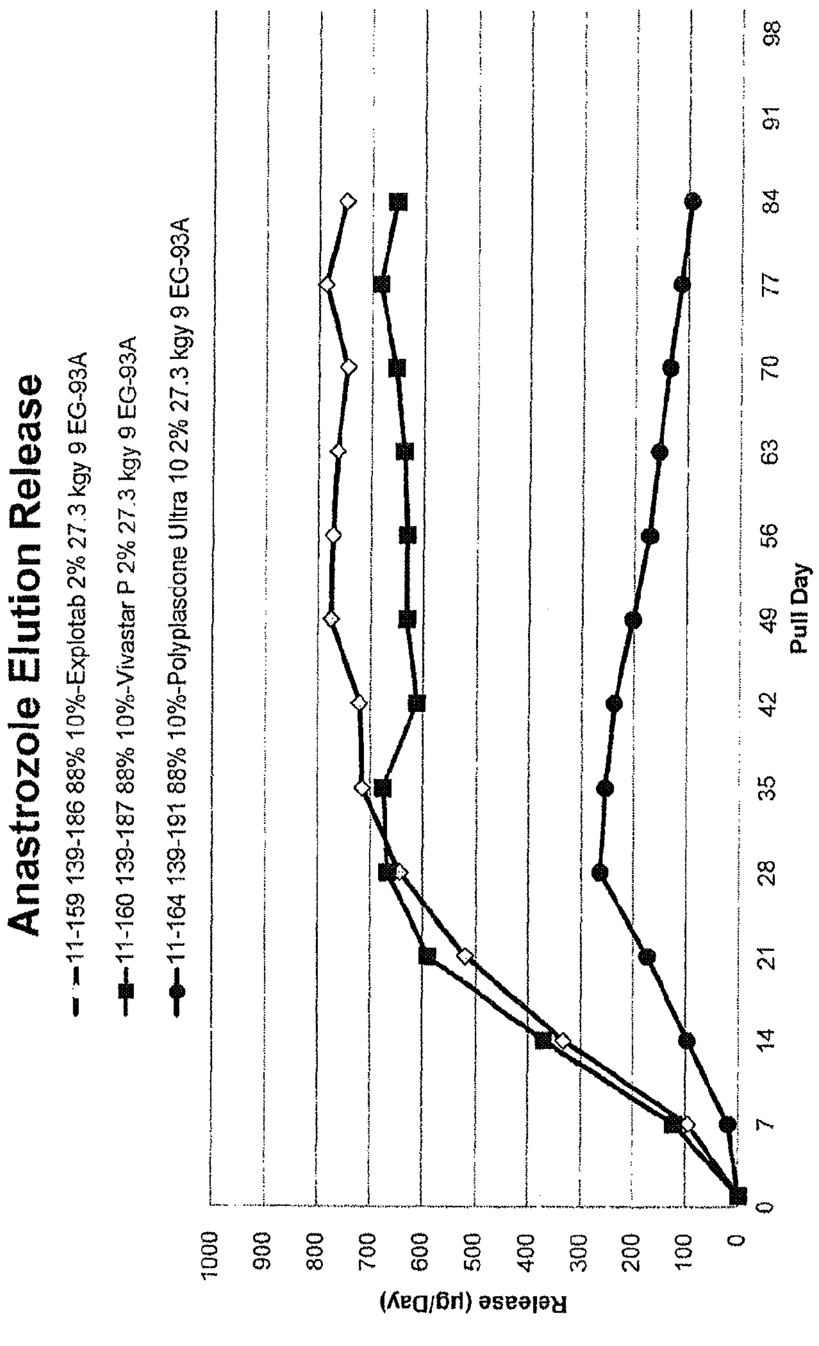

Anastrozole Elution Release
11-159 139-186 88% 10%-Explotab 2% 27.3 kgy 9 EG-93A
11-160 139-187 88% 10%-Vivastar P 2% 27.3 kgy 9 EG-93A
11-164 139-191 88% 10%-Polyplasdone Ultra 10 2% 27.3 kgy 9 EG-93A
Release (µg/Day)
1000
900
800
700
600
500
400
300
200
100
0
0
7
14
21
28
35
42
49
56
63
70
77
84
91
98
Pull Day


Figure 14

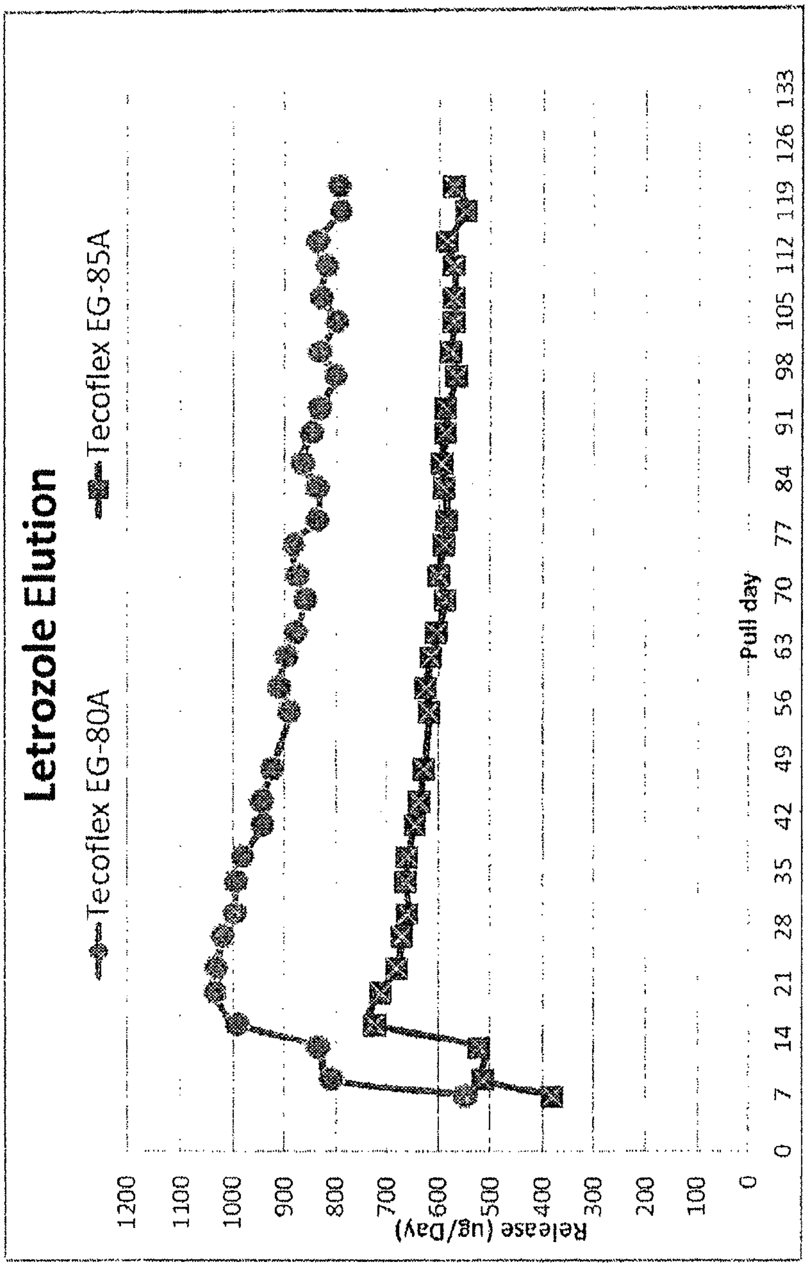
Letrozole Elution
Tecoflex EG-80A
Tecoflex EG-85A
Release (ug/Day)
1200
1100
1000
900
800
700
600
500
400
300
200
100
0
0 7 14 21 28 35 42 49 56 63 70 77 84 91 98 105 112 119 126 133
Pull day

Figure 15

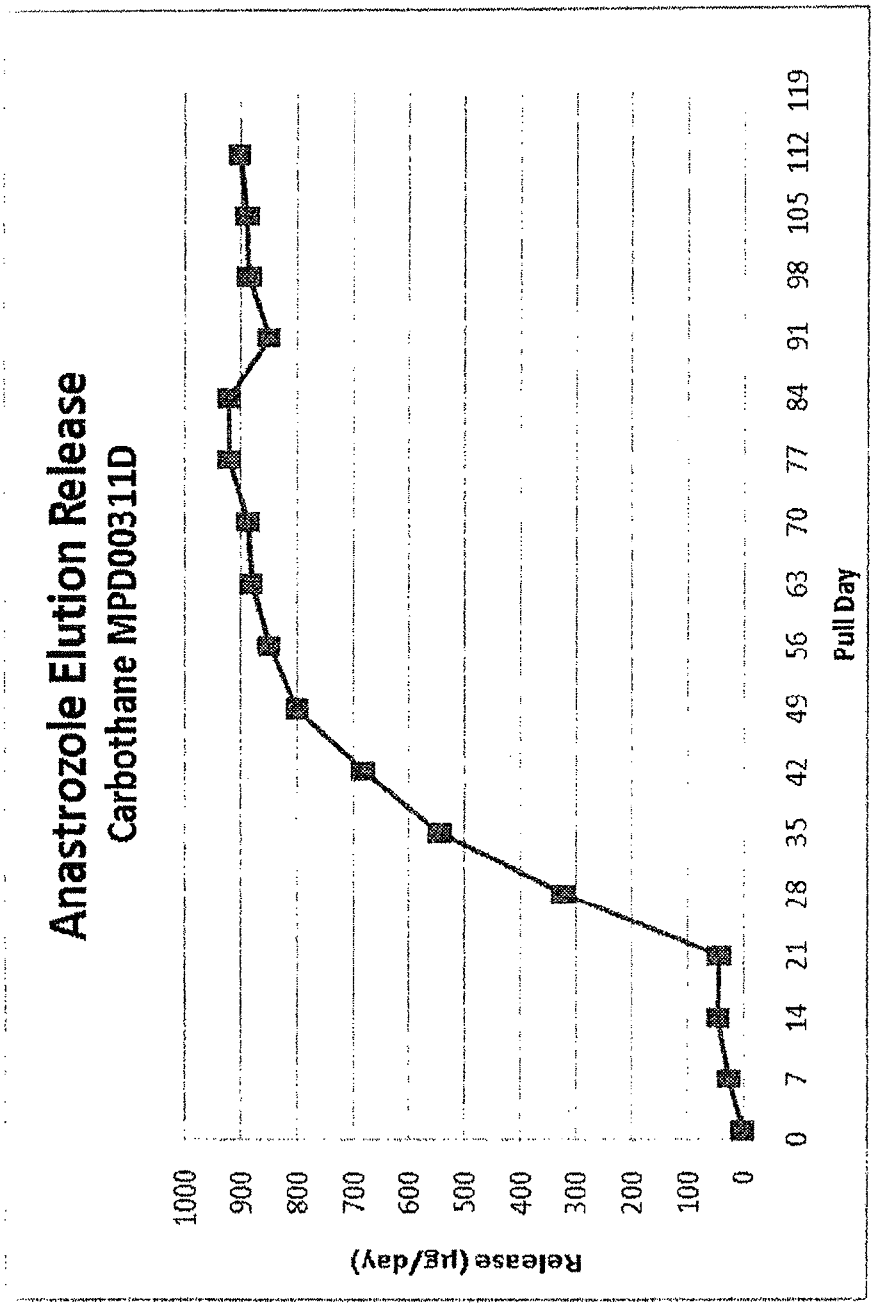
Anastrozole Elution Release
Carbothane MPD00311D
Release (µg/day)
0
100
200
300
400
500
600
700
800
900
1000
0 7 14 21 28 35 42 49 56 63 70 77 84 91 98 105 112 119
Pull Day

Figure 16

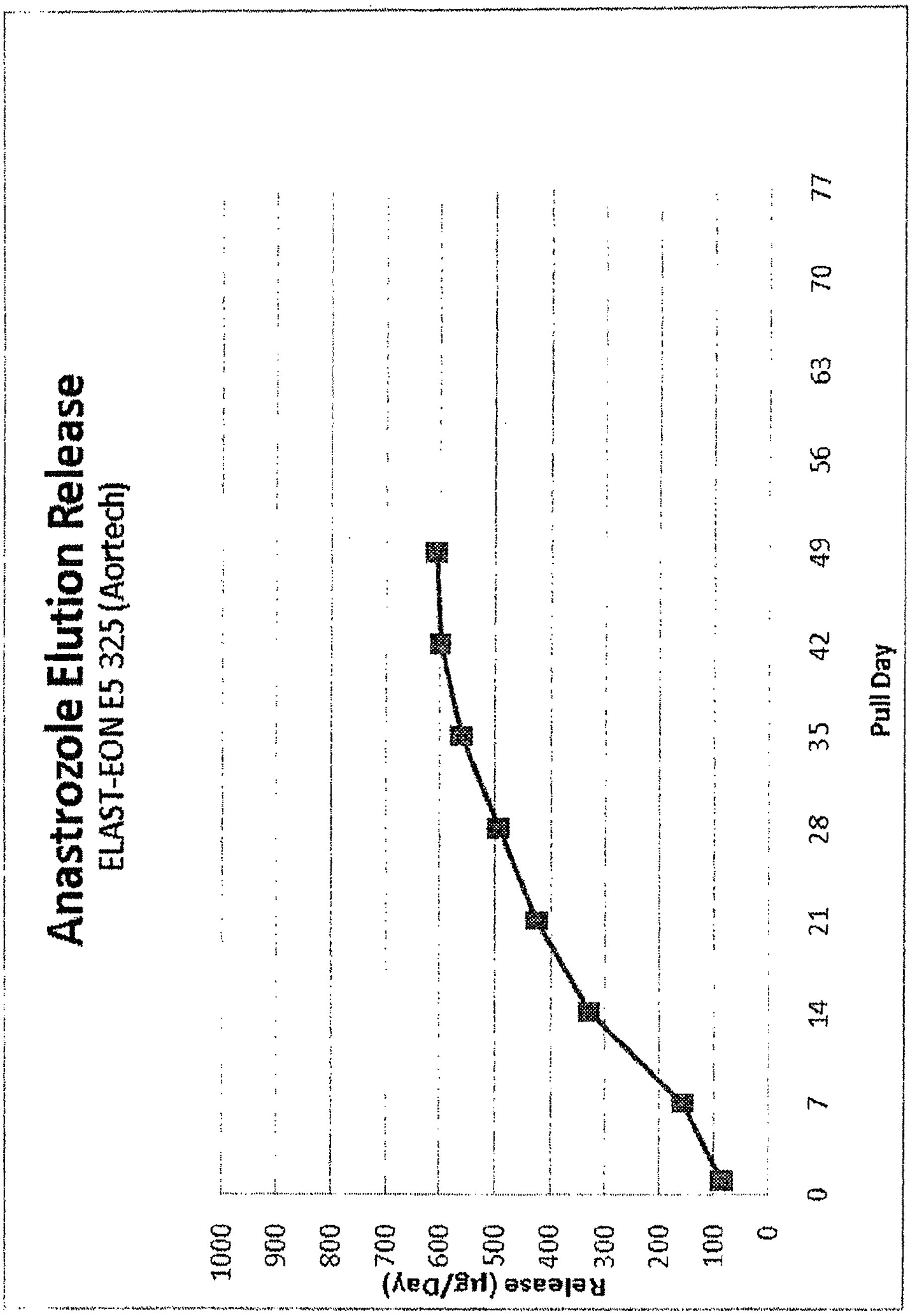
Anastrozole Elution Release
ELAST-EON E5 325 (Aortech)
Release (µg/Day)
1000
900
800
700
600
500
400
300
200
100
0
0
7
14
21
28
35
42
49
56
63
70
77
Pull Day

Figure 17

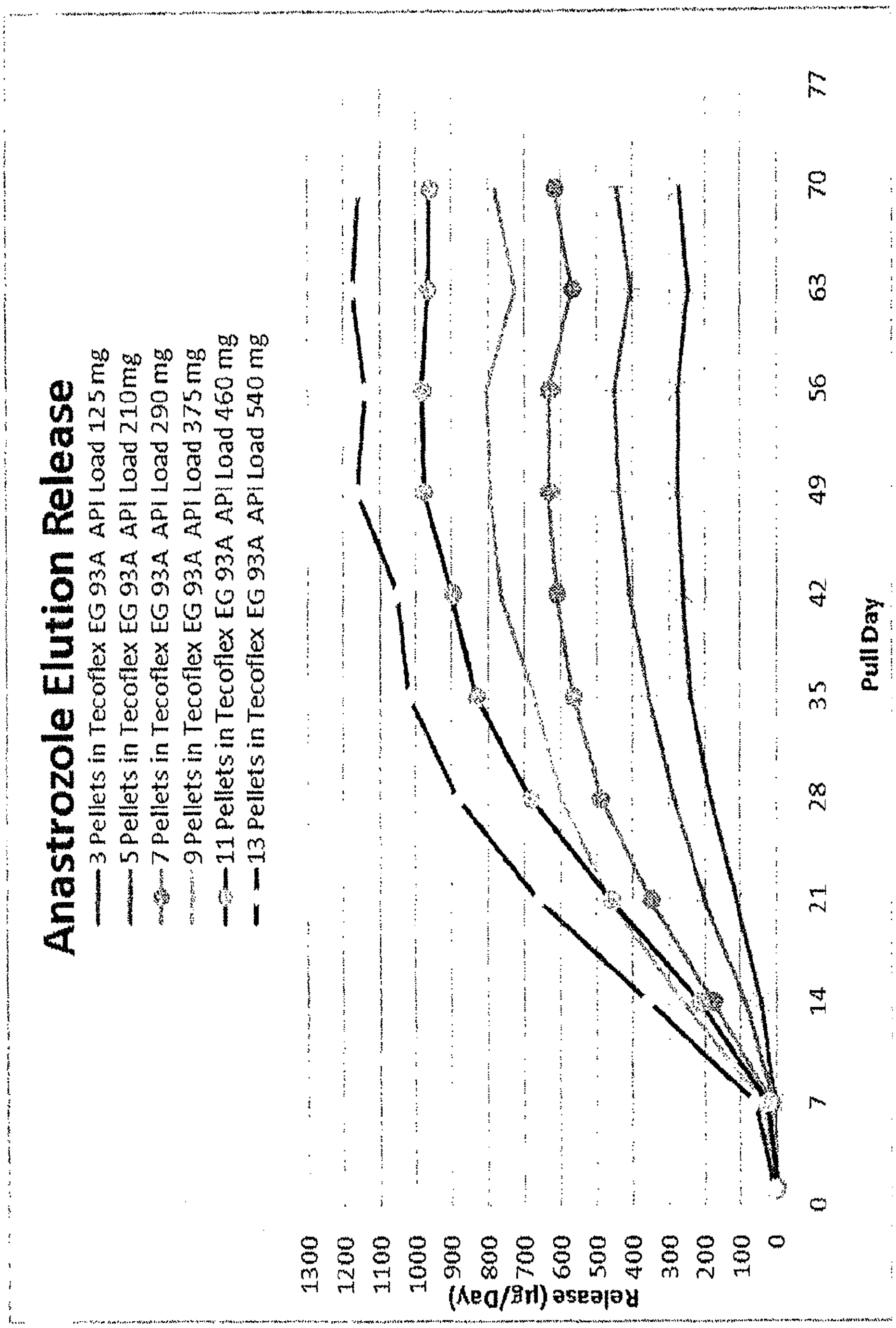

Anastrozole Elution Release
3 Pellets in Tecoflex EG 93A API Load 125 mg
5 Pellets in Tecoflex EG 93A API Load 210mg
7 Pellets in Tecoflex EG 93A API Load 290 mg
9 Pellets in Tecoflex EG 93A API Load 375 mg
11 Pellets in Tecoflex EG 93A API Load 460 mg
13 Pellets in Tecoflex EG 93A API Load 540 mg
Release (µg/Day)
1300
1200
1100
1000
900
800
700
600
500
400
300
200
100
0
0
7
14
21
28
35
42
49
56
63
70
77
Pull Day


Figure 18

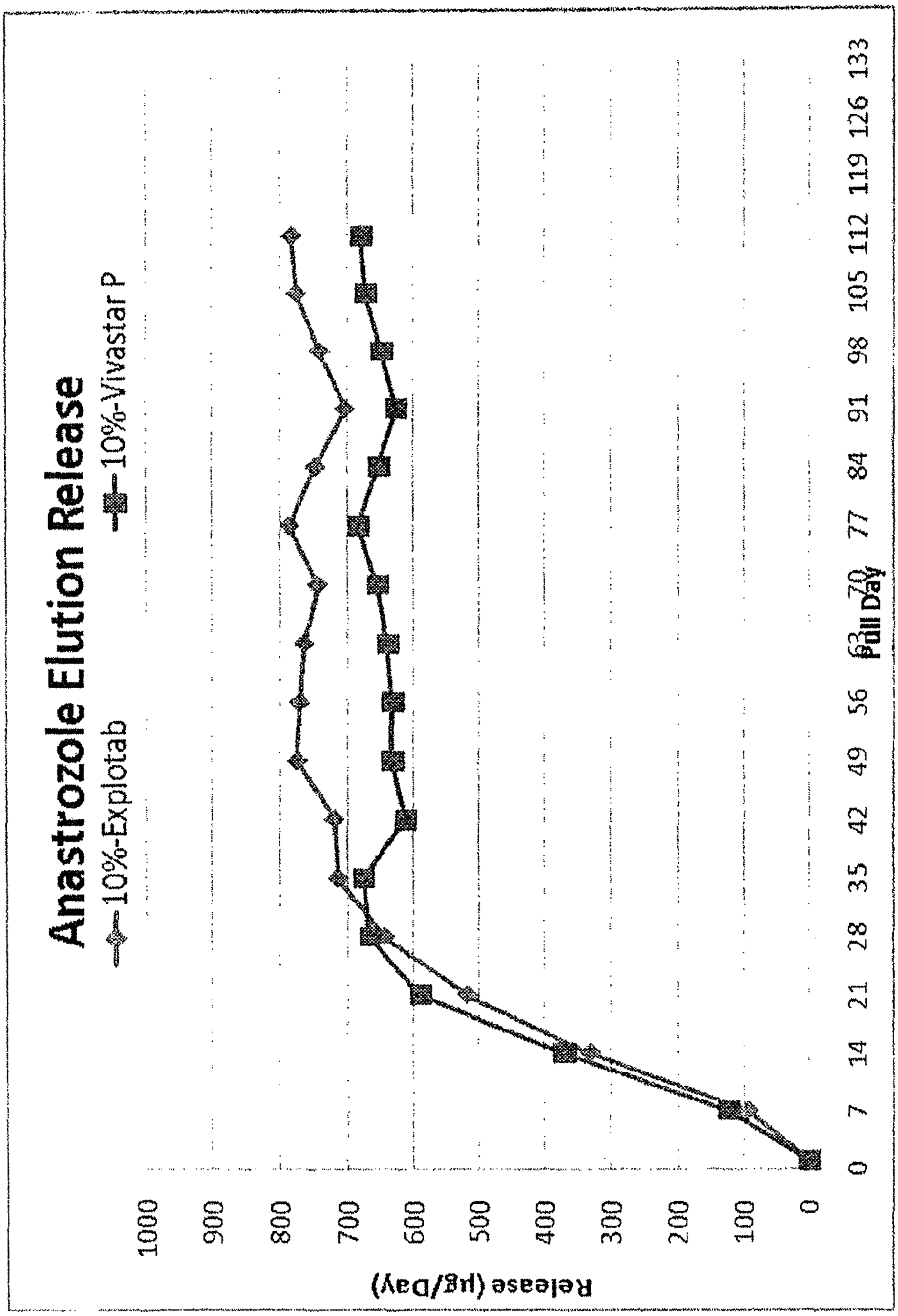
Anastrozole Elution Release
10%-Explotab
10%-Vivastar P
Release (µg/Day)
1000
900
800
700
600
500
400
300
200
100
0
0 7 14 21 28 35 42 49 56 63 70 77 84 91 98 105 112 119 126 133
Pull Day

Figure 19

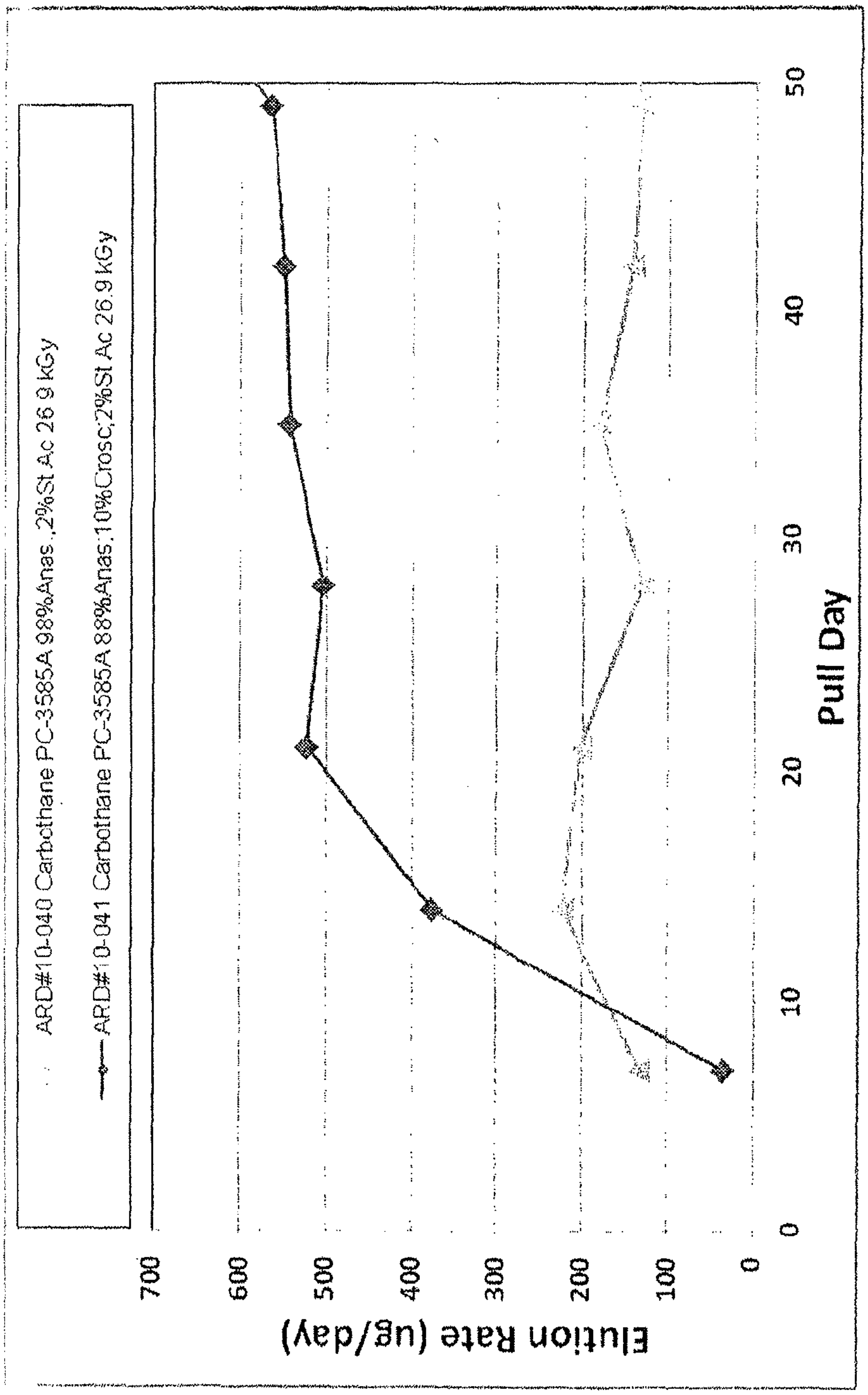
Anastrozole
ARD#10-040 Carbothane PC-3585A 98%Anas.,2%St Ac 26.9 kGy
ARD#10-041 Carbothane PC-3585A 88%Anas;10%Crosc;2%St Ac 26.9 kGy
Elution Rate (ug/day)
700
600
500
400
300
200
100
0
0
10
20
30
40
50
Pull Day

Figure 20

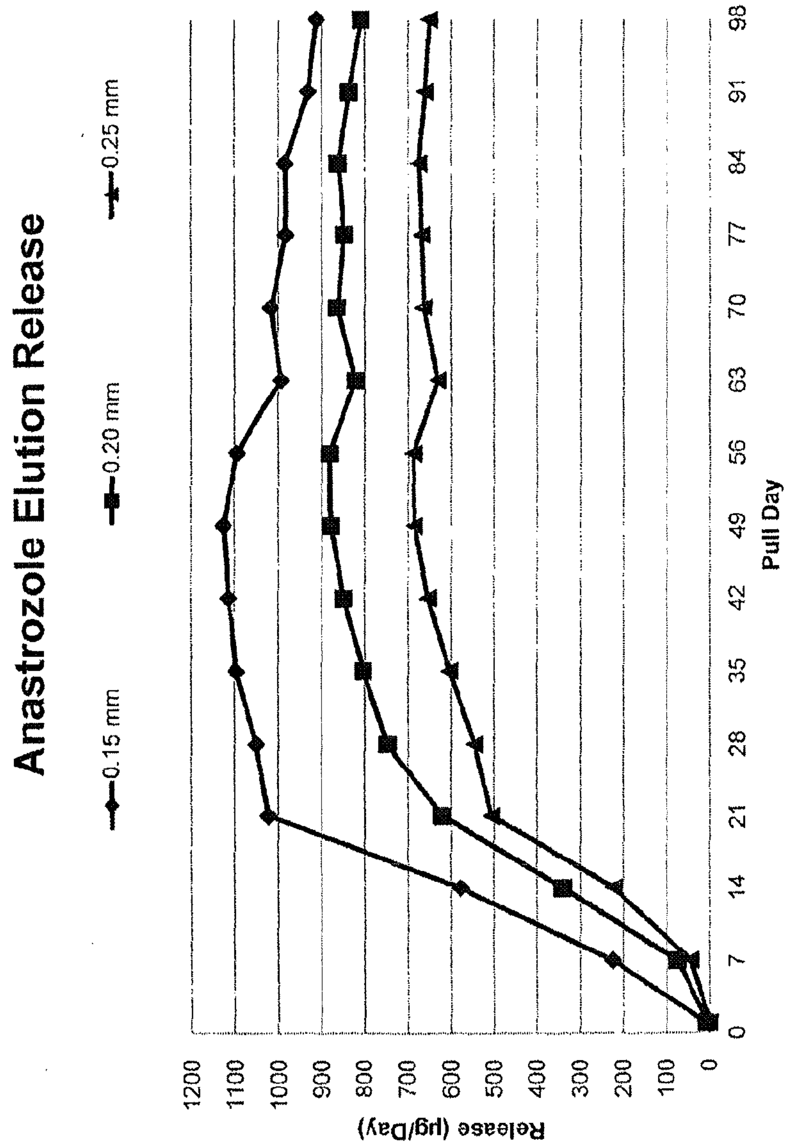

Anastrozole Elution Release
0.15 mm
0.20 mm
0.25 mm
Release (µg/Day)
1200
1100
1000
900
800
700
600
500
400
300
200
100
0
0
7
14
21
28
35
42
49
56
63
70
77
84
91
98
Pull Day


Figure 21

Anastrozole

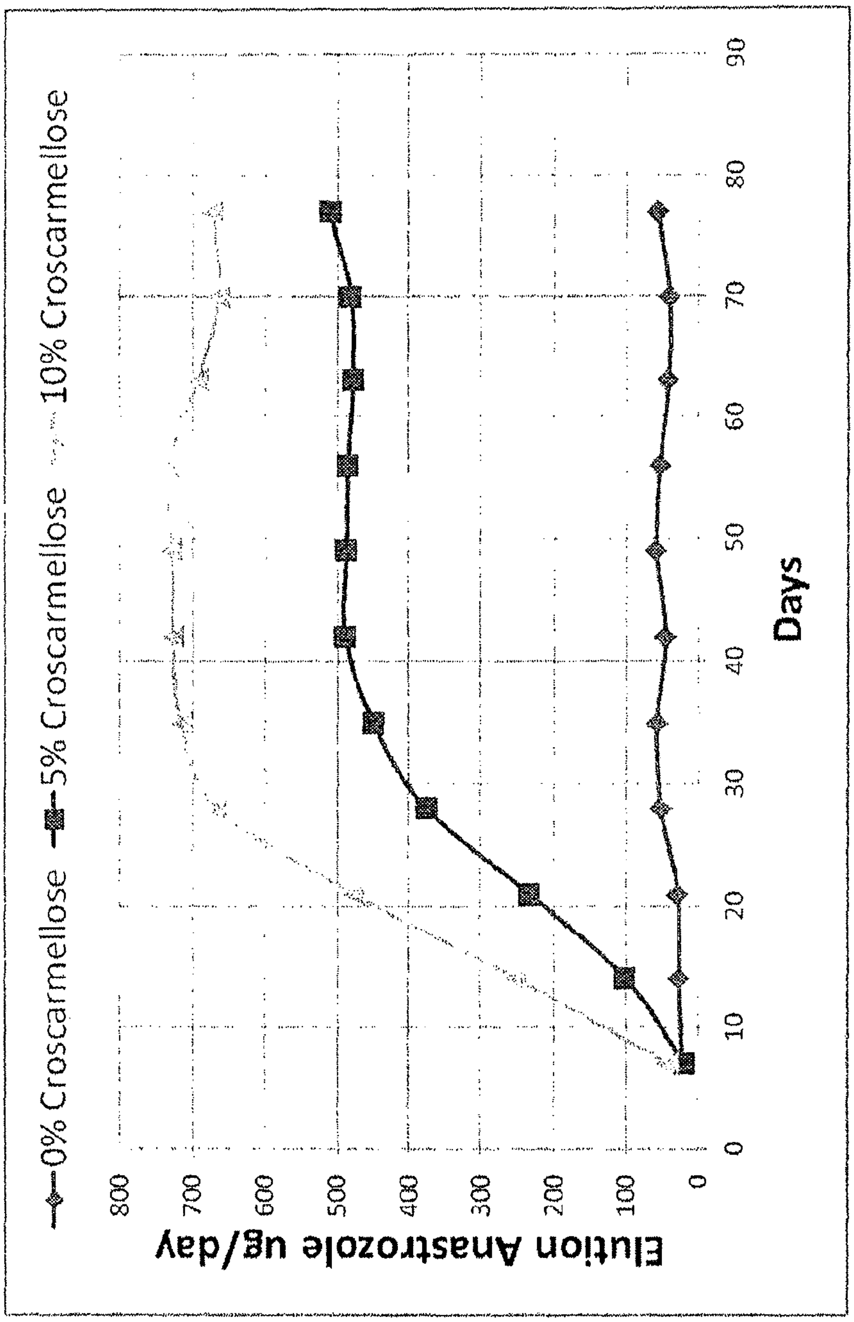


Figure 22

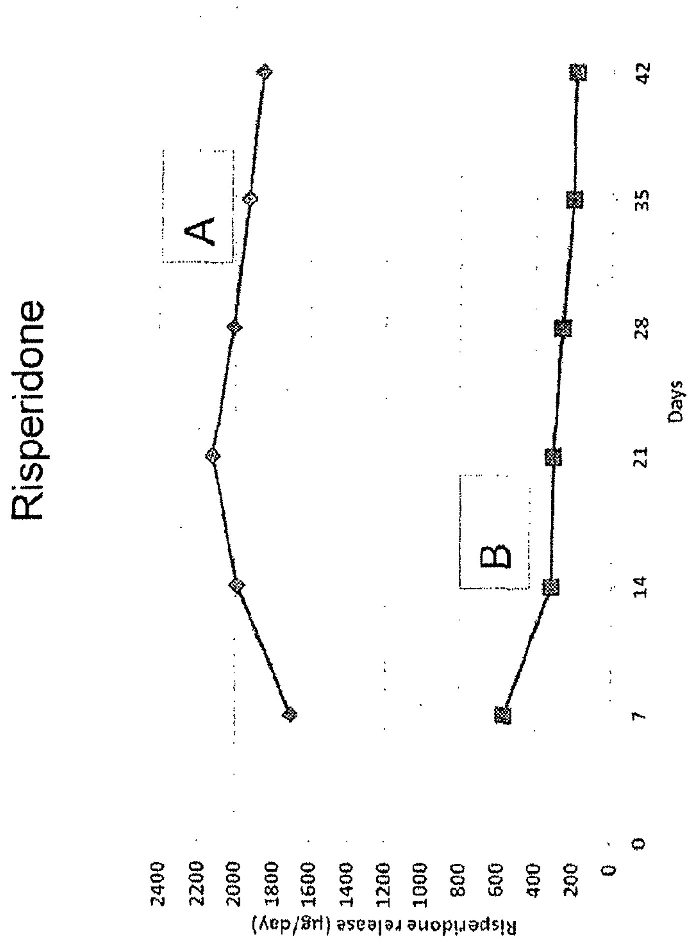
Risperidone
A
B
Risperidone release (μg/day)
2400
2200
2000
1800
1600
1400
1200
1000
800
600
400
200
0
0
7
14
21
28
35
42
Days

Figure 23

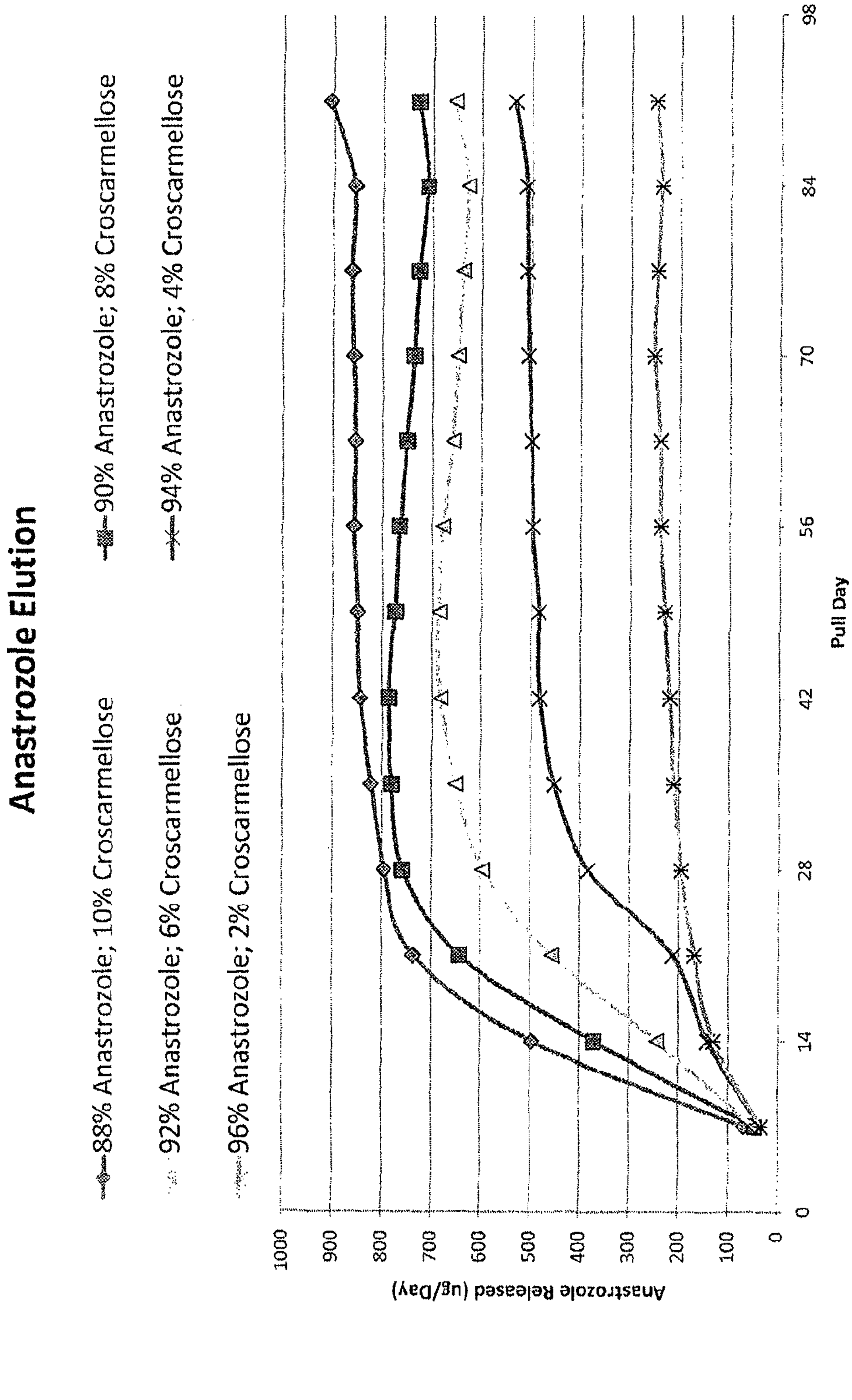

Anastrozole Elution
88% Anastrozole; 10% Croscarmellose
90% Anastrozole; 8% Croscarmellose
92% Anastrozole; 6% Croscarmellose
94% Anastrozole; 4% Croscarmellose
96% Anastrozole; 2% Croscarmellose
Anastrozole Released (ug/Day)
1000
900
800
700
600
500
400
300
200
100
0
0
14
28
42
56
70
84
98
Pull Day


Figure 24

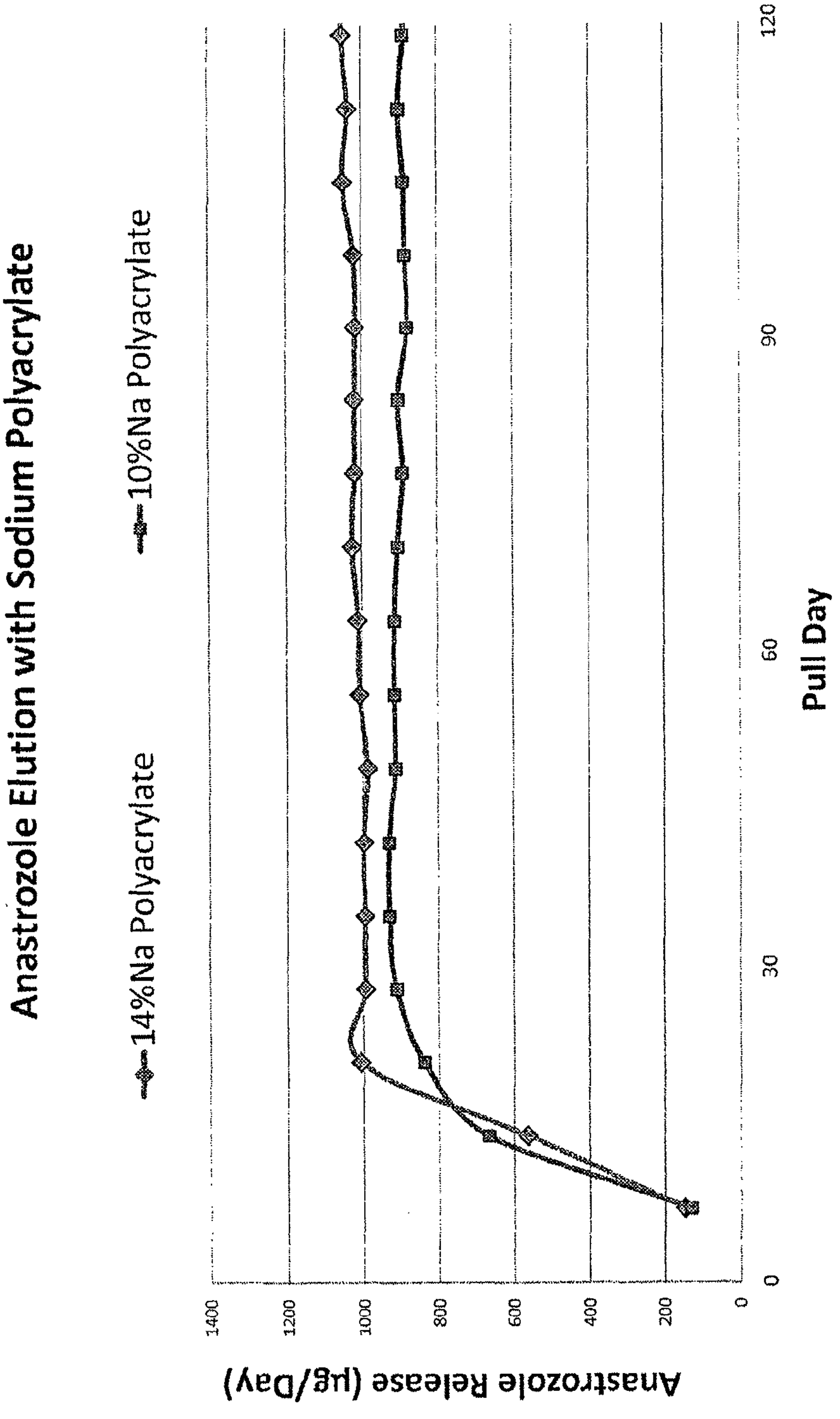
Anastrozole Elution with Sodium Polyacrylate
14%Na Polyacrylate
10%Na Polyacrylate
Anastrozole Release (µg/Day)
0
200
400
600
800
1000
1200
1400
0
30
60
90
120
Pull Day

Figure 25

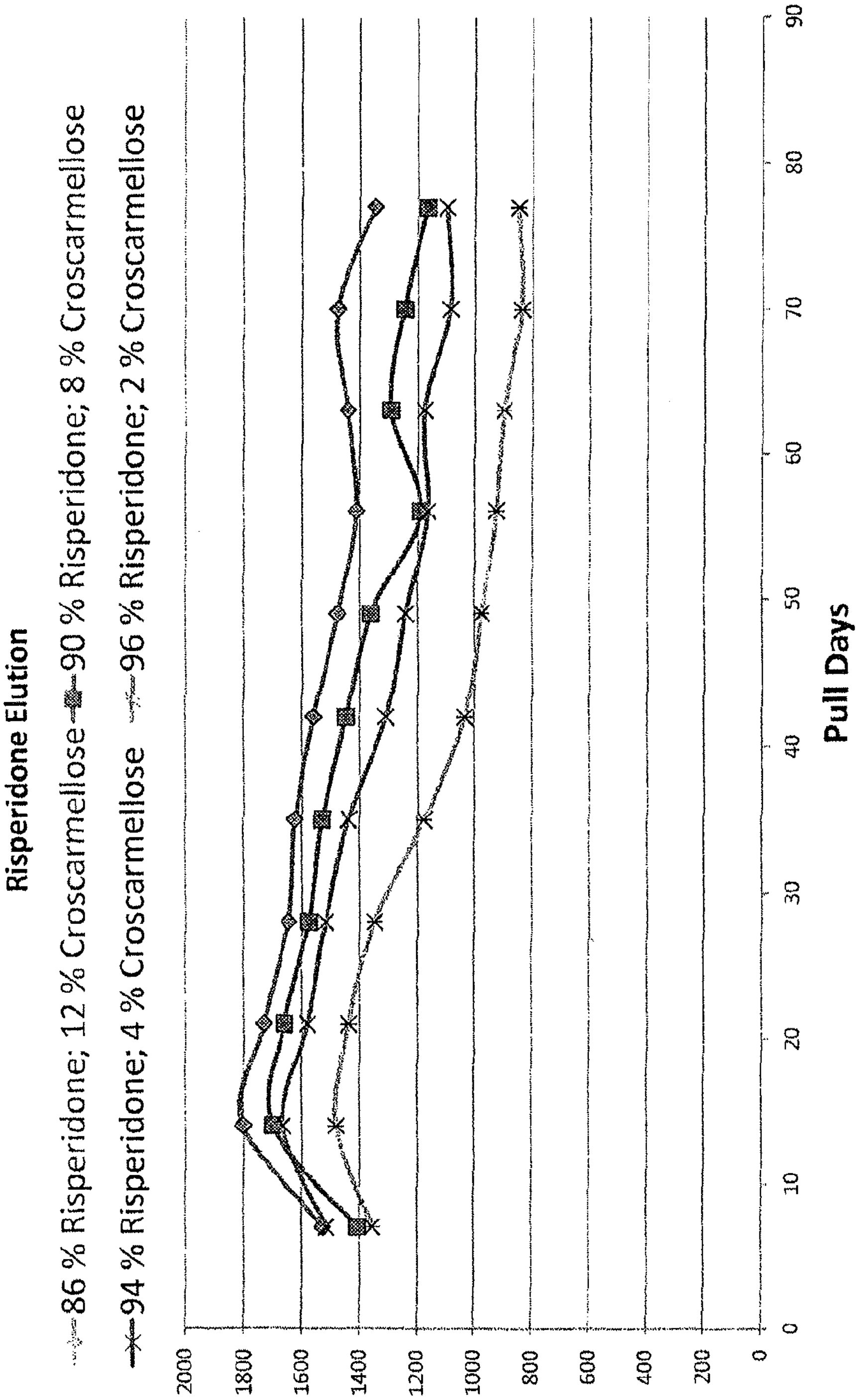
Risperidone Elution
86 % Risperidone; 12 % Croscarmellose
90 % Risperidone; 8 % Croscarmellose
94 % Risperidone; 4 % Croscarmellose
96 % Risperidone; 2 % Croscarmellose
Risperidone Release (µg/day)
2000
1800
1600
1400
1200
1000
800
600
400
200
0
0
10
20
30
40
50
60
70
80
90
Pull Days

Figure 26

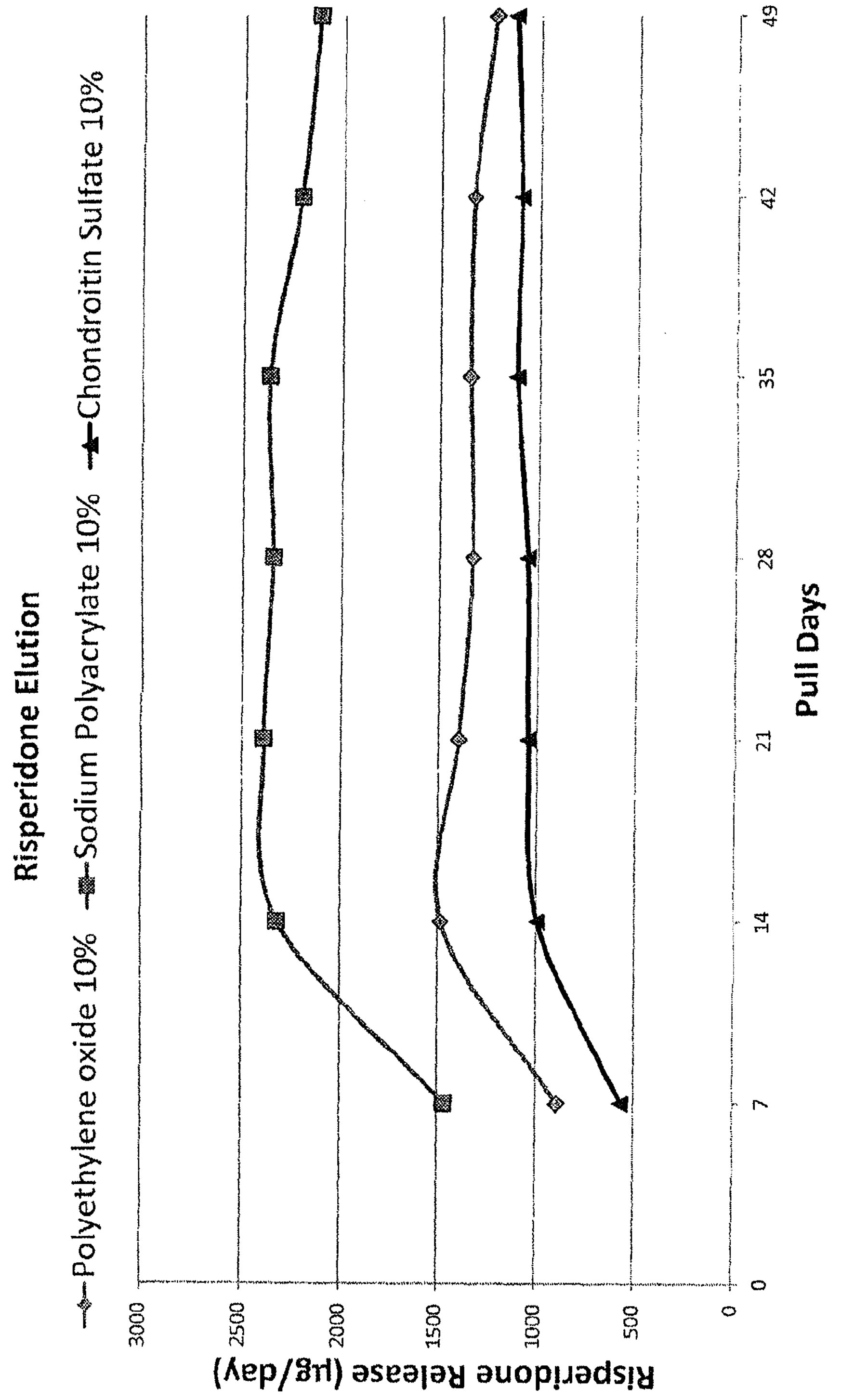
Risperidone Elution
Polyethylene oxide 10%
Sodium Polyacrylate 10%
Chondroitin Sulfate 10%
Risperidone Release (µg/day)
3000
2500
2000
1500
1000
500
0
0
7
14
21
28
35
42
49
Pull Days

Figure 27

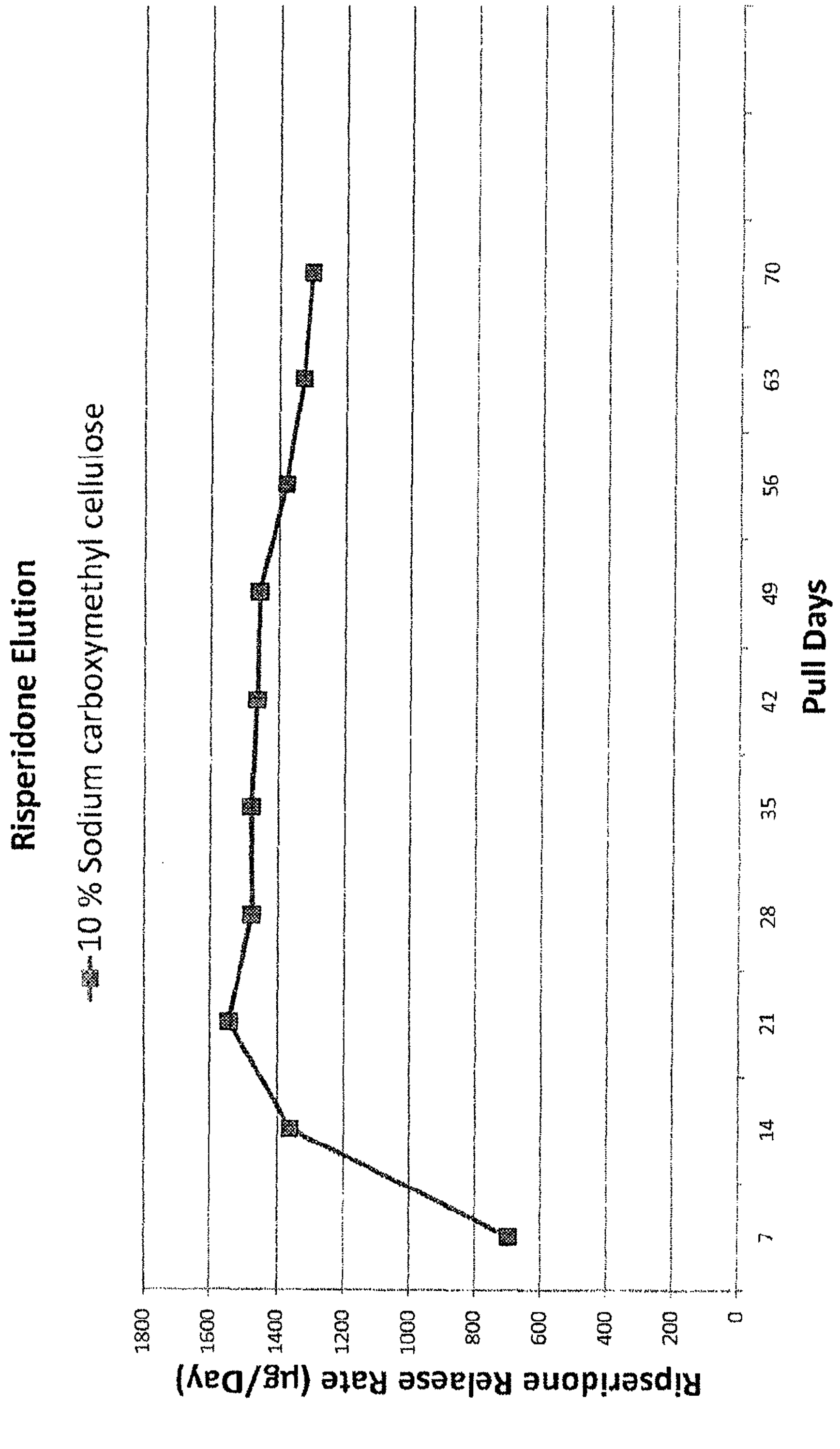
Risperidone Elution
10 % Sodium carboxymethyl cellulose
Ripseridone Relaese Rate (µg/Day)
1800
1600
1400
1200
1000
800
600
400
200
0
7
14
21
28
35
42
49
56
63
70
Pull Days

Figure 28

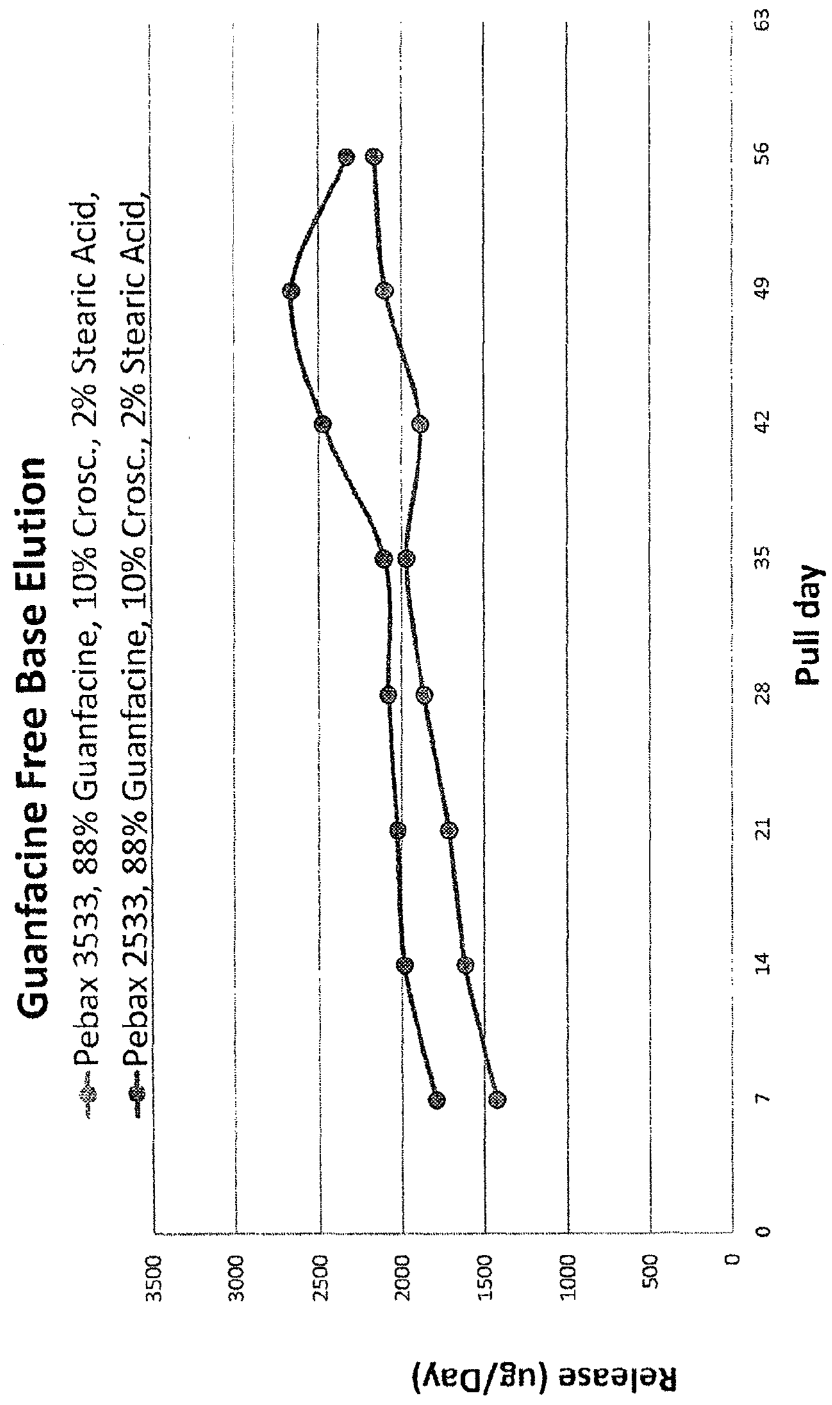
Guanfacine Free Base Elution
Pebax 3533, 88% Guanfacine, 10% Crosc., 2% Stearic Acid,
Pebax 2533, 88% Guanfacine, 10% Crosc., 2% Stearic Acid,
Release (ug/Day)
3500
3000
2500
2000
1500
1000
500
0
0
7
14
21
28
35
42
49
56
63
Pull day

Figure 29

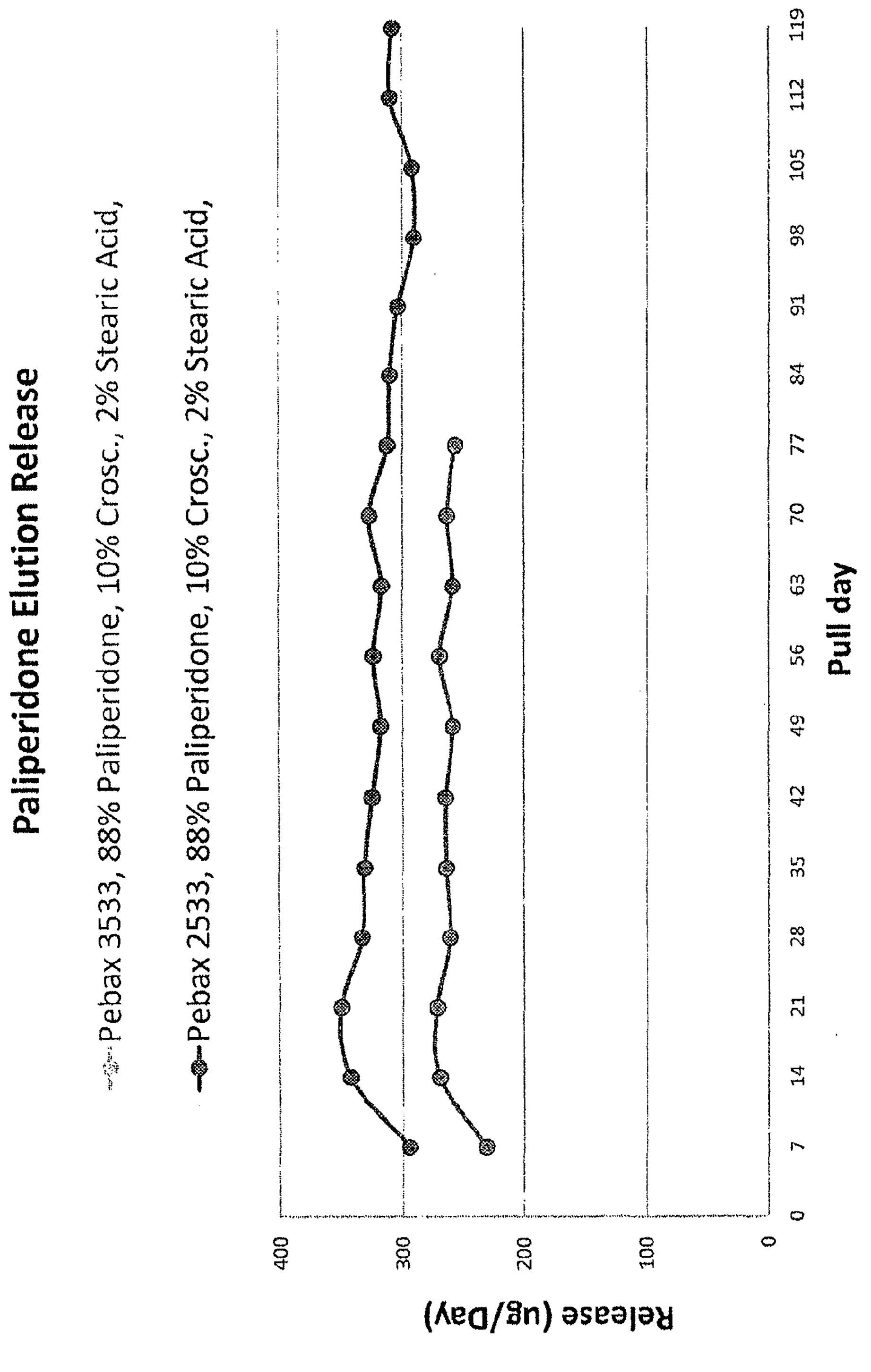
Paliperidone Elution Release
Pebax 3533, 88% Paliperidone, 10% Crosc., 2% Stearic Acid,
Pebax 2533, 88% Paliperidone, 10% Crosc., 2% Stearic Acid,
Release (ug/Day)
0
100
200
300
400
Pull day
0
7
14
21
28
35
42
49
56
63
70
77
84
91
98
105
112
119

Figure 30

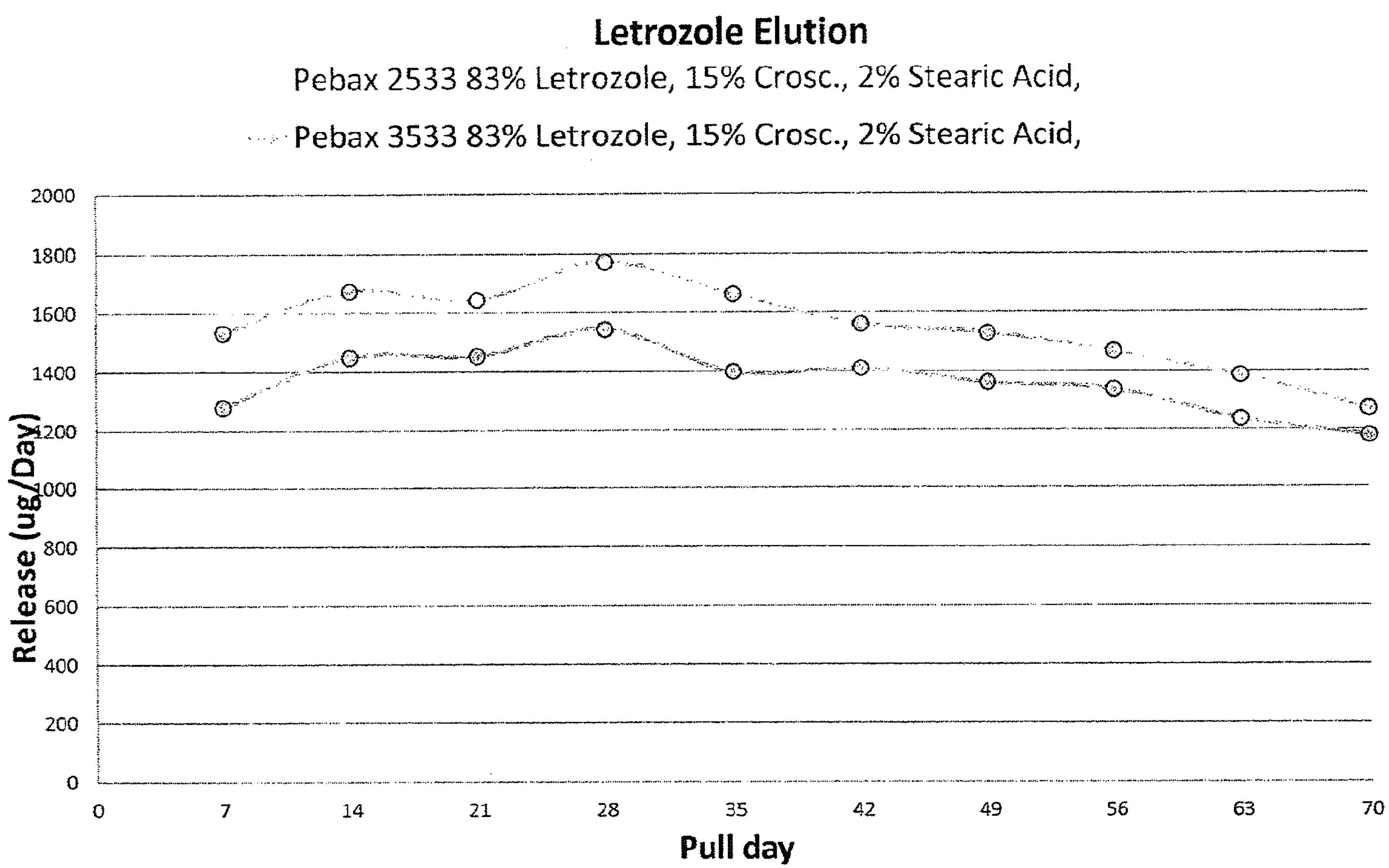
Letrozole Elution
Pebax 2533 83% Letrozole, 15% Crosc., 2% Stearic Acid,
Pebax 3533 83% Letrozole, 15% Crosc., 2% Stearic Acid,
2000
1800
1600
1400
1200
1000
800
600
400
200
0
Release (ug/Day)
0
7
14
21
28
35
42
49
56
63
70
Pull day

Figure 31

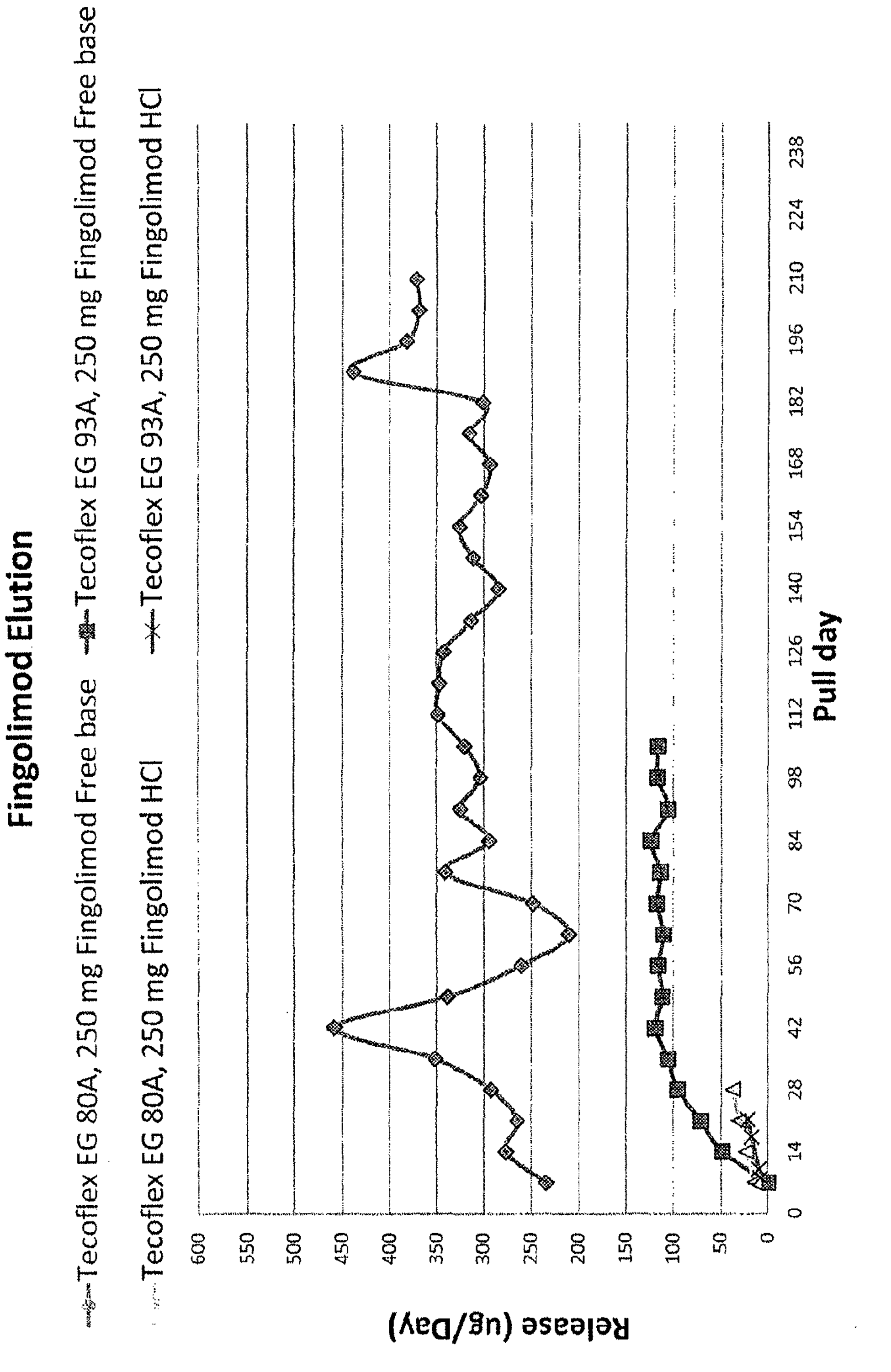
Fingolimod Elution
Tecoflex EG 80A, 250 mg Fingolimod Free base
Tecoflex EG 93A, 250 mg Fingolimod Free base
Tecoflex EG 80A, 250 mg Fingolimod HCl
Tecoflex EG 93A, 250 mg Fingolimod HCl
Release (ug/Day)
0
50
100
150
200
250
300
350
400
450
500
550
600
Pull day
0
14
28
42
56
70
84
98
112
126
140
154
168
182
196
210
224
238

Figure 32

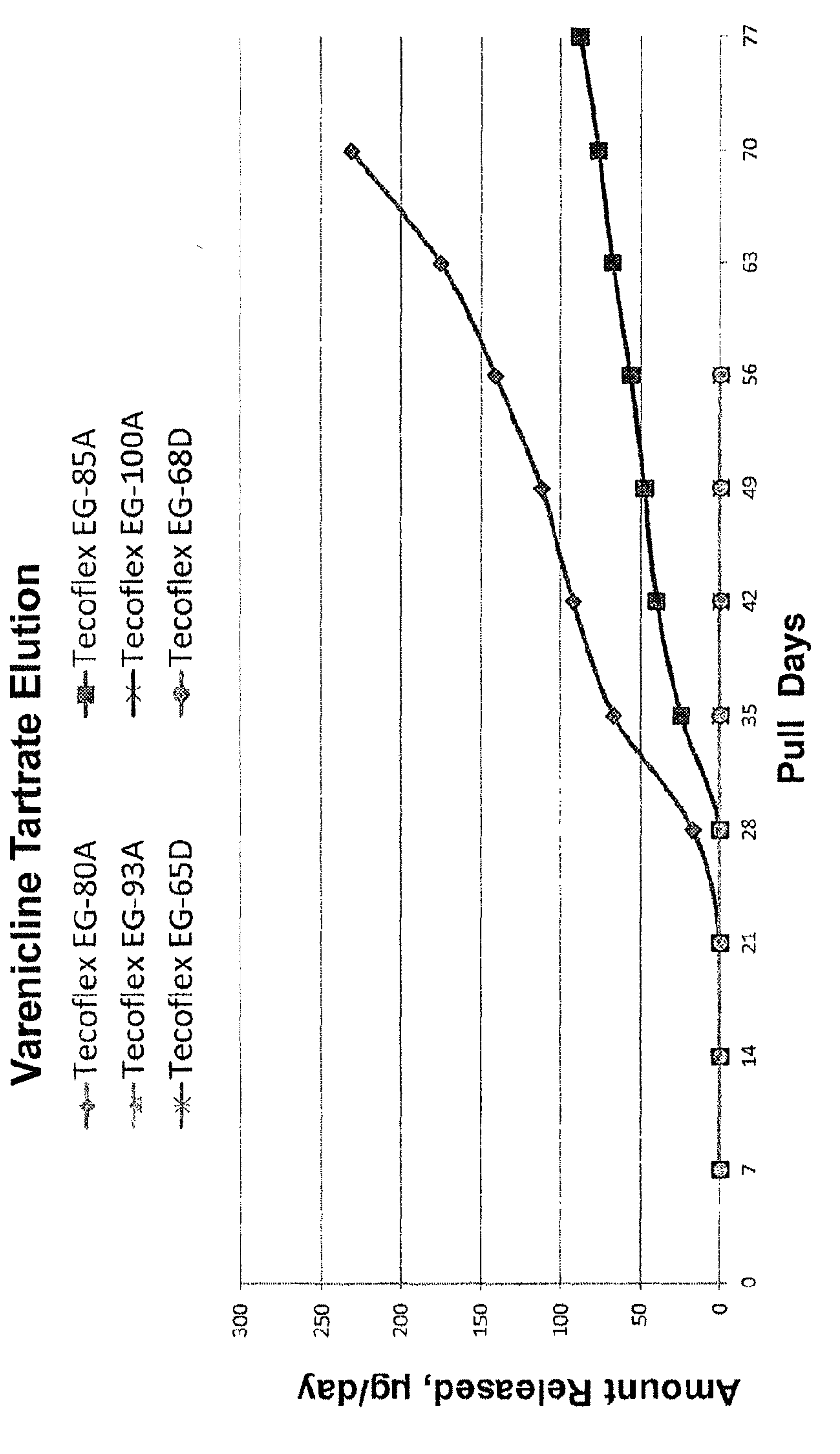
Varenicline Tartrate Elution
Tecoflex EG-80A
Tecoflex EG-85A
Tecoflex EG-93A
Tecoflex EG-100A
Tecoflex EG-65D
Tecoflex EG-68D
Amount Released, µg/day
0
50
100
150
200
250
300
0
7
14
21
28
35
42
49
56
63
70
77
Pull Days

Figure 33

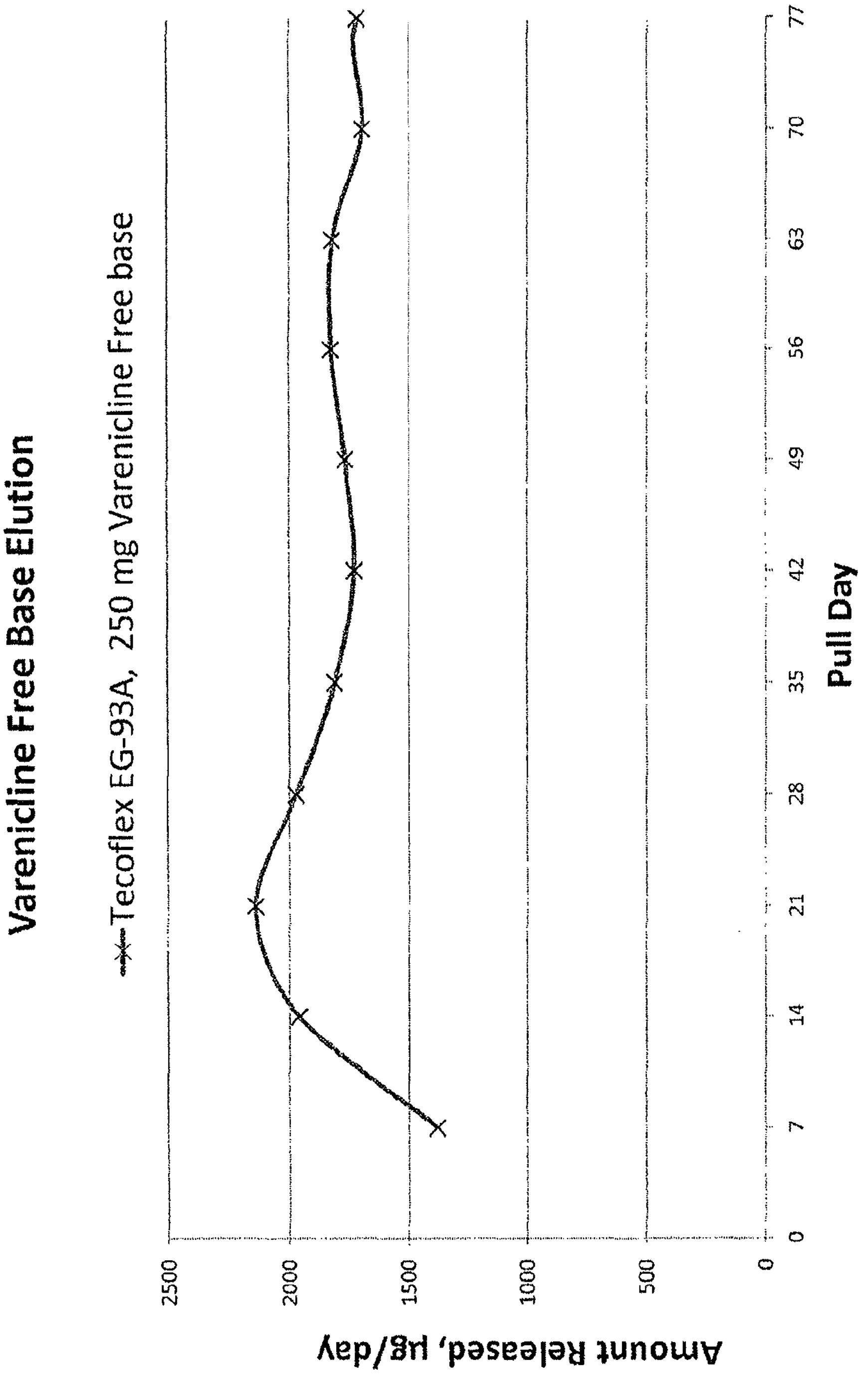
Varenicline Free Base Elution
Tecoflex EG-93A, 250 mg Varenicline Free base
Amount Released, µg/day
2500
2000
1500
1000
500
0
0
7
14
21
28
35
42
49
56
63
70
77
Pull Day

Figure 34

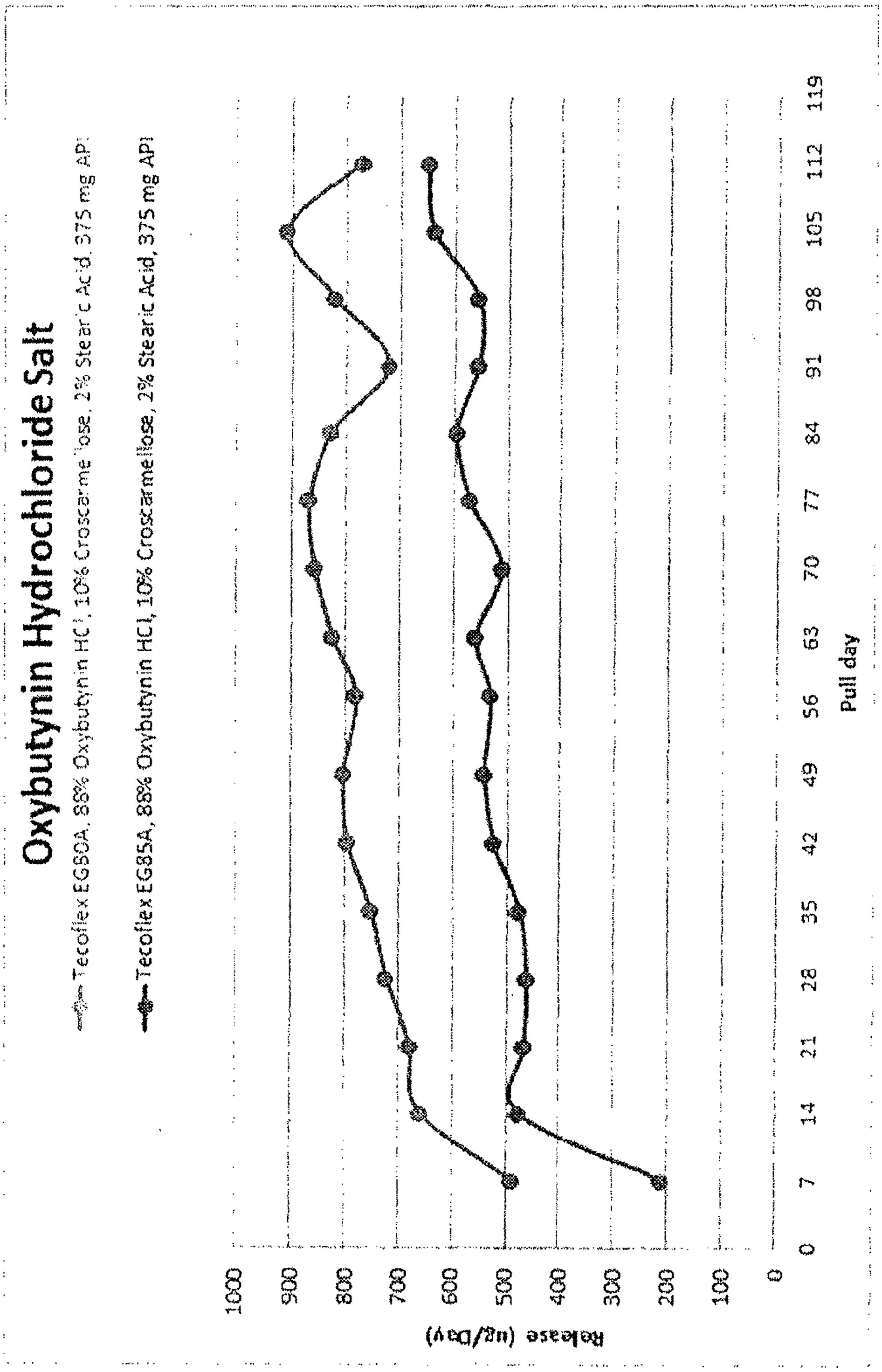
Oxybutynin Hydrochloride Salt
Tecoflex EG80A, 88% Oxybutynin HCl, 10% Croscarmellose, 2% Stearic Acid, 375 mg API
Tecoflex EG85A, 88% Oxybutynin HCl, 10% Croscarmellose, 2% Stearic Acid, 375 mg API
Release (ug/Day)
1000
900
800
700
600
500
400
300
200
100
0
0
7
14
21
28
35
42
49
56
63
70
77
84
91
98
105
112
119
Pull day

Figure 35

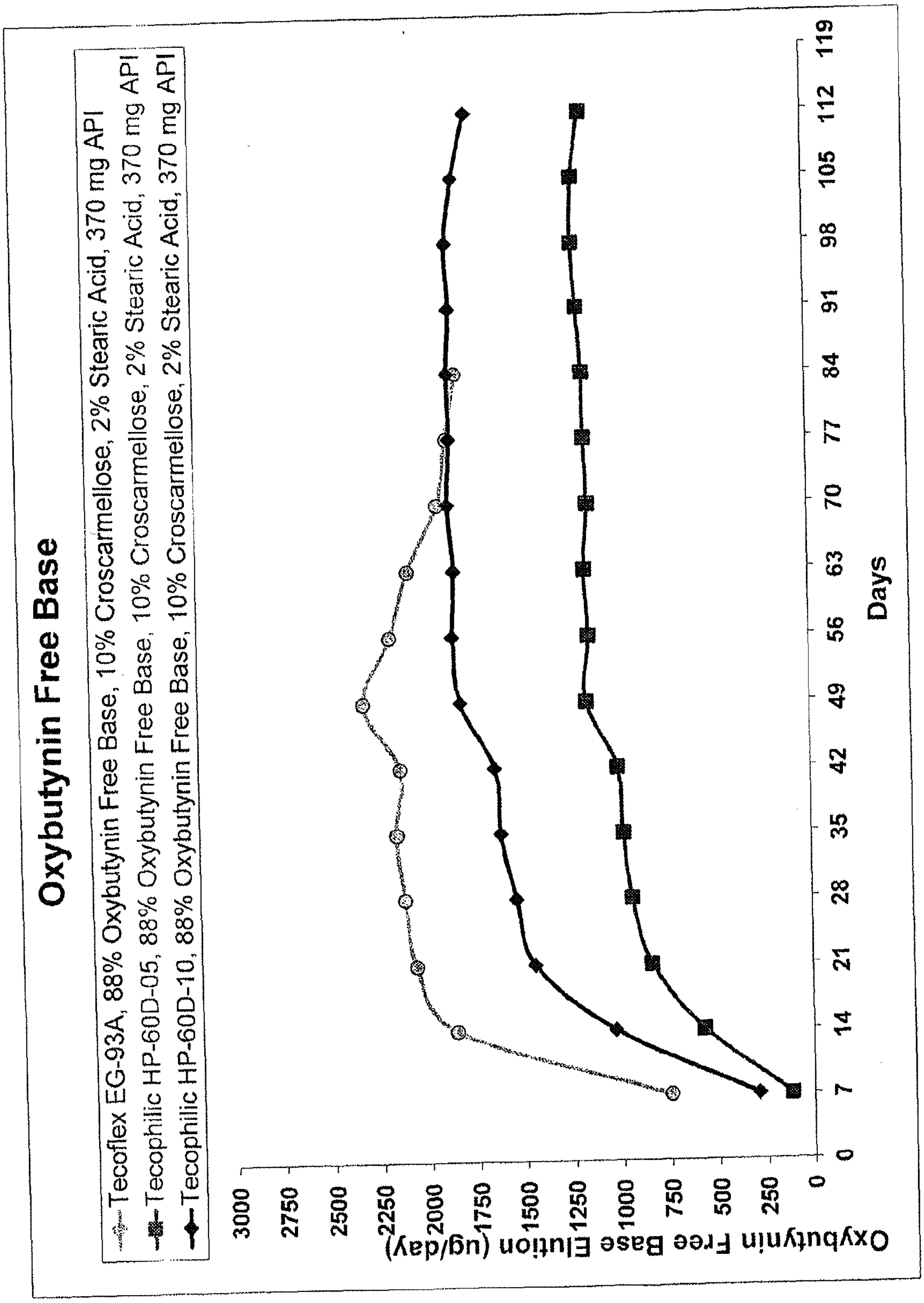
Oxybutynin Free Base
Tecoflex EG-93A, 88% Oxybutynin Free Base, 10% Croscarmellose, 2% Stearic Acid, 370 mg API
Tecophilic HP-60D-05, 88% Oxybutynin Free Base, 10% Croscarmellose, 2% Stearic Acid, 370 mg API
Tecophilic HP-60D-10, 88% Oxybutynin Free Base, 10% Croscarmellose, 2% Stearic Acid, 370 mg API
Oxybutynin Free Base Elution (ug/day)
3000
2750
2500
2250
2000
1750
1500
1250
1000
750
500
250
0
0
7
14
21
28
35
42
49
56
63
70
77
84
91
98
105
112
119
Days

Figure 36

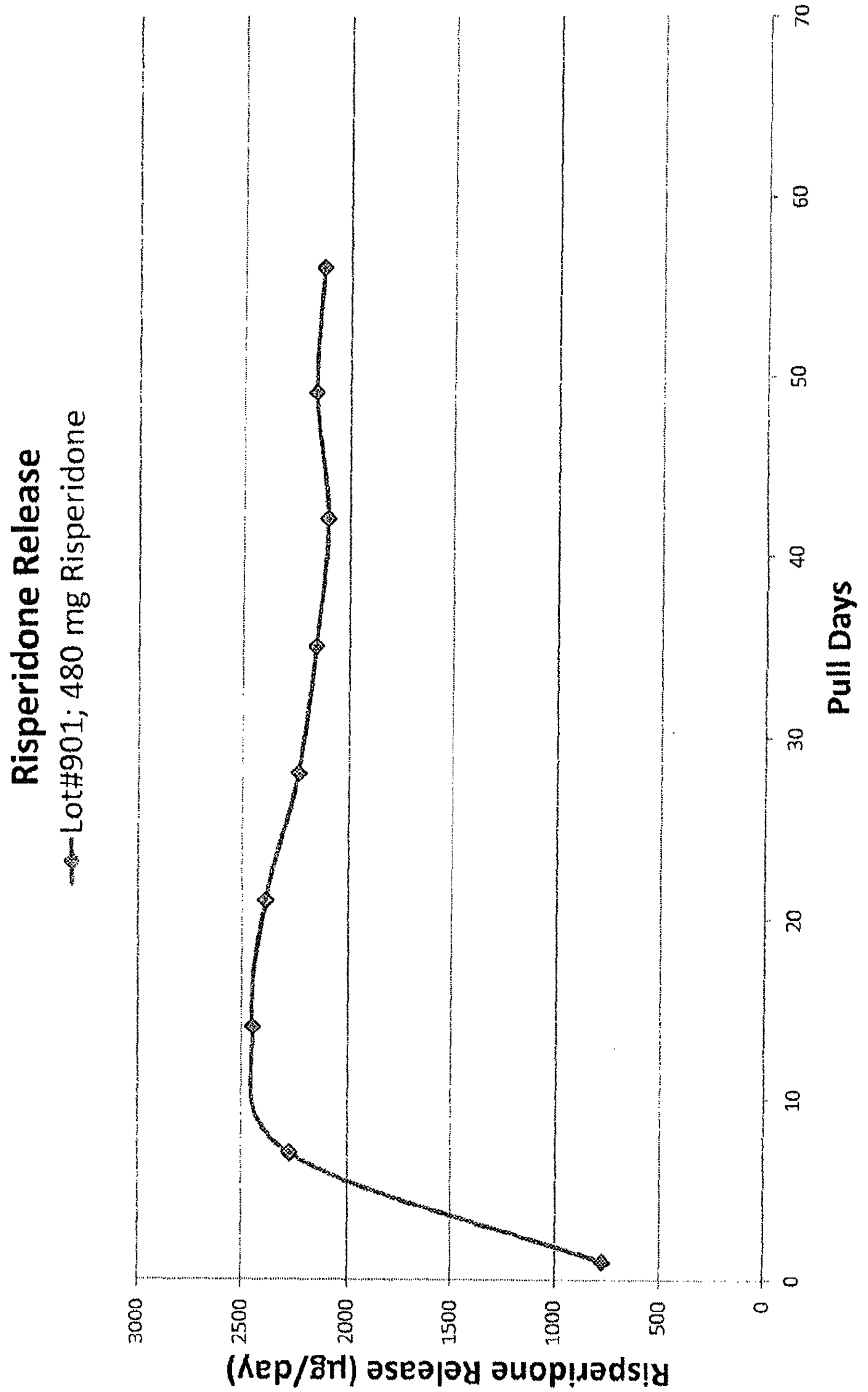

Risperidone Release
Lot#901; 480 mg Risperidone
Risperidone Release (µg/day)
3000
2500
2000
1500
1000
500
0
0
10
20
30
40
50
60
70
Pull Days


Figure 37

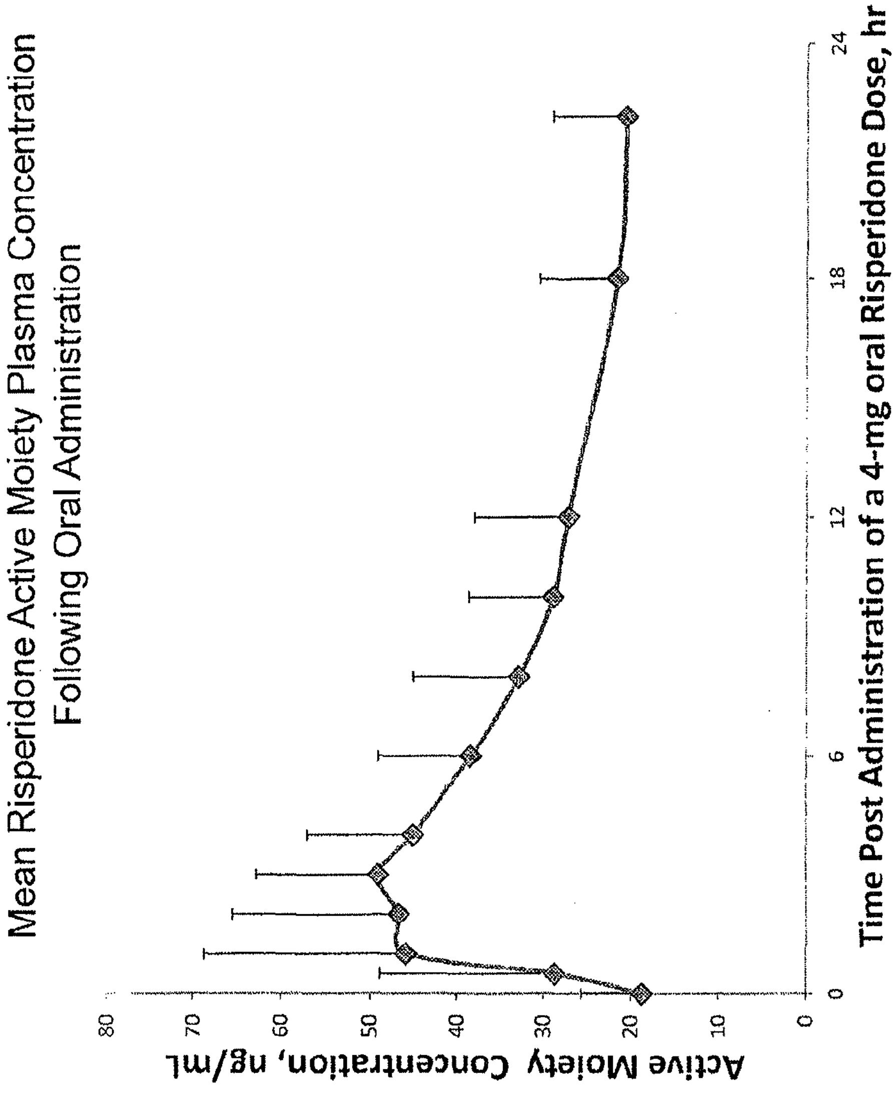
Mean Risperidone Active Moiety Plasma Concentration
Following Oral Administration
Active Moiety Concentration, ng/mL
0
10
20
30
40
50
60
70
80
0
6
12
18
24
Time Post Administration of a 4-mg oral Risperidone Dose, hr

Figure 38

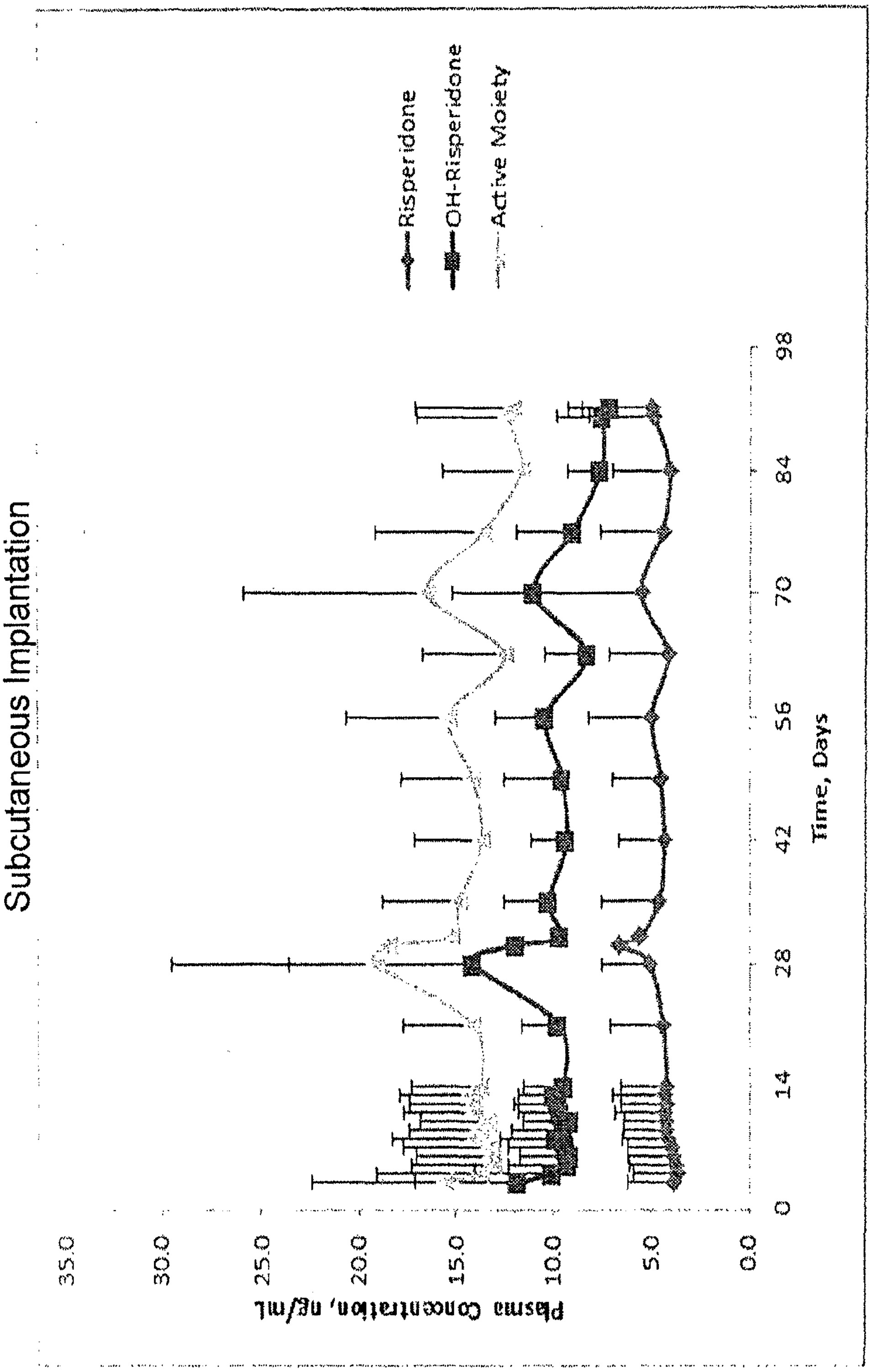
Mean Risperidone Plasma Concentrations Following Subcutaneous Implantation
Plasma Concentration, ng/mL
35.0
30.0
25.0
20.0
15.0
10.0
5.0
0.0
0
14
28
42
56
70
84
98
Time, Days
Risperidone
OH-Risperidone
Active Moiety

Figure 39

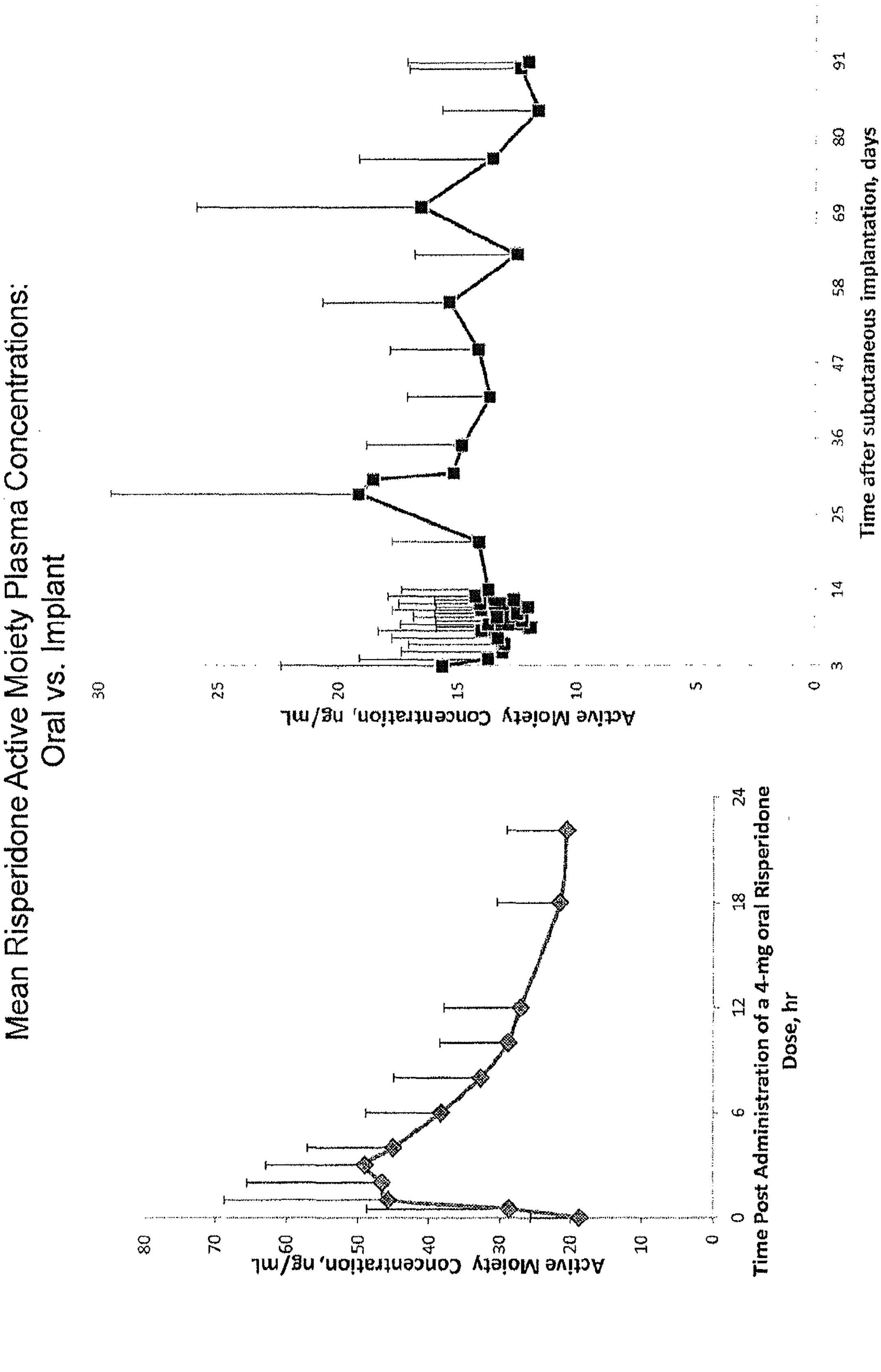
Mean Risperidone Active Moiety Plasma Concentrations:
Oral vs. Implant
Active Moiety Concentration, ng/mL
0
10
20
30
40
50
60
70
80
0
6
12
18
24
Time Post Administration of a 4-mg oral Risperidone Dose, hr
Active Moiety Concentration, ng/mL
0
5
10
15
20
25
30
3
14
25
36
47
58
69
80
91
Time after subcutaneous implantation, days

Figure 40

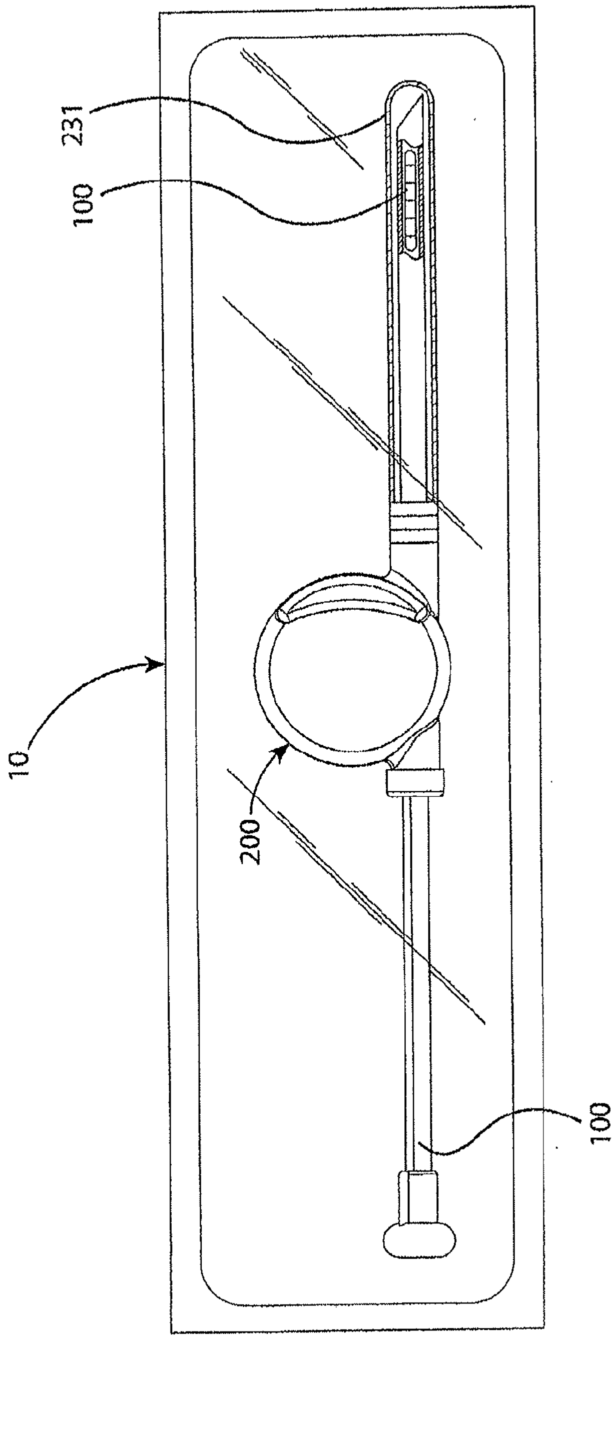


FIG. 41

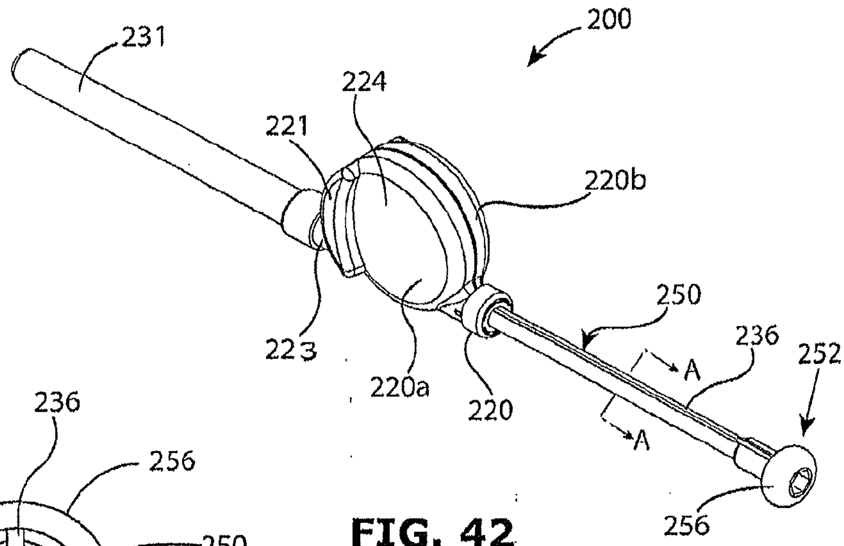


FIG. 42

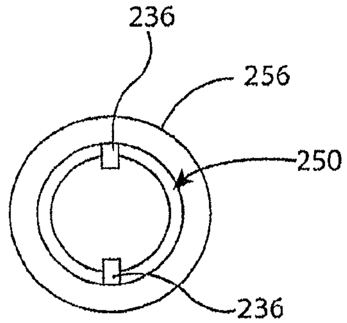


FIG. 42A

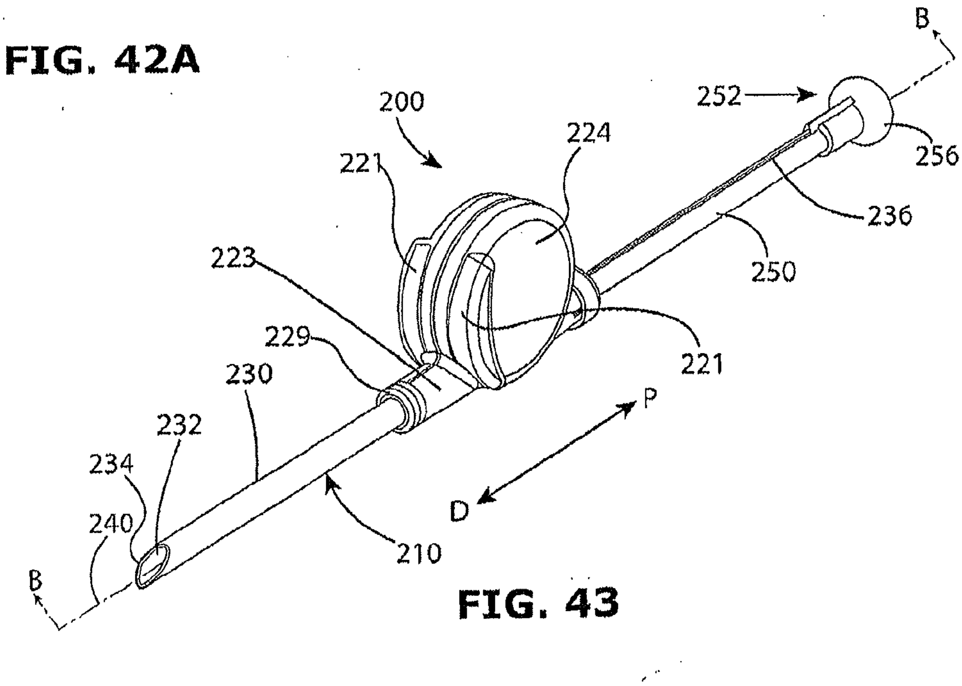


FIG. 43

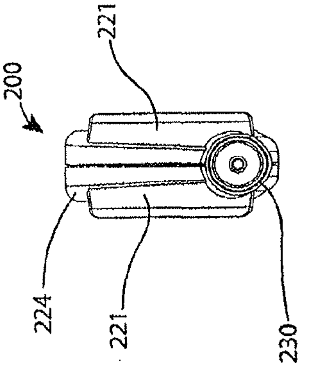


FIG.44

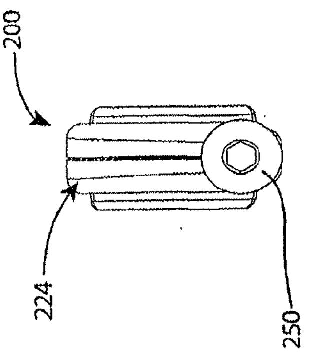


FIG. 45

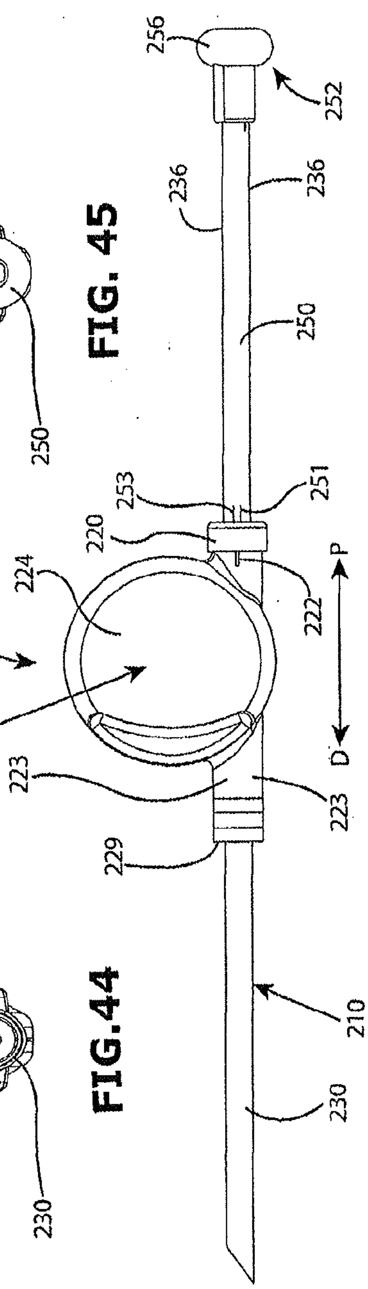


FIG. 46

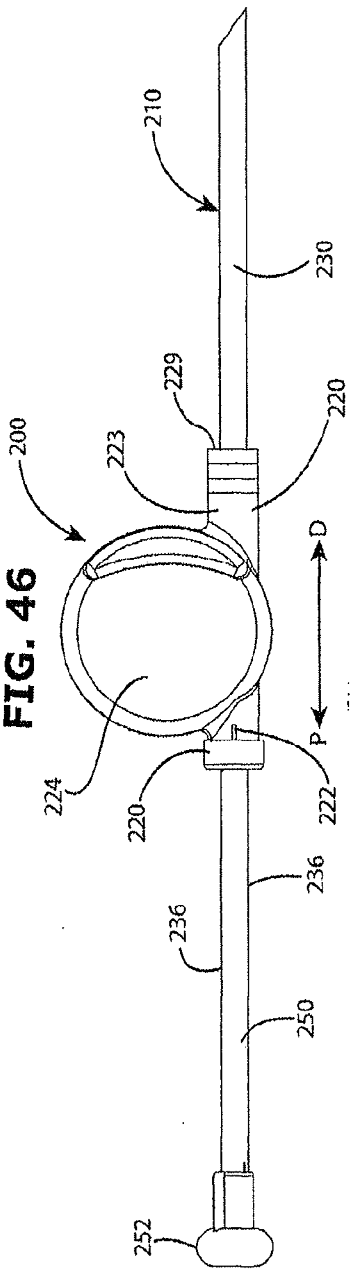


FIG. 47

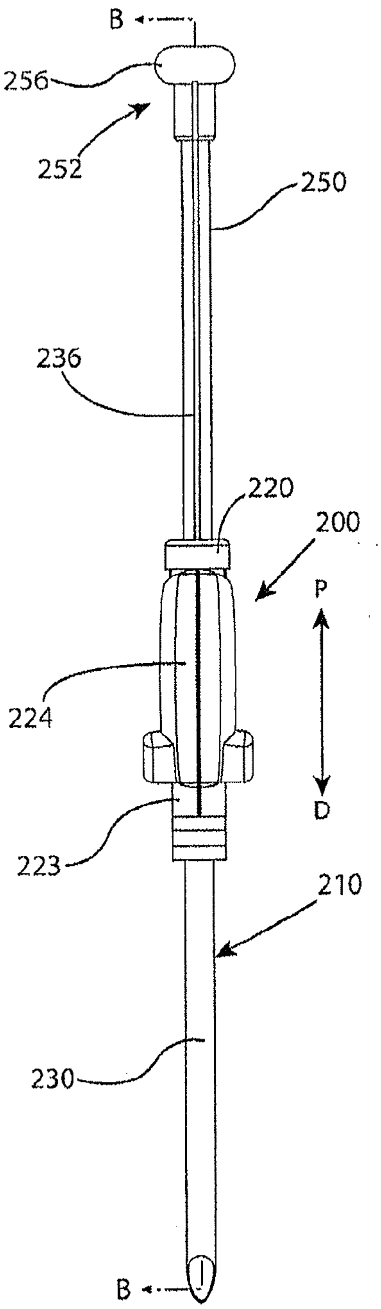
B
256
252
250
236
220
200
P
224
D
223
210
230
B

FIG. 48

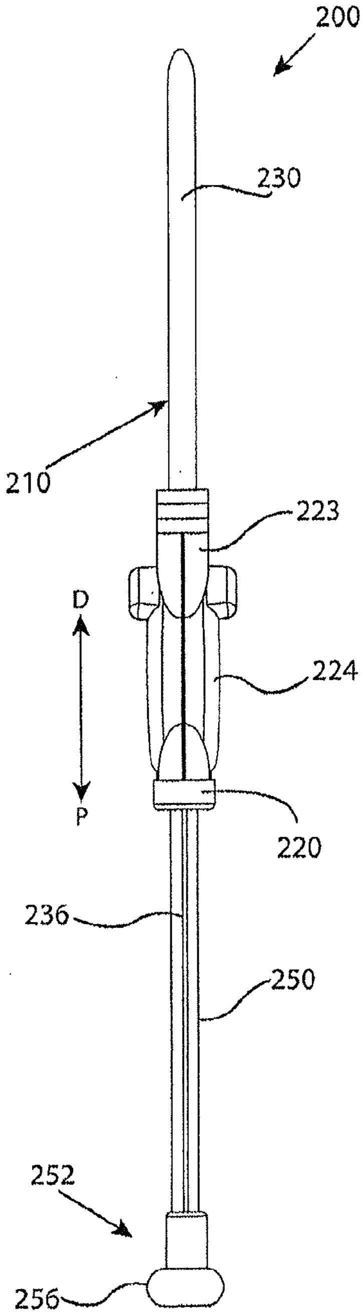
200
230
210
223
D
224
P
220
236
250
252
256

FIG. 49

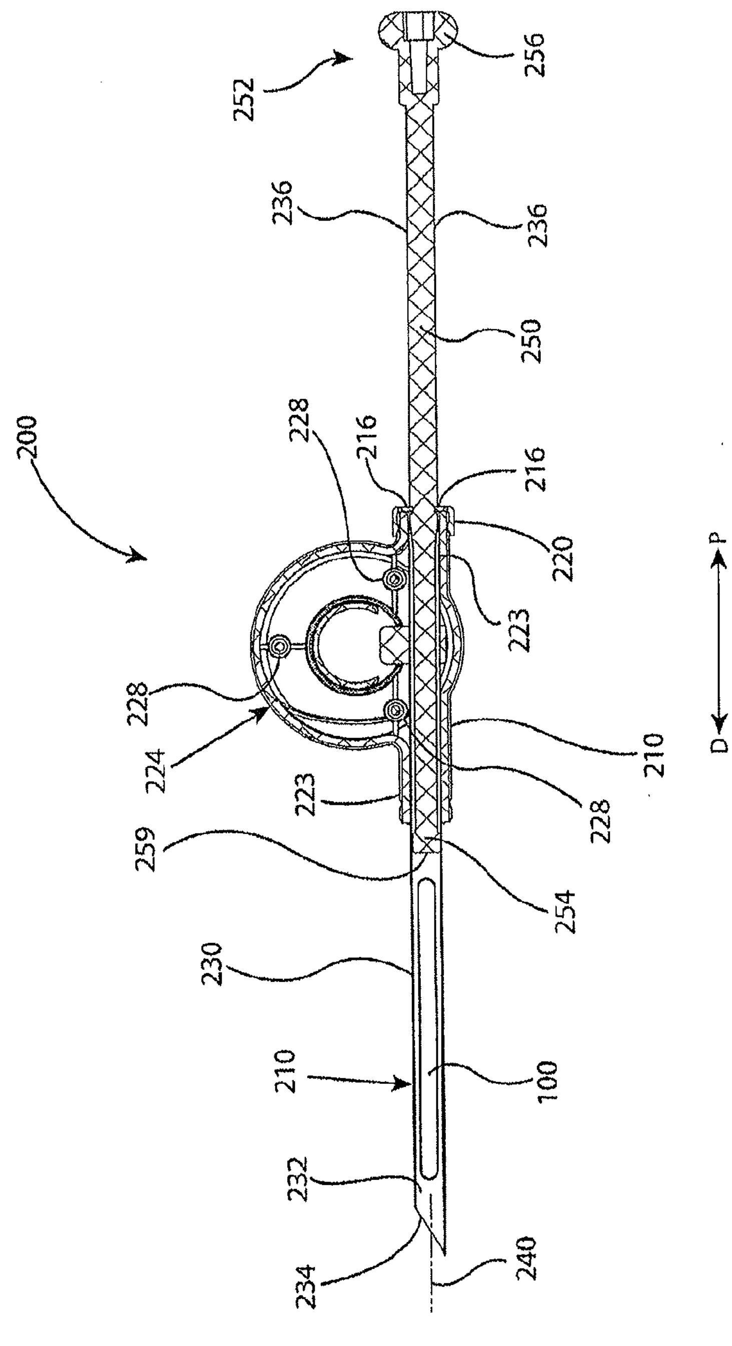
200
252
256
236
236
250
228
216
216
220
223
228
224
223
210
228
259
254
230
210
100
232
234
240
D
P

FIG. 50

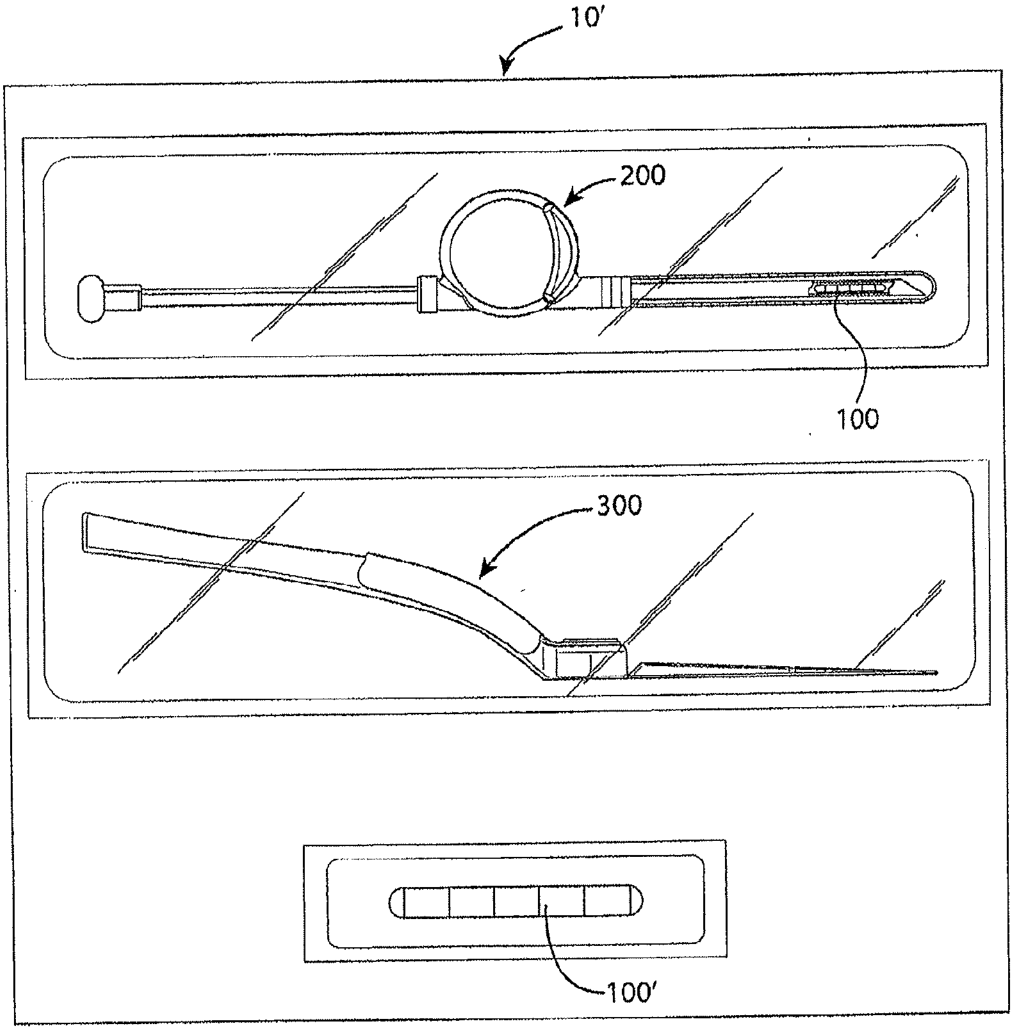


FIG. 51

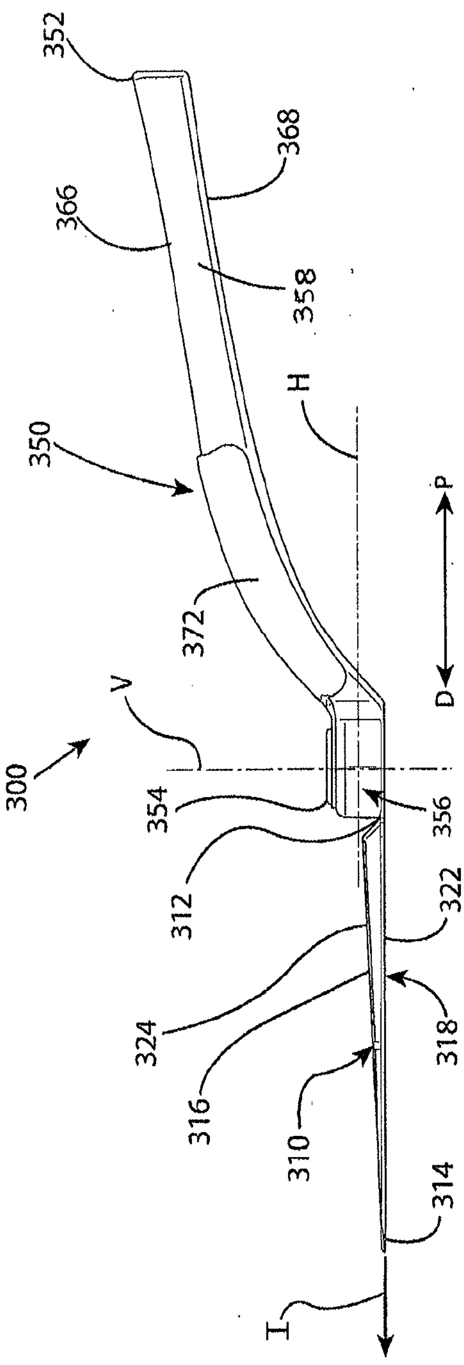
300
352
368
366
358
350
H
372
P
D
V
354
356
312
322
324
318
316
310
314
I

FIG. 52

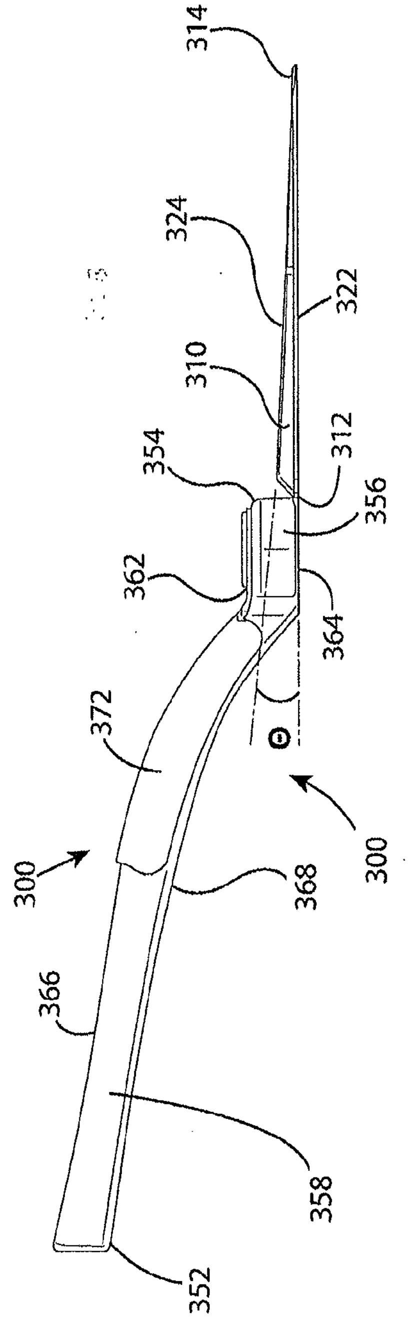
314
324
322
310
312
354
356
362
364
372
Θ
300
368
366
358
352

FIG. 53

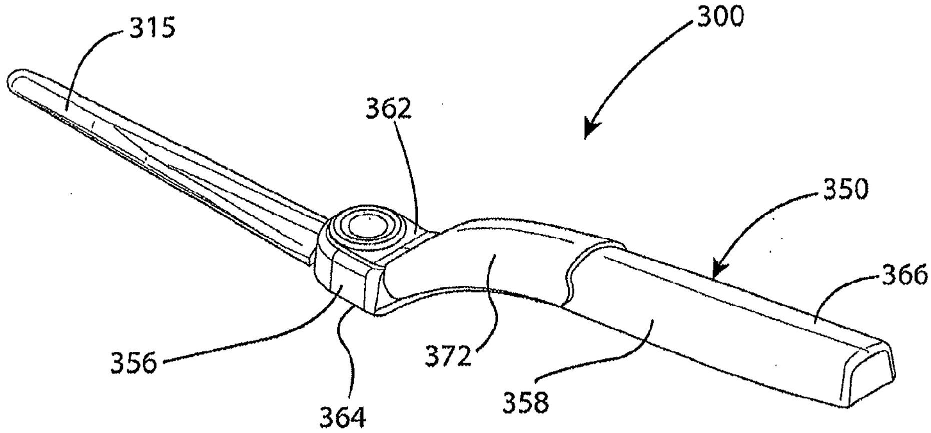


FIG. 54

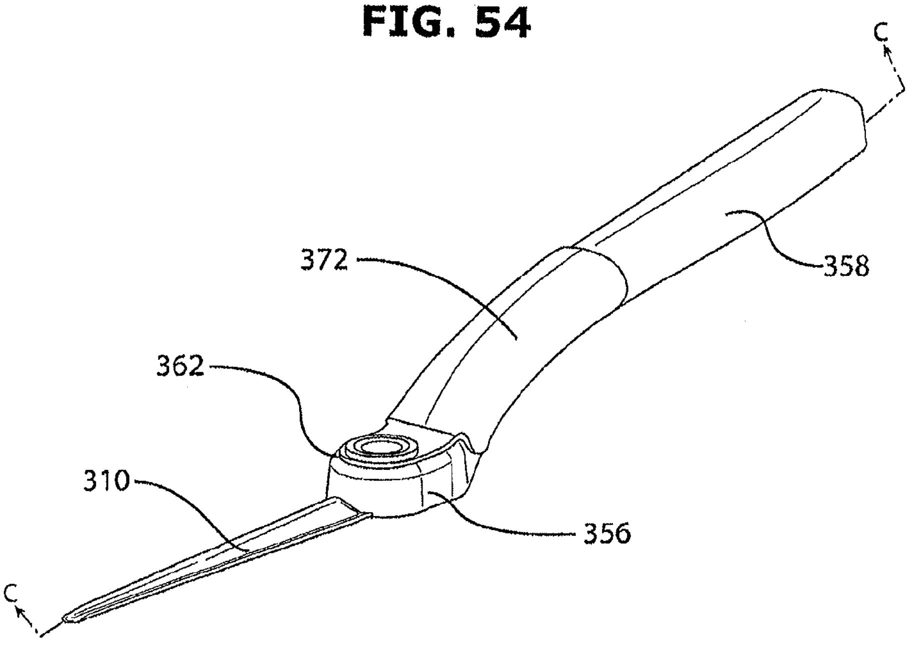


FIG. 55

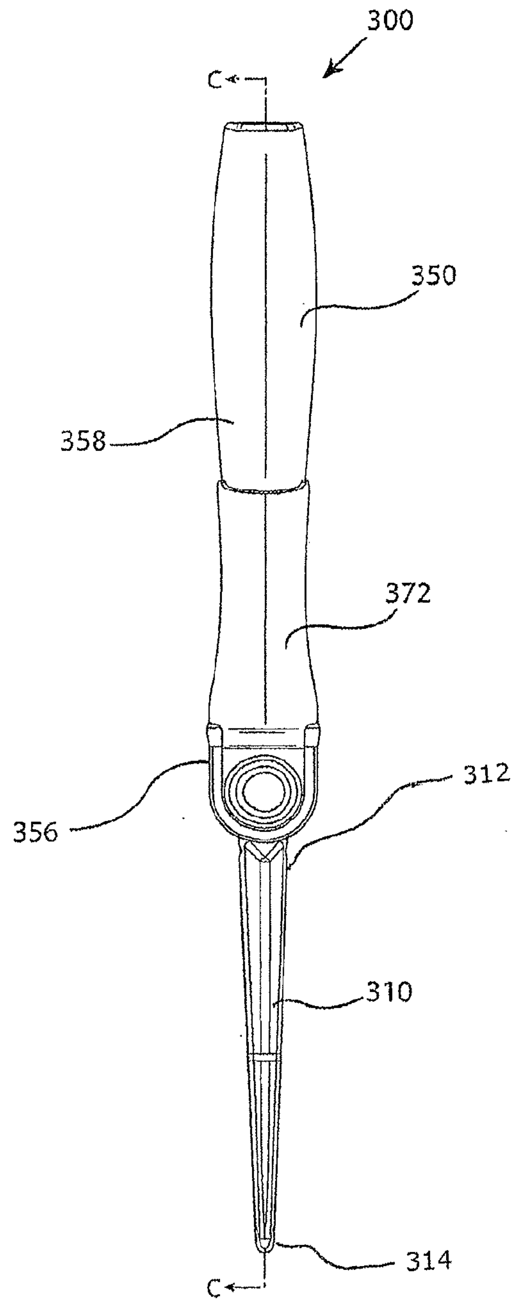


FIG. 56

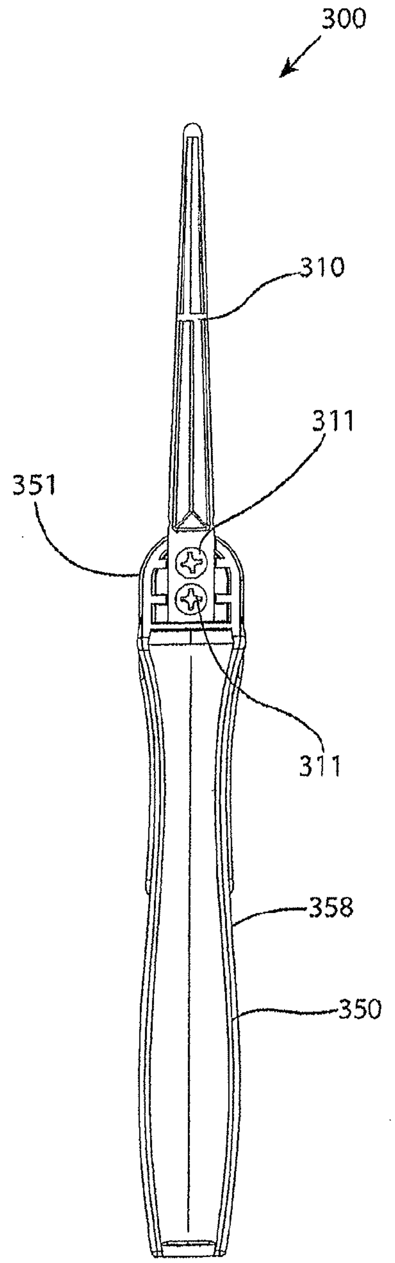


FIG. 57

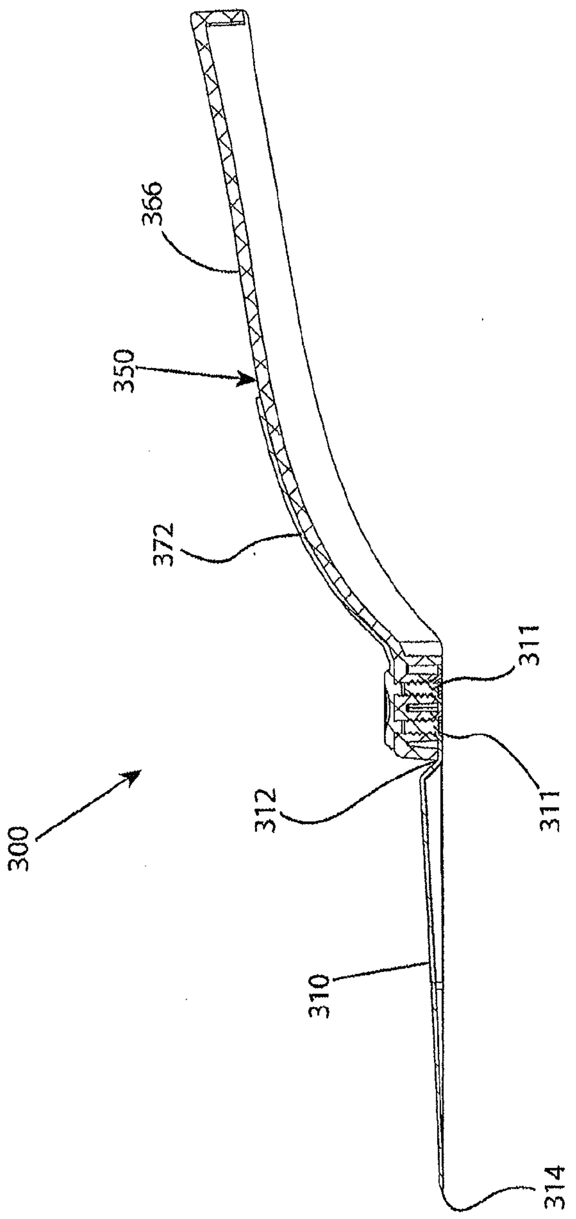


FIG. 58

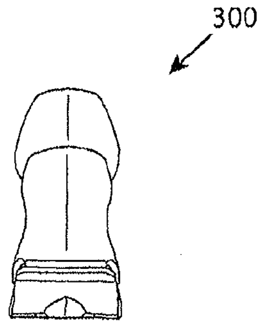


FIG. 59

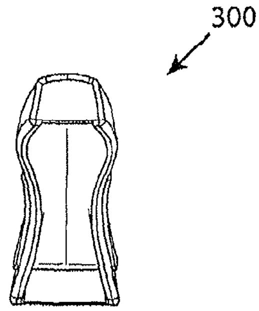


FIG. 60

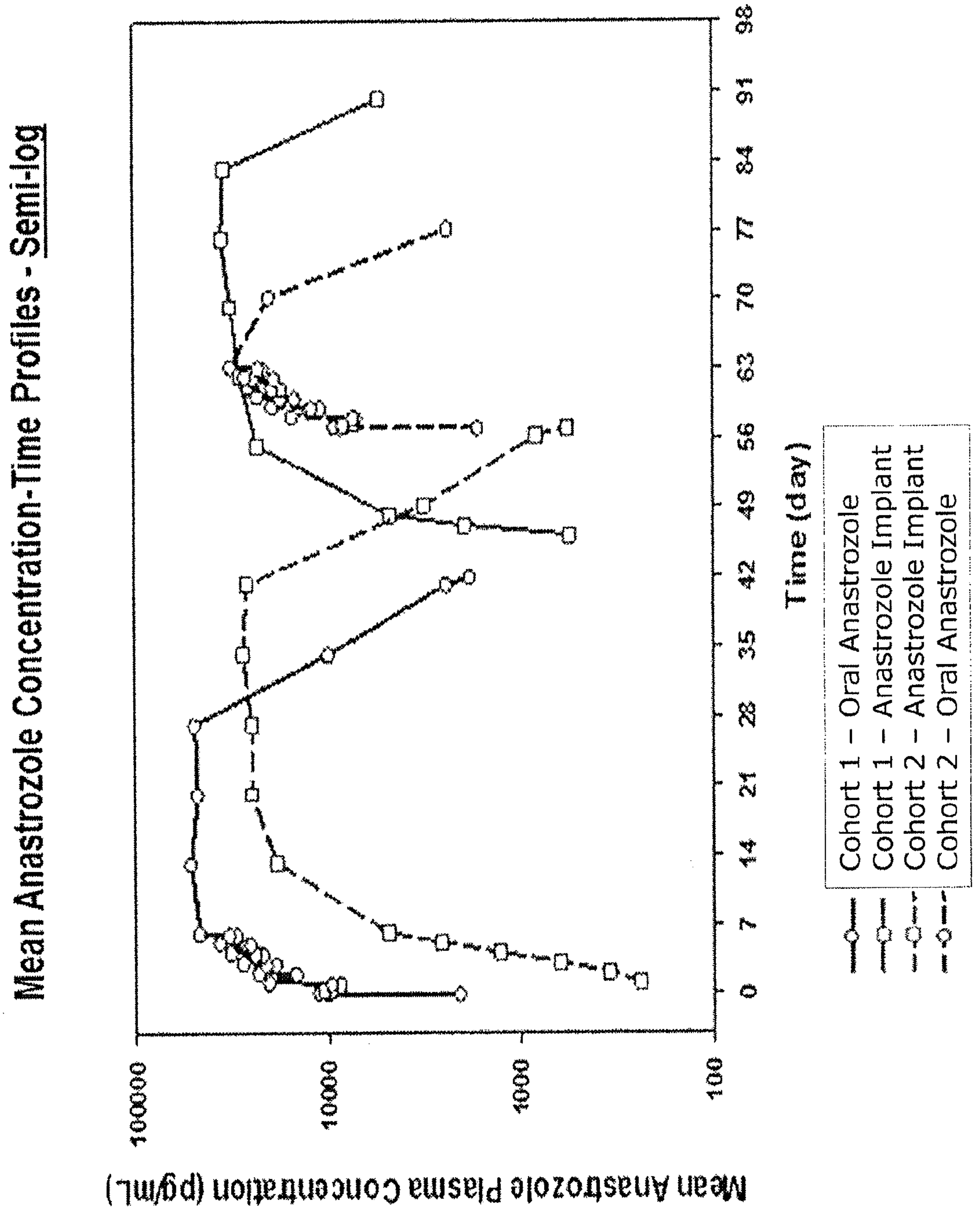
Mean Anastrozole Concentration-Time Profiles - Semi-log
Mean Anastrozole Plasma Concentration (pg/mL)
100000
10000
1000
100
0
7
14
21
28
35
42
49
56
63
70
77
84
91
98
Time (day)
Cohort 1 – Oral Anastrozole
Cohort 1 – Anastrozole Implant
Cohort 2 – Anastrozole Implant
Cohort 2 – Oral Anastrozole

Figure 61

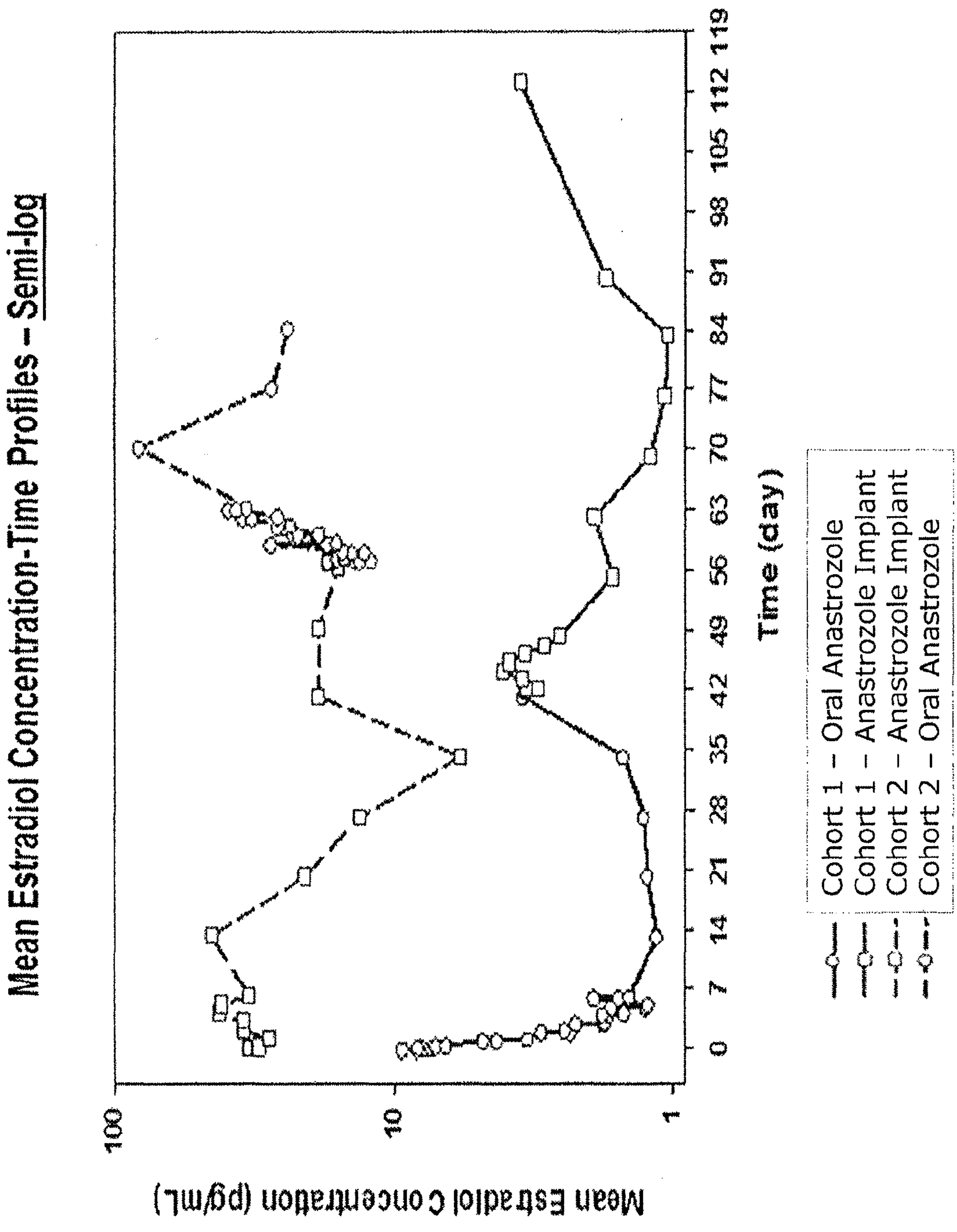
Mean Estradiol Concentration-Time Profiles – Semi-log
Mean Estradiol Concentration (pg/mL)
100
10
1
0
7
14
21
28
35
42
49
56
63
70
77
84
91
98
105
112
119
Time (day)
Cohort 1 – Oral Anastrozole
Cohort 1 – Anastrozole Implant
Cohort 2 – Anastrozole Implant
Cohort 2 – Oral Anastrozole

Figure 62

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 7214206 B
- US 7510549 B


### Non-patent literature cited in the description


- **PARTRIDGE et al.** Adherence to Initial Adjuvant Anastrozole Therapy Among Women With Early-Stage Breast Cancer. *Journal of Clinical Oncology,* 01 February 2008, vol. 26 (4
- **HERSHMAN et al.** Early Discontinuation and Non-adherence to Adjuvant Hormonal Therapy in a Cohort of 8,769 Early-Stage Breast Cancer Patients. *Journal of Clinical Oncology,* 20 September 2010, vol. 26 (27